(19) Europäisches Patentamt
European Patent Office
Office européen des brevets

(11) Veröffentlichungsnummer: **0 456 112 B1**

(12) **EUROPÄISCHE PATENTSCHRIFT**

(45) Veröffentlichungstag der Patentschrift: **31.08.94**

(21) Anmeldenummer: **91107095.1**

(22) Anmeldetag: **02.05.91**

Die Akte enthält technische Angaben, die nach dem Eingang der Anmeldung eingereicht wurden und die nicht in dieser Patentschrift enthalten sind.

(51) Int. Cl.5: **C07C 215/50**, C07C 251/54, C07C 323/29, C07C 323/47, C07D 307/42, C07D 307/14, C07D 309/20, C07D 333/16, C07D 317/28, C07D 409/12, C07D 407/12, //C07D339/06, A01N43/90,A01N43/02, A01N33/24

# (54) Cyclohexenonoximether, Verfahren zu ihrer Herstellung und ihre Verwendung als Herbizide.

(30) Priorität: **09.05.90 DE 4014986**
**20.10.90 DE 4033423**

(43) Veröffentlichungstag der Anmeldung:
**13.11.91 Patentblatt 91/46**

(45) Bekanntmachung des Hinweises auf die Patenterteilung:
**31.08.94 Patentblatt 94/35**

(84) Benannte Vertragsstaaten:
**AT BE CH DE ES FR GB IT LI NL**

(56) Entgegenhaltungen:
**EP-A- 0 172 551**
**EP-A- 0 177 913**
**EP-A- 0 238 021**
**EP-A- 0 332 076**
**EP-A- 0 368 227**

(73) Patentinhaber: **BASF Aktiengesellschaft**
**Carl-Bosch-Strasse 38**
**D-67063 Ludwigshafen (DE)**

(72) Erfinder: **Misslitz, Ulf, Dr.**
**Am Herzel 40**
**W-6730 Neustadt (DE)**
Erfinder: **Meyer, Norbert, Dr.**
**Dossenheimer Weg 22**
**W-6802 Ladenburg (DE)**
Erfinder: **Kast, Juergen**
**Kastanienstrasse 24**
**W-6737 Boehl-Iggelheim (DE)**
Erfinder: **Goetz, Norbert, Dr.**
**Schoefferstrasse 25**
**W-6520 Worms 1 (DE)**
Erfinder: **Harreus, Albrecht, Dr.**
**Teichgasse 13**
**W-6700 Ludwigshafen (DE)**

EP 0 456 112 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Erfinder: **Wuerzer, Bruno, Dr.**
**Ruedigerstrasse 13**
**W-6701 Otterstadt (DE)**
Erfinder: **Walter, Helmut, Dr.**
**Gruenstadter Strasse 82**
**W-6719 Obrigheim (DE)**
Erfinder: **Westphalen, Karl-Otto, Dr.**
**Mausbergweg 58**
**W-6720 Speyer (DE)**
Erfinder: **Gerber, Matthias, Dr.**
**Ritterstrasse 3**
**W-6704 Mutterstadt (DE)**

## Beschreibung

Die Erfindung betrifft neue herbizid wirksame Cyclohexenonoximether der Formel I

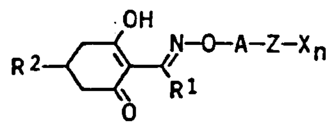

(I)

in der die Substituenten folgende Bedeutung haben:

$R^1$ eine $C_1$-$C_6$-Alkylgruppe;

A eine ggf. $C_1$-$C_3$-Alkyl-substituierte $C_3$-$C_6$-Alkylen- oder $C_4$-$C_6$-Alkenylenkette, in der eine Methylengruppe durch ein Sauerstoff-, ein Schwefelatom, eine Sulfoxid-, eine Sulfongruppe oder -N($R^a$)-, worin

$R^a$ Wasserstoff, eine $C_1$-$C_4$-Alkylgruppe, eine $C_3$-$C_6$-Alkenylgruppe oder eine $C_3$-$C_6$-Alkinylgruppe bedeutet,
ersetzt ist;

Z Phenyl,
ein 5-gliedriger Heteroaromat mit ein bis drei Heteroatomen, ausgewählt aus einer Gruppe bestehend aus drei Stickstoffatomen und einem Sauerstoff- oder Schwefelatom,
ein 6-gliedriger Heteroaromat mit ein bis vier Stickstoffatomen;

X eine Aminogruppe -$NR^aR^b$, worin

$R^a$ Wasserstoff, eine $C_1$-$C_4$-Alkylgruppe, eine $C_3$-$C_6$-Alkenylgruppe oder eine $C_3$-$C_6$-Alkinylgruppe und

$R^b$ Wasserstoff, eine $C_1$-$C_4$-Alkylgruppe, eine $C_3$-$6_6$-Alkenylgruppe, eine $C_3$-$C_6$-Alkinylgruppe, eine $C_1$-$C_6$-Acylgruppe oder ein Benzoylrest, wobei der aromatische Ring ein bis drei Substituenten tragen kann, ausgewählt aus einer Gruppe bestehend aus Nitro, Cyano, Halogen, $C_1$-$C_4$-Alkyl, $C_1$-$C_4$-Alkoxy, $C_1$-$C_4$-Alkylthio und $C_1$-$C_4$-Halogenalkyl
bedeuten oder
Nitro, Cyano, Halogen, $C_1$-$C_4$-Alkyl, partiell oder vollständig halogeniertes $C_1$-$C_4$-Alkyl, $C_1$-$C_4$-Alkoxy, $C_1$-$C_4$-Alkylthio, partiell oder vollständig halogeniertes $C_1$-$C_4$-Alkoxy, Carboxyl, $C_1$-$C_4$-Alkoxycarbonyl, Benzyloxycarbonyl, Phenyl, wobei die aromatischen Reste zusätzlich einen bis drei der folgenden Substituenten tragen können: Nitro, Cyano, Halogen, $C_1$-$C_4$-Alkyl, partiell oder vollständig halogeniertes $C_1$-$C_4$-Alkyl, $C_1$-$C_4$-Alkoxy, $C_1$-$C_4$-Alkylthio, partiell oder vollständig halogeniertes $C_1$-$C_4$-Alkoxy, Carboxyl, $C_1$-$C_4$-Alkoxycarbonyl, Benzyloxycarbonyl,

n 0 bis 3 oder 1 bis 5 für den Fall, daß X Halogen bedeutet;

$R^2$ eine $C_1$-$C_4$-Alkoxy-$C_1$-$C_6$-alkyl- oder $C_1$-$C_4$-Alkylthio-$C_1$-$C_6$-alkylgruppe;
eine $C_3$-$C_7$-Cycloalkylgruppe oder eine $C_5$-$C_7$-Cycloalkenylgruppe, wobei diese Gruppen ein bis drei Substituenten tragen können, ausgewählt aus einer Gruppe bestehend aus $C_1$-$C_4$-Alkyl, $C_1$-$C_4$-Alkoxy, $C_1$-$C_4$-Alkylthio, partiell oder vollständig halogeniertem $C_1$-$C_4$-Alkyl, Hydroxy und Halogen;
ein 5-gliedriger gesättigter Heterocyclus der ein oder zwei Heteroatome enthält, ausgewählt aus einer Gruppe bestehend aus Sauerstoff und Schwefel, wobei der Heterocyclus noch einen bis drei Reste tragen kann, ausgewählt aus einer Gruppe bestehend aus $C_1$-$C_4$-Alkyl, $C_1$-$C_4$-Alkoxy, $C_1$-$C_4$-Alkylthio und partiell oder vollständig halogeniertem $C_1$-$C_4$-Alkyl;
ein 6- oder 7-gliedriger gesättigter, ein- oder zweifach ungesättigter Heterocyclus, enthaltend ein oder zwei Heteroatome, ausgewählt aus einer Gruppe bestehend aus Sauerstoff und Schwefel, wobei der Heterocyclus noch einen bis drei Reste tragen kann, ausgewählt aus einer Gruppe bestehend aus Hydroxy, Halogen, $C_1$-$C_4$-Alkyl, $C_1$-$C_4$-Alkoxy, $C_1$-$C_4$-Alkylthio und partiell oder vollständig halogeniertem $C_1$-$C_4$-Alkyl;
ein 5-gliedriger Heteroaromat, enthaltend ein bis drei Heteroatome, ausgewählt aus einer Gruppe bestehend aus zwei Stickstoffatomen und einem Sauerstoff- oder Schwefelatom, wobei dieser Ring noch einen bis drei Reste tragen kann, ausgewählt aus einer Gruppe bestehend aus Halogen, Cyano, $C_1$-$C_4$-Alkyl, $C_1$-$C_4$-Alkoxy. $C_1$-$C_4$-Alkylthio, partiell oder vollständig halogeniertem $C_1$-$C_4$-Alkyl, $C_2$-$C_6$-Alkenyl, $C_2$-$C_6$-Alkenyloxy, partiell oder vollständig halogeniertem $C_2$-$C_6$-Alkenyl und $C_1$-$C_4$-Alkoxy-$C_1$-$C_4$-alkyl;
die Phenylgruppe oder die Pyridylgruppe, wobei diese Gruppen noch ein bis drei Reste tragen

können, ausgewählt aus einer Gruppe bestehend aus Halogen, Nitro, Cyano, $C_1$-$C_4$-Alkyl, $C_1$-$C_4$-Alkoxy, $C_1$-$C_4$-Alkylthio, partiell oder vollständig halogeniertem $C_1$-$C_4$-Alkyl, $C_3$-$C_6$-Alkenyloxy, $C_3$-$C_6$-Alkinyloxy und $-NR^aR^b$, worin $R^a$ und $R^b$ die oben genannte Bedeutung haben,
bedeuten,

sowie ihre landwirtschaftlich nutzbaren Salze und Ester von $C_1$-$C_{10}$-Carbonsäuren und anorganischen Säuren.

Die erfindungsgemäßen Cyclohexenone I haben offensichtlich sauren Charakter, d.h. sie können einfache Umsetzungsprodukte wie Salze von Alkali- oder Erdalkaliverbindungen oder Enolester bilden.

Die Verbindungen der Formel I können in mehreren tautomeren Formen auftreten, die alle vom Anspruch erfaßt werden. In der Literatur sind Cyclohexenone der allgemeinen Formel I'

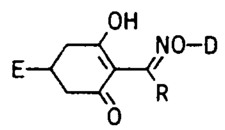

I'

in der u.a.

- D Benzyl und E 2-Ethylthiopropyl (US-A 4 440 566);
- D Benzyl, But-2-enyl und E einen substituierten 5-gliedrigen Heteroarylrest (EP-A 238 021, EP-A 125 094);
- D Benzyl, But-2-enyl und E ein substituiertes Phenyl (EP-A 80 301);
- D 2-Fluor-, 2-Chlorethyl, But-2-en-2-yl, Methoxymethyl oder Methoxyethyl und E einen 5- bis 7-gliedrigen heterocyclischen Ring mit bis zu zwei O-, S-Atomen und mit bis zu zwei Doppelbindungen (EP-A 218 233)

bedeuten, als Herbizide beschrieben.

Außerdem sind aus der EP-A 368 227 herbizid wirksame Cyclohenonoximether sowie deren Salze und Ester vom Typ der Verbindungen I bekannt. Bei diesen bekannten Cyclohexenonen besteht der Oximether-Teil allerdings aus einer 4-Phenylbutyloximino- oder 4-Phenylbutenyloximino-Gruppe, deren Phenylring seinerseits Substituenten tragen kann.

Gemäß der Lehre der EP-A 238 021 sind 1-Hydroxycyclohexen-3-on-Derivate, die in 2-Position u.a. eine (Alkoxyalkyloximino)alkyl- oder geg. subst. Benzyloximinoalkyl-Gruppe und in 5-Position einen Isoxazol-5-yl-Ring, der seinerseits in 3-Stellung durch Alkyl, Alkenyl, Alkinyl oder Cycloalkyl substituiert ist, tragen, ebenfalls herbizid wirksam.

Schließlich offenbart die GB-A 21 25 042 Cyclohexan-1,3-dione, die in 5-Position einen 2-Ethylthiopropyl-Rest tragen und die als Oximether-Teil eine 3-Chlorallyloximino- oder 4-Chlorbenzyloximino-Gruppe aufweisen. Für diese Verbindungen sowie deren Salze und Ester wird eine herbizide Wirkung gegen Ungräser angegeben.

Es werden jedoch Verbindungen gesucht, die bei geringer Aufwandmenge hohe Selektivität aufweisen, d.h. unerwünschte Pflanzen bekämpfen, die Kulturpflanzen dagegen weit weniger schädigen.

Entsprechend dieser Aufgabe wurden die neuen Cyclohexenonoximether der Formel I gefunden, die sich durch eine gute herbizide Wirkung gegen unerwünschte Gräser auszeichnen. Die Verbindungen sind mit breitblättrigen Kulturpflanzen sowie einige mit Gramineenkulturen wie Reis verträglich.

Die Cyclohexenone der Formel I können in an sich bekannter Weise aus schon bekannten Derivaten der Formel II (EP-A 80 301, EP-A-125 094, EP-A 142 741, US-A 4 249 937, EP-A 137 174 und EP-A 177 913) und den entsprechenden Hydroxylaminen der Formel III (Houben-Weyl, 10/1 S. 1181 ff) hergestellt werden (EP-A 169 521).

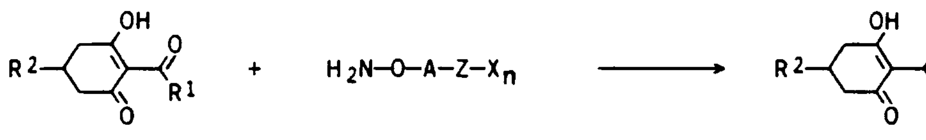

II III I

Zweckmäßig führt man die Umsetzung in heterogener Phase in einem Lösungsmittel, bei einer ausreichenden Temperatur unterhalb von etwa 80 °C, in Gegenwart einer Base durch und verwendet das Hydroxylamin III in Form seines Ammoniumsalzes.

Geeignete Basen sind z. B. Carbonate, Hydrogencarbonate, Acetate, Alkoholate oder Oxide von Alkali- oder Erdalkalimetallen, insbesondere Natriumhydroxid, Kaliumhydroxid, Magnesiumoxid, Calciumoxid. Außerdem können organische Basen wie Pyridin oder tertiäre Amine Verwendung finden. Die Base wird beispielsweise in einer Menge von 0,5 bis 2 Mol-Äquivalent bezogen auf die Ammoniumverbindung zugesetzt.

Als Lösungsmittel eignen sich beispielsweise Dimethylsulfoxid; Alkohole wie Methanol, Ethanol und Isopropanol; aromatische Kohlenwasserstoffe wie Benzol und Toluol; chlorierte Kohlenwasserstoffe wie Chloroform und Dichlorethan; aliphatische Kohlenwasserstoffe wie Hexan und Cyclohexan; Ester wie Essigsäureethylester und Ether wie Diethylether, Dioxan und Tetrahydrofuran. Vorzugsweise führt man die Umsetzung in Methanol mit Natriumhydrogencarbonat als Base durch.

Die Reaktion ist nach wenigen Stunden beendet. Die Zielverbindung kann z.B. durch Einengen der Mischung, Verteilung des Rückstandes in Methylenchlorid/Wasser und Abdestillieren des Lösungsmittels unter vermindertem Druck isoliert werden.

Man kann für diese Umsetzung aber auch unmittelbar die freie Hydroxylaminbase, z.B. in Form einer wäßrigen Lösung, verwenden; je nach verwendetem Lösungsmittel für die Verbindung II erhält man ein- oder zweiphasiges Reaktionsgemisch.

Geeignete Lösungsmittel für diese Variante sind beispielsweise Alkohole wie Methanol, Ethanol, Isopropanol und Cyclohexanol; aliphatische und aromatische, gegebenenfalls chlorierte Kohlenwasserstoffe wie Hexan, Cyclohexan, Methylenchlorid, Toluol und Dichlorethan; Ester wie Essigsäureethylester; Nitrile wie Acetonitril und cyclische Ether wie Dioxan und Tetrahydrofuran.

Alkalimetallsalze der Verbindungen I können durch Behandeln der 3-Hydroxyverbindungen mit Natrium- oder Kaliumhydroxid bzw. -alkoholat in wäßriger Lösung oder in einem organischen Lösungsmittel wie Methanol, Ethanol, Aceton und Toluol erhalten werden.

Andere Metallsalze wie Mangan-, Kupfer-, Zink-, Eisen-, Calcium-, Magnesium- und Bariumsalze können aus den Natriumsalzen in üblicher Weise hergestellt werden, ebenso Ammonium- und Phosphoniumsalze mittels Ammoniak, Phosphonium-, Sulfonium- oder Sulfoxoniumhydroxiden.

Die Verbindungen des Typs II können z.B. aus den entsprechenden Cyclohexan-1,3-dionen der Formel IV

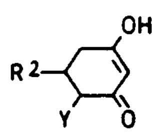

IV,

in der

Y Wasserstoff oder Methoxycarbonyl bedeutet

nach bekannten Methoden (Tetrahedron Lett., 2491 (1975)) hergestellt werden.

Es ist auch möglich, die Verbindungen der Formel II über die Zwischenstufe der Enolester herzustellen, die bei der Umsetzung von Verbindungen der Formel IV mit Säurechloriden in Gegenwart von Basen anfallen und anschließend mit bestimmten Imidazol- oder Pyridinderivaten umgelagert werden (JP-OS 79/063 052).

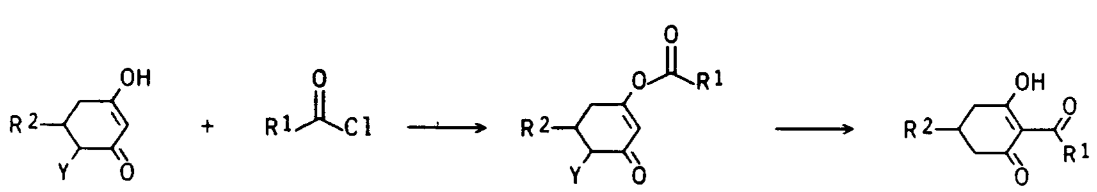

IV II

Zu den Verbindungen der Formel IV gelangt man über eine Reihe bekannter Verfahrensschritte ausgehend von bekannten Vorprodukten.

Die Synthese der Hydroxylamine III erfolgt gemäß dem nachstehenden Reaktionsschema:

$X_n$–Z–A–Y

VII

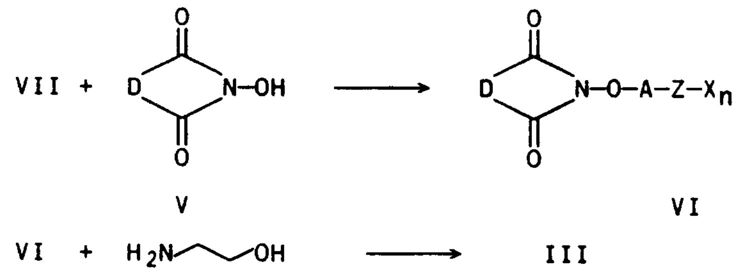

Y = Abgangsgruppe, z.B. Halogen wie Chlor, Brom, Jod oder $CH_3SO_2$-O-.

Die zur Synthese der neuen Hydroxylamine der Formel III benötigten Alkylierungsmittel sind nach literaturbekannten Methoden herstellbar [vgl. DE-A 3 437 919; Tetrahedron Lett. 28, 2639 (1979); Org. Synth., Coll. Vol. 1, 436 (1944); DE-A 2 654 646; DE-A 2 714 561; J. Org. Chem. 52, 3587 (1987); DE-A 948 871; DE-A 948 872; J. Med. Chem. 26, 1570 (1983), Synthesis, 675 (1983) und J. Org. Chem. 48, 4970 (1983)]

VII wird mit einem cyclischen Hydroxyimid V gekoppelt und das erhaltene geschützte Hydroxylaminderivat VI z.B. mit 2-Aminoethanol zum freien Hydroxylamin III gespalten.

In den cyclischen Hydroxyimiden V steht D z.B. für $C_2$-$C_3$-Alkylen, $C_2$-Alkenylen oder einen mit bis zu 3 Doppelbindungen und gegebenenfalls 1 Stickstoffatom enthaltenden 5- oder 6-Ring, z.B. für Phenylen, Pyridinylen, Cyclopentylen, Cyclohexylen oder Cyclohexenylen. Beispielsweise kommen folgende Substanzen in Betracht:

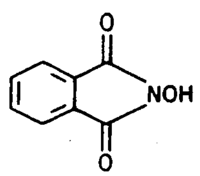 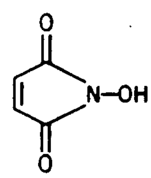 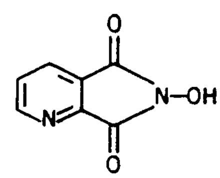

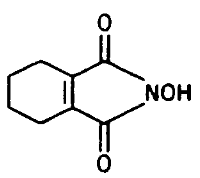 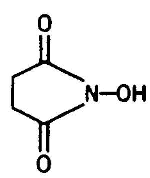 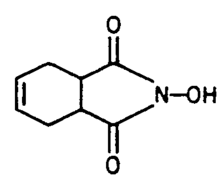

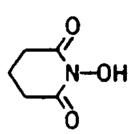

Die Umsetzung der Verbindungen VII mit den Hydroxyimiden V wird zweckmäßigerweise in Gegenwart einer Base ausgeführt. Geeignet sind prinzipiell alle Basen, die in der Lage sind, die Hydroxyimide V zu deprotonieren ohne das Imidsystem anzugreifen. Dies sind insbesondere die sogenannten nichtnucleophilen Basen. Beispielsweise genannt seien Mineralbasen wie Alkalimetall- und Erdalkalimetallcarbonate, Alkalimetall- und Erdalkalimetallhydrogencarbonate, organische Basen wie aliphatische, cycloaliphatische und aromatische tertiäre Amine. Es können auch Gemische dieser Basen verwendet werden.

Als Einzelverbindungen seien folgende Basen beispielhaft aufgeführt: Natriumcarbonat, Kaliumcarbonat, Magnesiumcarbonat, Calciumcarbonat, Bariumcarbonat, die Hydrogencarbonate dieser Metalle, Trimethylamin, Triethylamin, Tributylamin, Ethyldiisopropylamin, N,N-Dimethylanilin, 4-N,N-Dimethylaminopyridin, Diazabicyclooctan, Diazabicycloundecan, N-Methylpiperidin, 1,4-Dimethylpiperazin, Pyridin, Chinolin, Bipyridin, Phenanthrolin. Bevorzugt sind die preiswerten Basen Natrium- und Kaliumcarbonat.

Die Base wird im allgemeinen in äquivalenten Mengen bis zu einem überschuß von 5 Äquivalenten, bezogen auf das Hydroxyimid, zugegeben. Ein höherer überschuß ist möglich, entbehrt aber zusätzliche Vorteile. Die Verwendung einer geringen Basenmenge ist ebenfalls möglich. Bevorzugt wird jedoch eine Basenmenge von 1 bis 3, insbesondere von 1 bis 2 Äquivalenten, bezogen auf das Hydroxyimid V eingesetzt.

Die Verwendung von nucleophilen Basen, z.B. Alkalimetall- und Erdalkalimetallhydroxiden, insbesondere Natrium- und Kaliumhydroxid, ist ebenfalls möglich. In diesem Falle ist es vorteilhaft, die Base in äquivalenten Mengen bezüglich des Hydroxyimids V einzusetzen, um einem nucleophilen Angriff der Hydroxylionen auf die Carbonylfunktion der Imidgruppierung vorzubeugen.

Zweckmäßigerweise setzt man die Ausgangsverbindungen VII mit den Hydroxyimiden V in einem Lösungsmittel um, das sich unter den Reaktionsbedingungen inert verhält. Vorteilhafte Lösungsmittel sind z.B. polareaprotische Lösungsmittel wie Dimethylformamid, N-Methylpyrrolidon, Dimethylsulfoxid, Sulfolan und cyclische Harnstoffe. Die Lösungsmittelmenge ist im allgemeinen nicht kritisch.

Die Umsetzung der Ausgangsverbindungen VII mit den Hydroxyimiden V kann auch unter Anwendung der Phasentransfer-Katalyse ausgeführt werden. In diesem Falle werden mit Wasser zwei Phasen bildende Lösungsmittel, bevorzugt Chlorkohlenwasserstoffe, eingesetzt. Als Phasentransferkatalysatoren eignen sich die üblicherweise zu solchen Zwecken verwendeten quartären Ammonium- und Phosphoniumsalze, Polyethylenglykole, Polyethylenglykolether und Kronenether, wie sie z.B. in Dehmlow et al., Phase Transfer Catalysis, S. 37 - 45 und S. 86 - 93, Verlag Chemie, Weinheim 1980, beschrieben sind. Die Phasentransferkatalysatoren werden zweckmäßigerweise in Mengen von 1 bis 10 Vol%, bevorzugt in Mengen von 3 bis 5 Vol%, bezogen auf das Volumen der Reaktionsmischung, eingesetzt.

Die Umsetzung der Ausgangsverbindungen VII mit den Hydroxyimiden V wird im allgemeinen im Temperaturbereich zwischen 0 und 140 °C, bevorzugt zwischen 20 und 100 °C, insbesondere zwischen 40 und 80 °C, durchgeführt. Zweckmäßigerweise wird dabei so vorgegangen, daß man das Hydroxyimid V zusammen mit der Base im Lösungsmittel vorlegt und das Ausgangsmaterial VII zu dieser Lösung dosiert. Dabei kann es sich als günstig erweisen, wenn das Hydroxyimid bei einer tieferen Temperatur, bespielsweise bei 0 bis 50 °C, zugegeben und die Reaktionsmischung erst nach dieser Zugabe auf die eigentliche Reaktionstemperatur erhitzt wird.

Nach beendeter Reaktion wird die abgekühlte Reaktionsmischung zweckmäßigerweise mit Wasser versetzt, wobei sich die gebildeten Hydroxylaminderivate VI als kristalline Festkörper oder als Öle abscheiden. Die auf diese Weise erhaltenen Hydroxylaminderivate können, falls gewünscht, durch Umkristallisation oder durch Extraktion weiter gereinigt werden.

Die Hydroxylaminderivate VI können zwischengelagert werden oder sogleich in die Hydroxylaminderivate III mit freier Aminogruppe umgewandelt werden. Diese Umwandlung kann nach an sich bekannten Verfahren durchgeführt werden, wie sie beispielsweise in DE-A 36 15 973 und den darin zitierten Schriften beschrieben sind. Bevorzugt wird das Verfahren gemäß DE-A 36 15 973 angewandt, nach dem die Hydroxylaminderivate III mittels Ethanolamin freigesetzt werden. Die Freisetzung der Hydroxylaminderivate III mit Hilfe anderer Basen wie wäßrigen Mineralbasen, mit Aminen, Hydrazinen, Hydroxylaminen oder mittels wäßriger Säuren ist ebenfalls möglich.

Aus den nach diesen Verfahren erhaltenen Reaktionsgemischen können die Hydroxylaminderivate III mittels üblicher Aufarbeitungsmethoden isoliert werden, beispielsweise durch Extraktion oder durch Kristallisation. Zur Erhöhung der Kristallisationstendenz dieser Hydroxylaminderivate kann es oftmals förderlich sein, diese in ihre Salze mit Mineralsäuren oder organischen Säuren überzuführen. Dazu werden im allgemeinen verdünnte Lösungen dieser Säuren mit den Hydroxylaminderivaten umgesetzt, und zwar zweckmäßigerweise in äquivalenten Mengen. Die erhaltenen Hydroxylammoniumsalze können wie die Hydroxylaminderivate mit freier Aminogruppe direkt zu den Herbiziden der Formel I weiterverarbeitet werden oder auch, falls gewünscht, gelagert werden.

Im Hinblick auf die biologische Wirksamkeit werden Cyclohexenone der Formel I bevorzugt, in denen die Substituenten folgende Bedeutung haben:

$R^1$ Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl$_1$ 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere Ethyl und Propyl;

A Alkylen oder Alkenylen, in dem eine Methylengruppe durch ein Sauerstoff-, ein Schwefelatom, eine Sulfoxid-, eine Sulfongruppe oder -N($R^a$)- ersetzt ist, wie
3-Oxapropylen, 3-Azapropylen, 3-Thiapropylen, 3-Thiapropylen-3-oxid, 3-Thiapropylen-3,3-dioxid,

3-Oxabutylen, 3-Azabutylen, 3-Thiabutylen, 3-Thiabutylen-3-oxid, 3-Thiabutylen-3,3-dioxid, 4-Oxabutylen, 4-Azabutylen, 4-Thiabutylen, 4-Thiabutylen-4-oxid, 4-Thiabutylen-4,4-dioxid, 4-Oxabut-2-enylen, 4-Azabut-2-enylen, 4-Thiabut-2-enylen, 3-Oxapentylen, 3-Azapentylen, 3-Thiapentylen, 3-Thiapentylen-3-oxid, 3-Thiapentylen-3,3-dioxid, 4-Oxapentylen, 4-Azapentylen, 4-Thiapentylen, 4-Thiapentylen-4-oxid, 4-Thiapentylen-4,4-dioxid, 5-Oxapentylen, 5-Azapentylen, 5-Thiapentylen, 5-Thiapentylen-5-oxid, 5-Thiapentylen-5,5-dioxid, 5-Oxapent-3-enylen, 5-Azapent-3-enylen, 5-Thiapent-3-enylen, 3-Oxahexylen, 3-Azahexylen, 3-Thiahexylen, 3-Thiahexylen-3-oxid, 3-Thiahexylen-3,3-dioxid, 4-Oxahexylen, 4-Azahexylen, 4-Thiahexylen, 4-Thiahexylen-4-oxid, 4-Thiahexylen-4,4-dioxid, 5-Oxahexylen, 5-Azahexylen, 5-Thiahexylen, 5-Thiahexylen-5-oxid, 5-Thiahexylen-5,5-dioxid, 6-Oxahexylen, 6-Azahexylen, 6-Thiahexylen, 6-Thiahexylen-6-oxid, 6-Thiahexylen-6,6-dioxid, 6-Oxahex-4-enylen, 6-Azahex-4-enylen, 6-Thiahex-4-enylen. Besonders bevorzugt sind 3-Oxapropylen, 3-Oxabutylen und 4-Oxabutylen.

$R^a$ Wasserstoff;

Alkyl wie Methyl, Ethyl, Propyl, 1-Methylethyl, Butyl, 1-Methylpropyl, 2-Methylpropyl und 1,1-Dimethylethyl, insbesondere Methyl und Ethyl;

Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,2-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, insbesondere 2-Propenyl und 2-Butenyl;

Alkinyl wie 2-Propinyl, 2-Butinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, insbesondere 2-Propinyl und 2-Butinyl.

Z Phenyl,

5-gliedriges Heteroaryl wie Furanyl, Pyrrolyl, Thienyl, Imidazolyl, Pyrazolyl, Isoxazolyl, Oxazolyl, Isothiazolyl, Thiazolyl, Oxadiazolyl, Thiadiazolyl, Triazolyl, insbesondere Furanyl und Thienyl,

6-gliedriges Heteroaryl wie Pyridyl, Pyridazyl, Pyrimidyl, Pyrazyl, Triazyl, Tetrazyl, insbesondere Pyridyl und Pyrimidyl;

X Nitro, Cyano,

Halogen wie Fluor, Chlor, Brom und Iod, insbesondere Fluor und Chlor;

Alkyl wie Methyl, Ethyl, Propyl, 1-Methylethyl, Butyl, 1-Methylpropyl, 2-Methylpropyl und 1,1-Dimethylethyl, insbesondere Methyl und 1,1-Dimethylethyl,

Alkoxy wie Methoxy, Ethoxy, Propoxy, 1-Methylethoxy, Butoxy, 1-Methylpropoxy, 2-Methylpropoxy und 1,1-Dimethylethoxy, insbesondere Methoxy, Ethoxy, 1-Methylethoxy und 1,1-Dimethylethoxy,

Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio und Ethylthio,

Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Difluormethyl, Trifluormethyl, 2,2,2-Trifluorethyl und Pentafluorethyl,

Halogenalkoxy wie Difluormethoxy, Trifluormethoxy, Chlor-difluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy,

Carboxyl,

Alkoxycarbonyl wie Methoxycarbonyl, Ethoxycarbonyl, Propyloxycarbonyl, 1-Methylethoxycarbonyl, Butyloxycarbonyl und 1,1-Dimethylethoxycarbonyl, insbesondere Methoxycarbonyl, Ethoxycarbonyl und 1,1-Dimethylethoxycarbonyl, insbesondere Methoxycarbonyl sowie

Benzyloxycarbonyl und Phenyl, wobei die aromatischen Reste ihrerseits ein bis drei der folgenden Reste tragen können: Nitro, Cyano, Carboxyl, Benzyloxycarbonyl, Halogen wie im allgemeinen und im besonderen bei X genannt; Alkyl wie bei $R^1$ genannt, insbesondere Methyl, Ethyl und 1-Methylethyl; Alkoxy wie vorstehend genannt, insbesondere Methoxy und Ethoxy; Alkylthio wie vorstehend genannt, insbesondere Methylthio; Halogenalkyl wie vorstehend genannt, insbesondere Trifluormethyl; Halogenalkoxy wie vorstehend genannt, insbesondere Difluormethoxy und Trifluormethoxy und/oder Alkoxycarbonyl wie vorstehend genannt, insbesondere Methoxycarbonyl und Ethoxycarbonyl.

Eine Aminogruppe $-NR^aR^b$:

$R^a$ Wasserstoff, Alkyl, Alkenyl und Alkinyl wie bei A im allgemeinen und im besonderen genannt;

$R^b$ Wasserstoff, Alkyl, Alkenyl und Alkinyl wie bei $R^a$ im allgemeinen und im besonderen genannt; Acylgruppen wie Acetyl, Propionyl, Butyryl, 2-Methylpropionyl, Pentanoyl, 2-Methylbutyryl, 3-Methylbutyryl, 2,2-Dimethylpropionyl, Hexanoyl, 2-Methylpentanoyl, 3-Methylpentanoyl, 4-Methylpentanoyl, 2,2-Dimethylbutyryl, 2,3-Dimethylbutyryl, 3,3-Dimethylbutyryl und 2-Ethylbutyryl, insbesondere Acetyl und Propionyl;

oder ein Benzoylrest, wobei der aromatische Rest ein bis drei der folgenden Reste tragen kann: Nitro, Cyano, Carboxyl, Benzyloxycarbonyl, Halogen wie im allgemeinen und im besonderen bei X genannt; Alkyl wie bei $R^1$ genannt, insbesondere Methyl; Alkoxy wie vorstehend genannt, insbesondere Methoxy und Ethoxy; Alkylthio wie vorstehend genannt, insbesondere Methylthio; Halogenalkyl wie vorstehend genannt, insbesondere Trifluormethyl; Halogenalkoxy wie vorstehend genannt, insbesondere Difluromethoxy und Trifluormethoxy und/oder Alkoxycarbonyl wie vorstehend genannt insbesondere Methoxycarbonyl und Ethoxycarbonyl.

n 0, 1, 2 oder 3, insbesondere 0, 1 und 2, im Fall daß X Halogen bedeutet auch 1 bis 5. Bevorzugt steht n für 0, 1 oder 2. Bei mehreren Resten X können die Substituenten gleich oder verschieden sein.

$R^2$ Alkyl wie unter X genannt, sowie Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, welche mit einer der unter X genannten Alkoxy- oder Alkylthiogruppen bevorzugt in 1-, 2- oder 3-Position substituiert sind, insbesondere 2-Ethylthiopropyl;

5-gliedriges Heterocycloalkyl wie Tetrahydrofuranyl, Tetrahydrothienyl, Dioxolanyl, Dithiolanyl und Oxathiolanyl, insbesondere Tetrahydrofuranyl, Tetrahydrothienyl und Dioxolanyl, wobei diese Ringe ein bis drei der bereits unter X genannten $C_1$-$C_4$-Alkylgruppen, $C_1$-$C_4$-Alkoxygruppen, $C_1$-$C_4$-Alkylthiogruppen und/oder $C_1$-$C_4$-Halogenalkylgruppen tragen können,

5-gliedriges Heteroaryl wie Pyrrolyl, Pyrazolyl, Imidazolyl, Isoxazolyl, Oxazolyl, Isothiazolyl, Thiazolyl, Furanyl und Thienyl, insbesondere Isoxazolyl und Furanyl,

ein 6- oder 7-gliedriger Heterocyclus wie Tetrahydropyran-3-yl, Dihydropyran-3-yl, Tetrahydropyran-4-yl, Dihydropyran-4-yl, Tetrahydrothiopyran-3-yl, Dihydrothiopyran-3-yl, Tetrahydrothiopyran-4-yl, Dihydrothiopyran-4-yl und Dioxepan-5-yl, insbesondere Tetrahydropyran-3-yl, Tetrahydropyran-4-yl und Tetrahydrothiopyran-3-yl,

ein Phenyl- oder ein Pyridylrest,

wobei die cyclischen Reste ein bis drei der unter X genannten Alkylgruppen, Alkoxygruppen, Alkylthiogruppen und/oder Halogenalkylgruppen tragen können.

Die 5-gliedrigen Heteroaromaten in der Bedeutung $R^2$ können als Substituenten folgende Reste tragen:

Halogenatom wie unter X genannt, insbesondere Fluor und Chlor, Alkoxyalkyl wie Methoxymethyl, 2-Methoxyethyl, 2-Methoxypropyl, 3-Methoxypropyl, 2-Methoxy-1-methylethyl, Ethoxymethyl, 2-Ethoxyethyl, 2-Ethoxypropyl, 3-Ethoxypropyl, 2-Ethoxy-1-methylethyl und 1-Ethoxy-1-methylethyl, insbesondere Methoxyethyl und Ethoxyethyl,

Alkenyl wie Ethenyl, 1-Propenyl, 2-Propenyl, 1-Methylethenyl, 1-Butenyl, 2-Butenyl, 3-Butenyl, 1-Methyl-1-propenyl, 1-Methyl-2-propenyl, 2-Methyl-1-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, insbesondere 1-Methylethenyl bzw. entsprechendes Alkenyloxy und/oder Halogenalkenyl.

Die 6- und 7-gliedrigen Heterocyclen können neben den vorstehend genannten Substituenten auch Hydroxygruppen tragen.

Bei den Phenyl- und Pyridylresten kommen als Substituenten neben den obengenannten Gruppen auch folgende Reste in Betracht:

Alkenyloxy wie 2-Propenyloxy, 2-Butenyloxy, 3-Butenyloxy, 1-Methyl-2-propenyloxy, 2-Methyl-2-propenyloxy, 2-Pentenyloxy, 3-Pentenyloxy, 4-Pentenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-2-propenyloxy, 1-Ethyl-2-propenyloxy, 2-Hexenyloxy, 3-Hexenyloxy, 4-Hexenyloxy, 5-Hexenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Dimethyl-3-butenyloxy, 1,2-Dimethyl-2-butenyloxy, 1,2-Dimethyl-3-butenyloxy, 1,3-Dimethyl-2-butenyloxy, 1,3-Dimethyl-3-butenyloxy, 2,2-Dimethyl-3-butenyloxy, 2,3-Dimethyl-2-butenyloxy, 2,3-Dimethyl-3-butenyloxy, 1-Ethyl-2-butenyloxy, 1-Ethyl-3-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy und 1-Ethyl-2-methyl-2-propenyloxy, insbesondere 2-Propenyloxy und 2-Butenyloxy;

Alkinyloxy wie 2-Propinyloxy, 2-Butinyloxy, 3-Butinyloxy, 1-Methyl-2-propinyloxy, 2-Pentinyloxy, 3-Pentinyloxy, 4-Pentinyloxy, 1-Methyl-3-butinyloxy, 2-Methyl-3-butinyloxy, 1-Methyl-2-butinyloxy, 1,1-Dimethyl-2-propinyloxy, 1-Ethyl-2-propinyloxy, 2-Hexinyloxy, 3-Hexinyloxy, 4-Hexinyloxy, 5-Hexinyloxy, 1-Methyl-2-pentinyloxy, 1-Methyl-3-pentinyloxy, 1-Methyl-4-pentinyloxy, 2-Methyl-3-pentinyloxy, 2-Methyl-4-pentinyloxy, 3-Methyl-4-pentinyloxy, 4-Methyl-2-pentinyloxy, 1,1-Dimethyl-2-butinyloxy, 1,1-Dimethyl-3-butinyloxy, 1,2-Dimethyl-3-butinyloxy, 2,2-Dimethyl-3-butinyloxy, 1-Ethyl-2-butinyloxy, 1-Ethyl-3-butinyloxy, 2-Ethyl-3-butinyloxy und 1-Ethyl-1-methyl-2-propinyloxy, insbesondere 2-Propinyloxy und 2-Butinyloxy;

$-NR^aR^b$ wie bei X im allgemeinen und im besonderen genannt;

Inbesondere bevorzugte Cyclohexenonoximether der Formel I sind in den folgenden Tabellen zusammengefaßt:

Tabelle 1

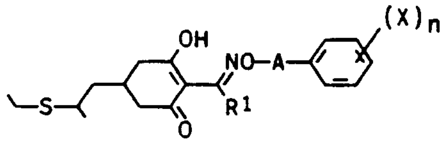

| $R^1$ | A | X | n |
|---|---|---|---|
| $CH_2CH_3$ | $CH_2CH_2O$ | - | 0 |
| $(CH_2)_2CH_3$ | $CH_2CH_2O$ | - | 0 |
| $CH_2CH_3$ | $CH_2CH_2O$ | 2-Cl | 1 |
| $(CH_2)_2CH_3$ | $CH_2CH_2O$ | 2-Cl | 1 |
| $CH_2CH_3$ | $CH_2CH_2O$ | 3-Cl | 1 |
| $(CH_2)_2CH_3$ | $CH_2CH_2O$ | 3-Cl | 1 |
| $CH_2CH_3$ | $CH_2CH_2O$ | 4-Cl | 1 |
| $(CH_2)_2CH_3$ | $CH_2CH_2O$ | 4-Cl | 1 |
| $CH_2CH_3$ | $CH_2CH_2O$ | 2-F | 1 |
| $(CH_2)_2CH_3$ | $CH_2CH_2O$ | 2-F | 1 |
| $CH_2CH_3$ | $CH_2CH_2O$ | 3-F | 1 |
| $(CH_2)_2CH_3$ | $CH_2CH_2O$ | 3-F | 1 |
| $CH_2CH_3$ | $CH_2CH_2O$ | 4-F | 1 |
| $(CH_2)_2CH_3$ | $CH_2CH_2O$ | 4-F | 1 |
| $CH_2CH_3$ | $CH_2CH_2O$ | $3-CF_3$ | 1 |
| $(CH_2)_2CH_3$ | $CH_2CH_2O$ | $3-CF_3$ | 1 |
| $CH_2CH_3$ | $CH_2CH_2CH_2O$ | - | 0 |
| $(CH_2)_2CH_3$ | $CH_2CH_2CH_2O$ | - | 0 |
| $CH_2CH_3$ | $CH_2CH_2CH_2O$ | 4-Cl | 1 |
| $(CH_2)_2CH_3$ | $CH_2CH_2CH_2O$ | 4-Cl | 1 |
| $CH_2CH_3$ | $CH_2CH_2CH_2O$ | 4-F | 1 |
| $(CH_2)_2CH_3$ | $CH_2CH_2CH_2O$ | 4-F | 1 |

Tabelle 1 (Fort.)

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 2-F | 1 |
| $(CH_2)_2CH_3$ | $CH_2CH_2OCH_2$ | 2-F | 1 |
| $CH_2CH_3$ | $CH_2CH_2OCH_2$ | 3-F | 1 |
| $(CH_2)_2CH_3$ | $CH_2CH_2OCH_2$ | 3-F | 1 |
| $CH_2CH_3$ | $CH_2CH_2OCH_2$ | 4-F | 1 |
| $(CH_2)_2CH_3$ | $CH_2CH_2OCH_2$ | 4-F | 1 |
| $CH_2CH_3$ | $CH_2CH_2OCH_2$ | 4-Cl | 1 |
| $(CH_2)_2CH_3$ | $CH_2CH_2OCH_2$ | 4-Cl | 1 |
| $CH_2CH_3$ | $CH_2CH_2OCH_2$ | 2-$CH_3$ | 1 |
| $(CH_2)_2CH_3$ | $CH_2CH_2OCH_2$ | 2-$CH_3$ | 1 |
| $CH_2CH_3$ | $CH_2CH_2OCH_2$ | 3-$CH_3$ | 1 |
| $(CH_2)_2CH_3$ | $CH_2CH_2OCH_2$ | 3-$CH_3$ | 1 |
| $CH_2CH_3$ | $CH_2CH_2OCH_2$ | 4-$CH_3$ | 1 |
| $(CH_2)_2CH_3$ | $CH_2CH_2OCH_2$ | 4-$CH_3$ | 1 |
| $CH_2CH_3$ | $CH_2CH_2OCH_2$ | 4-t$C_4H_9$ | 1 |
| $(CH_2)_2CH_3$ | $CH_2CH_2OCH_2$ | 4-t$C_4H_9$ | 1 |
| $CH_2CH_3$ | $CH_2CH_2SCH_2$ | - | 0 |
| $(CH_2)_2CH_3$ | $CH_2CH_2SCH_2$ | - | 0 |
| $CH_2CH_3$ | $CH_2CH_2SCH_2$ | 4-Cl | 1 |
| $(CH_2)_2CH_3$ | $CH_2CH_2SCH_2$ | 4-Cl | 1 |
| $CH_2CH_3$ | $CH_2CH_2SCH_2$ | 4-F | 1 |
| $(CH_2)_2CH_3$ | $CH_2CH_2SCH_2$ | 4-F | 1 |
| $CH_2CH_3$ | $CH_2CH_2CH_2CH_2O$ | - | 0 |
| $(CH_2)_2CH_3$ | $CH_2CH_2CH_2CH_2O$ | - | 0 |
| $CH_2CH_3$ | $CH_2CH_2CH_2CH_2O$ | 4-Cl | 1 |
| $(CH_2)_2CH_3$ | $CH_2CH_2CH_2CH_2O$ | 4-Cl | 1 |
| $CH_2CH_3$ | $CH_2CH_2CH_2CH_2O$ | 4-F | 1 |
| $(CH_2)_2CH_3$ | $CH_2CH_2CH_2CH_2O$ | 4-F | 1 |

Tabelle 1 (Fort.)

| $R^1$ | A | X | n |
|---|---|---|---|
| $CH_2CH_3$ | $CH_2CH_2CH_2CH_2CH_2O$ | - | 0 |
| $(CH_2)_2CH_3$ | $CH_2CH_2CH_2CH_2CH_2O$ | - | 0 |
| $CH_2CH_3$ | $CH_2CH_2CH_2CH_2CH_2O$ | 4-Cl | 1 |
| $(CH_2)_2CH_3$ | $CH_2CH_2CH_2CH_2CH_2O$ | 4-Cl | 1 |
| $CH_2CH_3$ | $CH_2CH_2CH_2CH_2CH_2O$ | 4-F | 1 |
| $(CH_2)_2CH_3$ | $CH_2CH_2CH_2CH_2CH_2O$ | 4-F | 1 |

Tabelle 2

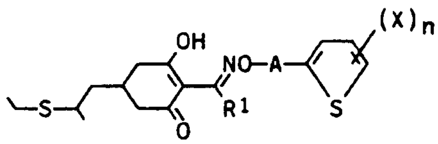

| R1 | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 5-Cl | 1 |
| $(CH_2)_2CH_3$ | $CH_2CH_2OCH_2$ | 5-Cl | 1 |
| $CH_2CH_3$ | $CH_2CH_2OCH_2$ | 5-$CH_3$ | 1 |
| $(CH_2)_2CH_3$ | $CH_2CH_2OCH_2$ | 5-$CH_3$ | 1 |
| $CH_2CH_3$ | $CH_2CH_2OCH_2$ | 5-$C_2H_5$ | 1 |
| $(CH_2)_2CH_3$ | $CH_2CH_2OCH_2$ | 5-$C_2H_5$ | 1 |
| $CH_2CH_3$ | $CH_2CH_2CH_2OCH_2$ | - | 0 |
| $(CH_2)_2CH_3$ | $CH_2CH_2CH_2OCH_2$ | - | 0 |
| $CH_2CH_3$ | $CH_2CH_2CH_2OCH_2$ | 5-Cl | 1 |
| $(CH_2)_2CH_3$ | $CH_2CH_2CH_2OCH_2$ | 5-Cl | 1 |
| $CH_2CH_3$ | $CH_2CH_2CH_2OCH_2$ | 5-$CH_3$ | 1 |
| $(CH_2)_2CH_3$ | $CH_2CH_2CH_2OCH_2$ | 5-$CH_3$ | 1 |
| $CH_2CH_3$ | $CH_2CH_2CH_2OCH_2$ | 5-$C_2H_5$ | 1 |
| $(CH_2)_2CH_3$ | $CH_2CH_2CH_2OCH_2$ | 5-$C_2H_5$ | 1 |
| $CH_2CH_3$ | $CH_2CH_2CH_2OCH_2CH_2$ | - | 0 |
| $(CH_2)_2CH_3$ | $CH_2CH_2CH_2OCH_2CH_2$ | - | 0 |
| $CH_2CH_3$ | $CH_2CH_2CH_2OCH_2CH_2$ | 5-Cl | 1 |
| $(CH_2)_2CH_3$ | $CH_2CH_2CH_2OCH_2CH_2$ | 5-Cl | 1 |
| $CH_2CH_3$ | $CH_2CH_2CH_2OCH_2CH_2$ | 5-$CH_3$ | 1 |
| $(CH_2)_2CH_3$ | $CH_2CH_2CH_2OCH_2CH_2$ | 5-$CH_3$ | 1 |
| $CH_2CH_3$ | $CH_2CH_2CH_2OCH_2CH_2$ | 5-$C_2H_5$ | 1 |
| $(CH_2)_2CH_3$ | $CH_2CH_2CH_2OCH_2CH_2$ | 5-$C_2H_5$ | 1 |

Tabelle 2 (Fort.)

| $R^1$ | A | X | n |
|---|---|---|---|
| $CH_2CH_3$ | $CH_2CH_2CH_2CH_2OCH_2$ | - | 0 |
| $(CH_2)_2CH_3$ | $CH_2CH_2CH_2CH_2OCH_2$ | - | 0 |
| $CH_2CH_3$ | $CH_2CH_2CH_2CH_2OCH_2$ | 5-Cl | 1 |
| $(CH_2)_2CH_3$ | $CH_2CH_2CH_2CH_2OCH_2$ | 5-Cl | 1 |
| $CH_2CH_3$ | $CH_2CH_2CH_2CH_2OCH_2$ | 5-$CH_3$ | 1 |
| $(CH_2)_2CH_3$ | $CH_2CH_2CH_2CH_2OCH_2$ | 5-$CH_3$ | 1 |
| $CH_2CH_3$ | $CH_2CH_2CH_2CH_2OCH_2$ | 5-$C_2H_5$ | 1 |
| $(CH_2)_2CH_3$ | $CH_2CH_2CH_2CH_2OCH_2$ | 5-$C_2H_5$ | 1 |

Tabelle 3

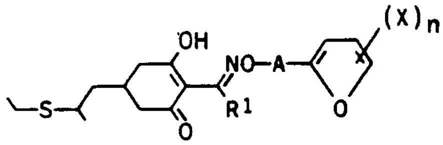

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 5-$CH_3$ | 1 |
| $(CH_2)_2CH_3$ | $CH_2CH_2OCH_2$ | 5-$CH_3$ | 1 |
| $CH_2CH_3$ | $CH_2CH_2OCH_2$ | 5-$C_2H_5$ | 1 |
| $(CH_2)_2CH_3$ | $CH_2CH_2OCH_2$ | 5-$C_2H_5$ | 1 |
| $CH_2CH_3$ | $CH_2CH_2CH_2OCH_2$ | - | 0 |
| $(CH_2)_2CH_3$ | $CH_2CH_2CH_2OCH_2$ | - | 0 |
| $CH_2CH_3$ | $CH_2CH_2CH_2OCH_2$ | 5-$CH_3$ | 1 |
| $(CH_2)_2CH_3$ | $CH_2CH_2CH_2OCH_2$ | 5-$CH_3$ | 1 |
| $CH_2CH_3$ | $CH_2CH_2CH_2OCH_2$ | 5-$C_2H_5$ | 1 |
| $(CH_2)_2CH_3$ | $CH_2CH_2CH_2OCH_2$ | 5-$C_2H_5$ | 1 |
| $CH_2CH_3$ | $CH_2CH_2CH_2OCH_2CH_2$ | - | 0 |
| $(CH_2)_2CH_3$ | $CH_2CH_2CH_2OCH_2CH_2$ | - | 0 |
| $CH_2CH_3$ | $CH_2CH_2CH_2OCH_2CH_2$ | 5-$CH_3$ | 1 |
| $(CH_2)_2CH_3$ | $CH_2CH_2CH_2OCH_2CH_2$ | 5-$CH_3$ | 1 |
| $CH_2CH_3$ | $CH_2CH_2CH_2OCH_2CH_2$ | 5-$C_2H_5$ | 1 |
| $(CH_2)_2CH_3$ | $CH_2CH_2CH_2OCH_2CH_2$ | 5-$C_2H_5$ | 1 |
| $CH_2CH_3$ | $CH_2CH_2CH_2CH_2OCH_2$ | - | 0 |
| $(CH_2)_2CH_3$ | $CH_2CH_2CH_2CH_2OCH_2$ | - | 0 |
| $CH_2CH_3$ | $CH_2CH_2CH_2CH_2OCH_2$ | 5-$CH_3$ | 1 |
| $(CH_2)_2CH_3$ | $CH_2CH_2CH_2CH_2OCH_2$ | 5-$CH_3$ | 1 |
| $CH_2CH_3$ | $CH_2CH_2CH_2CH_2OCH_2$ | 5-$C_2H_5$ | 1 |
| $(CH_2)_2CH_3$ | $CH_2CH_2CH_2CH_2OCH_2$ | 5-$C_2H_5$ | 1 |

Tabelle 4

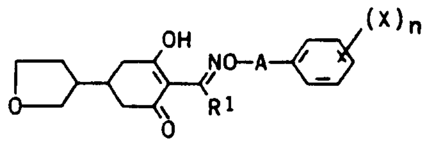

| $R^1$ | A | X | n |
|---|---|---|---|
| $CH_2CH_3$ | $CH_2CH_2O$ | - | 0 |
| $(CH_2)_2CH_3$ | $CH_2CH_2O$ | - | 0 |
| $CH_2CH_3$ | $CH_2CH_2O$ | 2-Cl | 1 |
| $(CH_2)_2CH_3$ | $CH_2CH_2O$ | 2-Cl | 1 |
| $CH_2CH_3$ | $CH_2CH_2O$ | 3-Cl | 1 |
| $(CH_2)_2CH_3$ | $CH_2CH_2O$ | 3-Cl | 1 |
| $CH_2CH_3$ | $CH_2CH_2O$ | 4-Cl | 1 |
| $(CH_2)_2CH_3$ | $CH_2CH_2O$ | 4-Cl | 1 |
| $CH_2CH_3$ | $CH_2CH_2O$ | 2-F | 1 |
| $(CH_2)_2CH_3$ | $CH_2CH_2O$ | 2-F | 1 |
| $CH_2CH_3$ | $CH_2CH_2O$ | 3-F | 1 |
| $(CH_2)_2CH_3$ | $CH_2CH_2O$ | 3-F | 1 |
| $CH_2CH_3$ | $CH_2CH_2O$ | 4-F | 1 |
| $(CH_2)_2CH_3$ | $CH_2CH_2O$ | 4-F | 1 |
| $CH_2CH_3$ | $CH_2CH_2O$ | $3-CF_3$ | 1 |
| $(CH_2)_2CH_3$ | $CH_2CH_2O$ | $3-CF_3$ | 1 |
| $CH_2CH_3$ | $CH_2CH_2CH_2O$ | - | 0 |
| $(CH_2)_2CH_3$ | $CH_2CH_2CH_2O$ | - | 0 |
| $CH_2CH_3$ | $CH_2CH_2CH_2O$ | 4-Cl | 1 |
| $(CH_2)_2CH_3$ | $CH_2CH_2CH_2O$ | 4-Cl | 1 |
| $CH_2CH_3$ | $CH_2CH_2CH_2O$ | 4-F | 1 |
| $(CH_2)_2CH_3$ | $CH_2CH_2CH_2O$ | 4-F | 1 |

Tabelle 4 (Fort.)

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 2-F | 1 |
| $(CH_2)_2CH_3$ | $CH_2CH_2OCH_2$ | 2-F | 1 |
| $CH_2CH_3$ | $CH_2CH_2OCH_2$ | 3-F | 1 |
| $(CH_2)_2CH_3$ | $CH_2CH_2OCH_2$ | 3-F | 1 |
| $CH_2CH_3$ | $CH_2CH_2OCH_2$ | 4-F | 1 |
| $(CH_2)_2CH_3$ | $CH_2CH_2OCH_2$ | 4-F | 1 |
| $CH_2CH_3$ | $CH_2CH_2OCH_2$ | 4-Cl | 1 |
| $(CH_2)_2CH_3$ | $CH_2CH_2OCH_2$ | 4-Cl | 1 |
| $CH_2CH_3$ | $CH_2CH_2OCH_2$ | $2-CH_3$ | 1 |
| $(CH_2)_2CH_3$ | $CH_2CH_2OCH_2$ | $2-CH_3$ | 1 |
| $CH_2CH_3$ | $CH_2CH_2OCH_2$ | $3-CH_3$ | 1 |
| $(CH_2)_2CH_3$ | $CH_2CH_2OCH_2$ | $3-CH_3$ | 1 |
| $CH_2CH_3$ | $CH_2CH_2OCH_2$ | $4-CH_3$ | 1 |
| $(CH_2)_2CH_3$ | $CH_2CH_2OCH_2$ | $4-CH_3$ | 1 |
| $CH_2CH_3$ | $CH_2CH_2OCH_2$ | $4-tC_4H_9$ | 1 |
| $(CH_2)_2CH_3$ | $CH_2CH_2OCH_2$ | $4-tC_4H_9$ | 1 |
| $CH_2CH_3$ | $CH_2CH_2SCH_2$ | - | 0 |
| $(CH_2)_2CH_3$ | $CH_2CH_2SCH_2$ | - | 0 |
| $CH_2CH_3$ | $CH_2CH_2SCH_2$ | 4-Cl | 1 |
| $(CH_2)_2CH_3$ | $CH_2CH_2SCH_2$ | 4-Cl | 1 |
| $CH_2CH_3$ | $CH_2CH_2SCH_2$ | 4-F | 1 |
| $(CH_2)_2CH_3$ | $CH_2CH_2SCH_2$ | 4-F | 1 |
| $CH_2CH_3$ | $CH_2CH_2CH_2CH_2O$ | - | 0 |
| $(CH_2)_2CH_3$ | $CH_2CH_2CH_2CH_2O$ | - | 0 |
| $CH_2CH_3$ | $CH_2CH_2CH_2CH_2O$ | 4-Cl | 1 |
| $(CH_2)_2CH_3$ | $CH_2CH_2CH_2CH_2O$ | 4-Cl | 1 |
| $CH_2CH_3$ | $CH_2CH_2CH_2CH_2O$ | 4-F | 1 |
| $(CH_2)_2CH_3$ | $CH_2CH_2CH_2CH_2O$ | 4-F | 1 |

Tabelle 4 (Fort.)

| $R^1$ | A | X | n |
|---|---|---|---|
| $CH_2CH_3$ | $CH_2CH_2CH_2CH_2CH_2O$ | - | 0 |
| $(CH_2)_2CH_3$ | $CH_2CH_2CH_2CH_2CH_2O$ | - | 0 |
| $CH_2CH_3$ | $CH_2CH_2CH_2CH_2CH_2O$ | 4-Cl | 1 |
| $(CH_2)_2CH_3$ | $CH_2CH_2CH_2CH_2CH_2O$ | 4-Cl | 1 |
| $CH_2CH_3$ | $CH_2CH_2CH_2CH_2CH_2O$ | 4-F | 1 |
| $(CH_2)_2CH_3$ | $CH_2CH_2CH_2CH_2CH_2O$ | 4-F | 1 |

Tabelle 5

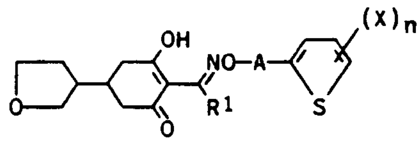

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 5-Cl | 1 |
| $(CH_2)_2CH_3$ | $CH_2CH_2OCH_2$ | 5-Cl | 1 |
| $CH_2CH_3$ | $CH_2CH_2OCH_2$ | 5-$CH_3$ | 1 |
| $(CH_2)_2CH_3$ | $CH_2CH_2OCH_2$ | 5-$CH_3$ | 1 |
| $CH_2CH_3$ | $CH_2CH_2OCH_2$ | 5-$C_2H_5$ | 1 |
| $(CH_2)_2CH_3$ | $CH_2CH_2OCH_2$ | 5-$C_2H_5$ | 1 |
| $CH_2CH_3$ | $CH_2CH_2CH_2OCH_2$ | - | 0 |
| $(CH_2)_2CH_3$ | $CH_2CH_2CH_2OCH_2$ | - | 0 |
| $CH_2CH_3$ | $CH_2CH_2CH_2OCH_2$ | 5-Cl | 1 |
| $(CH_2)_2CH_3$ | $CH_2CH_2CH_2OCH_2$ | 5-Cl | 1 |
| $CH_2CH_3$ | $CH_2CH_2CH_2OCH_2$ | 5-$CH_3$ | 1 |
| $(CH_2)_2CH_3$ | $CH_2CH_2CH_2OCH_2$ | 5-$CH_3$ | 1 |
| $CH_2CH_3$ | $CH_2CH_2CH_2OCH_2$ | 5-$C_2H_5$ | 1 |
| $(CH_2)_2CH_3$ | $CH_2CH_2CH_2OCH_2$ | 5-$C_2H_5$ | 1 |
| $CH_2CH_3$ | $CH_2CH_2CH_2OCH_2CH_2$ | - | 0 |
| $(CH_2)_2CH_3$ | $CH_2CH_2CH_2OCH_2CH_2$ | - | 0 |
| $CH_2CH_3$ | $CH_2CH_2CH_2OCH_2CH_2$ | 5-Cl | 1 |
| $(CH_2)_2CH_3$ | $CH_2CH_2CH_2OCH_2CH_2$ | 5-Cl | 1 |
| $CH_2CH_3$ | $CH_2CH_2CH_2OCH_2CH_2$ | 5-$CH_3$ | 1 |
| $(CH_2)_2CH_3$ | $CH_2CH_2CH_2OCH_2CH_2$ | 5-$CH_3$ | 1 |
| $CH_2CH_3$ | $CH_2CH_2CH_2OCH_2CH_2$ | 5-$C_2H_5$ | 1 |
| $(CH_2)_2CH_3$ | $CH_2CH_2CH_2OCH_2CH_2$ | 5-$C_2H_5$ | 1 |

Tabelle 5 (Fort.)

| $R^1$ | A | X | n |
|---|---|---|---|
| $CH_2CH_3$ | $CH_2CH_2CH_2CH_2OCH_2$ | - | 0 |
| $(CH_2)_2CH_3$ | $CH_2CH_2CH_2CH_2OCH_2$ | - | 0 |
| $CH_2CH_3$ | $CH_2CH_2CH_2CH_2OCH_2$ | 5-Cl | 1 |
| $(CH_2)_2CH_3$ | $CH_2CH_2CH_2CH_2OCH_2$ | 5-Cl | 1 |
| $CH_2CH_3$ | $CH_2CH_2CH_2CH_2OCH_2$ | 5-$CH_3$ | 1 |
| $(CH_2)_2CH_3$ | $CH_2CH_2CH_2CH_2OCH_2$ | 5-$CH_3$ | 1 |
| $CH_2CH_3$ | $CH_2CH_2CH_2CH_2OCH_2$ | 5-$C_2H_5$ | 1 |
| $(CH_2)_2CH_3$ | $CH_2CH_2CH_2CH_2OCH_2$ | 5-$C_2H_5$ | 1 |

Tabelle 6

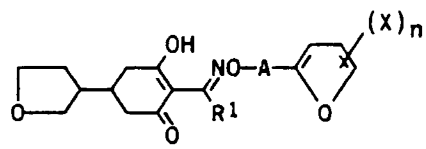

| R1 | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | $5-CH_3$ | 1 |
| $(CH_2)_2CH_3$ | $CH_2CH_2OCH_2$ | $5-CH_3$ | 1 |
| $CH_2CH_3$ | $CH_2CH_2OCH_2$ | $5-C_2H_5$ | 1 |
| $(CH_2)_2CH_3$ | $CH_2CH_2OCH_2$ | $5-C_2H_5$ | 1 |
| $CH_2CH_3$ | $CH_2CH_2CH_2OCH_2$ | - | 0 |
| $(CH_2)_2CH_3$ | $CH_2CH_2CH_2OCH_2$ | - | 0 |
| $CH_2CH_3$ | $CH_2CH_2CH_2OCH_2$ | $5-CH_3$ | 1 |
| $(CH_2)_2CH_3$ | $CH_2CH_2CH_2OCH_2$ | $5-CH_3$ | 1 |
| $CH_2CH_3$ | $CH_2CH_2CH_2OCH_2$ | $5-C_2H_5$ | 1 |
| $(CH_2)_2CH_3$ | $CH_2CH_2CH_2OCH_2$ | $5-C_2H_5$ | 1 |
| $CH_2CH_3$ | $CH_2CH_2CH_2OCH_2CH_2$ | - | 0 |
| $(CH_2)_2CH_3$ | $CH_2CH_2CH_2OCH_2CH_2$ | - | 0 |
| $CH_2CH_3$ | $CH_2CH_2CH_2OCH_2CH_2$ | $5-CH_3$ | 1 |
| $(CH_2)_2CH_3$ | $CH_2CH_2CH_2OCH_2CH_2$ | $5-CH_3$ | 1 |
| $CH_2CH_3$ | $CH_2CH_2CH_2OCH_2CH_2$ | $5-C_2H_5$ | 1 |
| $(CH_2)_2CH_3$ | $CH_2CH_2CH_2OCH_2CH_2$ | $5-C_2H_5$ | 1 |
| $CH_2CH_3$ | $CH_2CH_2CH_2CH_2OCH_2$ | - | 0 |
| $(CH_2)_2CH_3$ | $CH_2CH_2CH_2CH_2OCH_2$ | - | 0 |
| $CH_2CH_3$ | $CH_2CH_2CH_2CH_2OCH_2$ | $5-CH_3$ | 1 |
| $(CH_2)_2CH_3$ | $CH_2CH_2CH_2CH_2OCH_2$ | $5-CH_3$ | 1 |
| $CH_2CH_3$ | $CH_2CH_2CH_2CH_2OCH_2$ | $5-C_2H_5$ | 1 |
| $(CH_2)_2CH_3$ | $CH_2CH_2CH_2CH_2OCH_2$ | $5-C_2H_5$ | 1 |

Tabelle 7

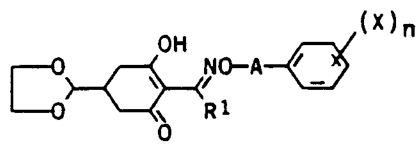

| $R^1$ | A | X | n |
|---|---|---|---|
| $CH_2CH_3$ | $CH_2CH_2O$ | - | 0 |
| $(CH_2)_2CH_3$ | $CH_2CH_2O$ | - | 0 |
| $CH_2CH_3$ | $CH_2CH_2O$ | 2-Cl | 1 |
| $(CH_2)_2CH_3$ | $CH_2CH_2O$ | 2-Cl | 1 |
| $CH_2CH_3$ | $CH_2CH_2O$ | 3-Cl | 1 |
| $(CH_2)_2CH_3$ | $CH_2CH_2O$ | 3-Cl | 1 |
| $CH_2CH_3$ | $CH_2CH_2O$ | 4-Cl | 1 |
| $(CH_2)_2CH_3$ | $CH_2CH_2O$ | 4-Cl | 1 |
| $CH_2CH_3$ | $CH_2CH_2O$ | 2-F | 1 |
| $(CH_2)_2CH_3$ | $CH_2CH_2O$ | 2-F | 1 |
| $CH_2CH_3$ | $CH_2CH_2O$ | 3-F | 1 |
| $(CH_2)_2CH_3$ | $CH_2CH_2O$ | 3-F | 1 |
| $CH_2CH_3$ | $CH_2CH_2O$ | 4-F | 1 |
| $(CH_2)_2CH_3$ | $CH_2CH_2O$ | 4-F | 1 |
| $CH_2CH_3$ | $CH_2CH_2O$ | $3-CF_3$ | 1 |
| $(CH_2)_2CH_3$ | $CH_2CH_2O$ | $3-CF_3$ | 1 |
| $CH_2CH_3$ | $CH_2CH_2CH_2O$ | - | 0 |
| $(CH_2)_2CH_3$ | $CH_2CH_2CH_2O$ | - | 0 |
| $CH_2CH_3$ | $CH_2CH_2CH_2O$ | 4-Cl | 1 |
| $(CH_2)_2CH_3$ | $CH_2CH_2CH_2O$ | 4-Cl | 1 |
| $CH_2CH_3$ | $CH_2CH_2CH_2O$ | 4-F | 1 |
| $(CH_2)_2CH_3$ | $CH_2CH_2CH_2O$ | 4-F | 1 |

Tabelle 7 (Fort.)

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 2-F | 1 |
| $(CH_2)_2CH_3$ | $CH_2CH_2OCH_2$ | 2-F | 1 |
| $CH_2CH_3$ | $CH_2CH_2OCH_2$ | 3-F | 1 |
| $(CH_2)_2CH_3$ | $CH_2CH_2OCH_2$ | 3-F | 1 |
| $CH_2CH_3$ | $CH_2CH_2OCH_2$ | 4-F | 1 |
| $(CH_2)_2CH_3$ | $CH_2CH_2OCH_2$ | 4-F | 1 |
| $CH_2CH_3$ | $CH_2CH_2OCH_2$ | 4-Cl | 1 |
| $(CH_2)_2CH_3$ | $CH_2CH_2OCH_2$ | 4-Cl | 1 |
| $CH_2CH_3$ | $CH_2CH_2OCH_2$ | 2-$CH_3$ | 1 |
| $(CH_2)_2CH_3$ | $CH_2CH_2OCH_2$ | 2-$CH_3$ | 1 |
| $CH_2CH_3$ | $CH_2CH_2OCH_2$ | 3-$CH_3$ | 1 |
| $(CH_2)_2CH_3$ | $CH_2CH_2OCH_2$ | 3-$CH_3$ | 1 |
| $CH_2CH_3$ | $CH_2CH_2OCH_2$ | 4-$CH_3$ | 1 |
| $(CH_2)_2CH_3$ | $CH_2CH_2OCH_2$ | 4-$CH_3$ | 1 |
| $CH_2CH_3$ | $CH_2CH_2OCH_2$ | 4-t$C_4H_9$ | 1 |
| $(CH_2)_2CH_3$ | $CH_2CH_2OCH_2$ | 4-t$C_4H_9$ | 1 |
| $CH_2CH_3$ | $CH_2CH_2SCH_2$ | - | 0 |
| $(CH_2)_2CH_3$ | $CH_2CH_2SCH_2$ | - | 0 |
| $CH_2CH_3$ | $CH_2CH_2SCH_2$ | 4-Cl | 1 |
| $(CH_2)_2CH_3$ | $CH_2CH_2SCH_2$ | 4-Cl | 1 |
| $CH_2CH_3$ | $CH_2CH_2SCH_2$ | 4-F | 1 |
| $(CH_2)_2CH_3$ | $CH_2CH_2SCH_2$ | 4-F | 1 |
| $CH_2CH_3$ | $CH_2CH_2CH_2CH_2O$ | - | 0 |
| $(CH_2)_2CH_3$ | $CH_2CH_2CH_2CH_2O$ | - | 0 |
| $CH_2CH_3$ | $CH_2CH_2CH_2CH_2O$ | 4-Cl | 1 |
| $(CH_2)_2CH_3$ | $CH_2CH_2CH_2CH_2O$ | 4-Cl | 1 |
| $CH_2CH_3$ | $CH_2CH_2CH_2CH_2O$ | 4-F | 1 |
| $(CH_2)_2CH_3$ | $CH_2CH_2CH_2CH_2O$ | 4-F | 1 |

Tabelle 7 (Fort.)

| $R^1$ | A | X | n |
|---|---|---|---|
| $CH_2CH_3$ | $CH_2CH_2CH_2CH_2CH_2O$ | - | 0 |
| $(CH_2)_2CH_3$ | $CH_2CH_2CH_2CH_2CH_2O$ | - | 0 |
| $CH_2CH_3$ | $CH_2CH_2CH_2CH_2CH_2O$ | 4-Cl | 1 |
| $(CH_2)_2CH_3$ | $CH_2CH_2CH_2CH_2CH_2O$ | 4-Cl | 1 |
| $CH_2CH_3$ | $CH_2CH_2CH_2CH_2CH_2O$ | 4-F | 1 |
| $(CH_2)_2CH_3$ | $CH_2CH_2CH_2CH_2CH_2O$ | 4-F | 1 |

Tabelle 8

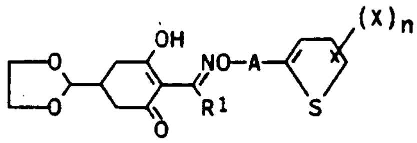

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 5-Cl | 1 |
| $(CH_2)_2CH_3$ | $CH_2CH_2OCH_2$ | 5-Cl | 1 |
| $CH_2CH_3$ | $CH_2CH_2OCH_2$ | 5-$CH_3$ | 1 |
| $(CH_2)_2CH_3$ | $CH_2CH_2OCH_2$ | 5-$CH_3$ | 1 |
| $CH_2CH_3$ | $CH_2CH_2OCH_2$ | 5-$C_2H_5$ | 1 |
| $(CH_2)_2CH_3$ | $CH_2CH_2OCH_2$ | 5-$C_2H_5$ | 1 |
| $CH_2CH_3$ | $CH_2CH_2CH_2OCH_2$ | - | 0 |
| $(CH_2)_2CH_3$ | $CH_2CH_2CH_2OCH_2$ | - | 0 |
| $CH_2CH_3$ | $CH_2CH_2CH_2OCH_2$ | 5-Cl | 1 |
| $(CH_2)_2CH_3$ | $CH_2CH_2CH_2OCH_2$ | 5-Cl | 1 |
| $CH_2CH_3$ | $CH_2CH_2CH_2OCH_2$ | 5-$CH_3$ | 1 |
| $(CH_2)_2CH_3$ | $CH_2CH_2CH_2OCH_2$ | 5-$CH_3$ | 1 |
| $CH_2CH_3$ | $CH_2CH_2CH_2OCH_2$ | 5-$C_2H_5$ | 1 |
| $(CH_2)_2CH_3$ | $CH_2CH_2CH_2OCH_2$ | 5-$C_2H_5$ | 1 |
| $CH_2CH_3$ | $CH_2CH_2CH_2OCH_2CH_2$ | - | 0 |
| $(CH_2)_2CH_3$ | $CH_2CH_2CH_2OCH_2CH_2$ | - | 0 |
| $CH_2CH_3$ | $CH_2CH_2CH_2OCH_2CH_2$ | 5-Cl | 1 |
| $(CH_2)_2CH_3$ | $CH_2CH_2CH_2OCH_2CH_2$ | 5-Cl | 1 |
| $CH_2CH_3$ | $CH_2CH_2CH_2OCH_2CH_2$ | 5-$CH_3$ | 1 |
| $(CH_2)_2CH_3$ | $CH_2CH_2CH_2OCH_2CH_2$ | 5-$CH_3$ | 1 |
| $CH_2CH_3$ | $CH_2CH_2CH_2OCH_2CH_2$ | 5-$C_2H_5$ | 1 |
| $(CH_2)_2CH_3$ | $CH_2CH_2CH_2OCH_2CH_2$ | 5-$C_2H_5$ | 1 |

Tabelle 8 (Fort.)

| $R^1$ | A | X | n |
|---|---|---|---|
| $CH_2CH_3$ | $CH_2CH_2CH_2CH_2OCH_2$ | - | 0 |
| $(CH_2)_2CH_3$ | $CH_2CH_2CH_2CH_2OCH_2$ | - | 0 |
| $CH_2CH_3$ | $CH_2CH_2CH_2CH_2OCH_2$ | 5-Cl | 1 |
| $(CH_2)_2CH_3$ | $CH_2CH_2CH_2CH_2OCH_2$ | 5-Cl | 1 |
| $CH_2CH_3$ | $CH_2CH_2CH_2CH_2OCH_2$ | 5-$CH_3$ | 1 |
| $(CH_2)_2CH_3$ | $CH_2CH_2CH_2CH_2OCH_2$ | 5-$CH_3$ | 1 |
| $CH_2CH_3$ | $CH_2CH_2CH_2CH_2OCH_2$ | 5-$C_2H_5$ | 1 |
| $(CH_2)_2CH_3$ | $CH_2CH_2CH_2CH_2OCH_2$ | 5-$C_2H_5$ | 1 |

Tabelle 9

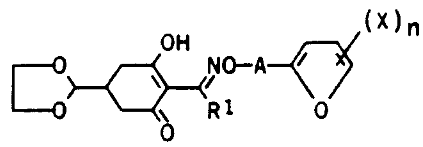

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 5-$CH_3$ | 1 |
| $(CH_2)_2CH_3$ | $CH_2CH_2OCH_2$ | 5-$CH_3$ | 1 |
| $CH_2CH_3$ | $CH_2CH_2OCH_2$ | 5-$C_2H_5$ | 1 |
| $(CH_2)_2CH_3$ | $CH_2CH_2OCH_2$ | 5-$C_2H_5$ | 1 |
| $CH_2CH_3$ | $CH_2CH_2CH_2OCH_2$ | - | 0 |
| $(CH_2)_2CH_3$ | $CH_2CH_2CH_2OCH_2$ | - | 0 |
| $CH_2CH_3$ | $CH_2CH_2CH_2OCH_2$ | 5-$CH_3$ | 1 |
| $(CH_2)_2CH_3$ | $CH_2CH_2CH_2OCH_2$ | 5-$CH_3$ | 1 |
| $CH_2CH_3$ | $CH_2CH_2CH_2OCH_2$ | 5-$C_2H_5$ | 1 |
| $(CH_2)_2CH_3$ | $CH_2CH_2CH_2OCH_2$ | 5-$C_2H_5$ | 1 |
| $CH_2CH_3$ | $CH_2CH_2CH_2OCH_2CH_2$ | - | 0 |
| $(CH_2)_2CH_3$ | $CH_2CH_2CH_2OCH_2CH_2$ | - | 0 |
| $CH_2CH_3$ | $CH_2CH_2CH_2OCH_2CH_2$ | 5-$CH_3$ | 1 |
| $(CH_2)_2CH_3$ | $CH_2CH_2CH_2OCH_2CH_2$ | 5-$CH_3$ | 1 |
| $CH_2CH_3$ | $CH_2CH_2CH_2OCH_2CH_2$ | 5-$C_2H_5$ | 1 |
| $(CH_2)_2CH_3$ | $CH_2CH_2CH_2OCH_2CH_2$ | 5-$C_2H_5$ | 1 |
| $CH_2CH_3$ | $CH_2CH_2CH_2CH_2OCH_2$ | - | 0 |
| $(CH_2)_2CH_3$ | $CH_2CH_2CH_2CH_2OCH_2$ | - | 0 |
| $CH_2CH_3$ | $CH_2CH_2CH_2CH_2OCH_2$ | 5-$CH_3$ | 1 |
| $(CH_2)_2CH_3$ | $CH_2CH_2CH_2CH_2OCH_2$ | 5-$CH_3$ | 1 |
| $CH_2CH_3$ | $CH_2CH_2CH_2CH_2OCH_2$ | 5-$C_2H_5$ | 1 |
| $(CH_2)_2CH_3$ | $CH_2CH_2CH_2CH_2OCH_2$ | 5-$C_2H_5$ | 1 |

Tabelle 10

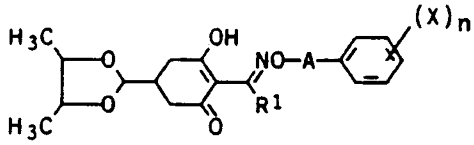

| $R^1$ | A | X | n |
|---|---|---|---|
| $CH_2CH_3$ | $CH_2CH_2O$ | - | 0 |
| $(CH_2)_2CH_3$ | $CH_2CH_2O$ | - | 0 |
| $CH_2CH_3$ | $CH_2CH_2O$ | 2-Cl | 1 |
| $(CH_2)_2CH_3$ | $CH_2CH_2O$ | 2-Cl | 1 |
| $CH_2CH_3$ | $CH_2CH_2O$ | 3-Cl | 1 |
| $(CH_2)_2CH_3$ | $CH_2CH_2O$ | 3-Cl | 1 |
| $CH_2CH_3$ | $CH_2CH_2O$ | 4-Cl | 1 |
| $(CH_2)_2CH_3$ | $CH_2CH_2O$ | 4-Cl | 1 |
| $CH_2CH_3$ | $CH_2CH_2O$ | 2-F | 1 |
| $(CH_2)_2CH_3$ | $CH_2CH_2O$ | 2-F | 1 |
| $CH_2CH_3$ | $CH_2CH_2O$ | 3-F | 1 |
| $(CH_2)_2CH_3$ | $CH_2CH_2O$ | 3-F | 1 |
| $CH_2CH_3$ | $CH_2CH_2O$ | 4-F | 1 |
| $(CH_2)_2CH_3$ | $CH_2CH_2O$ | 4-F | 1 |
| $CH_2CH_3$ | $CH_2CH_2O$ | $3-CF_3$ | 1 |
| $(CH_2)_2CH_3$ | $CH_2CH_2O$ | $3-CF_3$ | 1 |
| $CH_2CH_3$ | $CH_2CH_2CH_2O$ | - | 0 |
| $(CH_2)_2CH_3$ | $CH_2CH_2CH_2O$ | - | 0 |
| $CH_2CH_3$ | $CH_2CH_2CH_2O$ | 4-Cl | 1 |
| $(CH_2)_2CH_3$ | $CH_2CH_2CH_2O$ | 4-Cl | 1 |
| $CH_2CH_3$ | $CH_2CH_2CH_2O$ | 4-F | 1 |
| $(CH_2)_2CH_3$ | $CH_2CH_2CH_2O$ | 4-F | 1 |

Tabelle 10 (Fort.)

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 2-F | 1 |
| $(CH_2)_2CH_3$ | $CH_2CH_2OCH_2$ | 2-F | 1 |
| $CH_2CH_3$ | $CH_2CH_2OCH_2$ | 3-F | 1 |
| $(CH_2)_2CH_3$ | $CH_2CH_2OCH_2$ | 3-F | 1 |
| $CH_2CH_3$ | $CH_2CH_2OCH_2$ | 4-F | 1 |
| $(CH_2)_2CH_3$ | $CH_2CH_2OCH_2$ | 4-F | 1 |
| $CH_2CH_3$ | $CH_2CH_2OCH_2$ | 4-Cl | 1 |
| $(CH_2)_2CH_3$ | $CH_2CH_2OCH_2$ | 4-Cl | 1 |
| $CH_2CH_3$ | $CH_2CH_2OCH_2$ | 2-$CH_3$ | 1 |
| $(CH_2)_2CH_3$ | $CH_2CH_2OCH_2$ | 2-$CH_3$ | 1 |
| $CH_2CH_3$ | $CH_2CH_2OCH_2$ | 3-$CH_3$ | 1 |
| $(CH_2)_2CH_3$ | $CH_2CH_2OCH_2$ | 3-$CH_3$ | 1 |
| $CH_2CH_3$ | $CH_2CH_2OCH_2$ | 4-$CH_3$ | 1 |
| $(CH_2)_2CH_3$ | $CH_2CH_2OCH_2$ | 4-$CH_3$ | 1 |
| $CH_2CH_3$ | $CH_2CH_2OCH_2$ | 4-t$C_4H_9$ | 1 |
| $(CH_2)_2CH_3$ | $CH_2CH_2OCH_2$ | 4-t$C_4H_9$ | 1 |
| $CH_2CH_3$ | $CH_2CH_2SCH_2$ | - | 0 |
| $(CH_2)_2CH_3$ | $CH_2CH_2SCH_2$ | - | 0 |
| $CH_2CH_3$ | $CH_2CH_2SCH_2$ | 4-Cl | 1 |
| $(CH_2)_2CH_3$ | $CH_2CH_2SCH_2$ | 4-Cl | 1 |
| $CH_2CH_3$ | $CH_2CH_2SCH_2$ | 4-F | 1 |
| $(CH_2)_2CH_3$ | $CH_2CH_2SCH_2$ | 4-F | 1 |
| $CH_2CH_3$ | $CH_2CH_2CH_2CH_2O$ | - | 0 |
| $(CH_2)_2CH_3$ | $CH_2CH_2CH_2CH_2O$ | - | 0 |
| $CH_2CH_3$ | $CH_2CH_2CH_2CH_2O$ | 4-Cl | 1 |
| $(CH_2)_2CH_3$ | $CH_2CH_2CH_2CH_2O$ | 4-Cl | 1 |
| $CH_2CH_3$ | $CH_2CH_2CH_2CH_2O$ | 4-F | 1 |
| $(CH_2)_2CH_3$ | $CH_2CH_2CH_2CH_2O$ | 4-F | 1 |

Tabelle 10 (Fort.)

| $R^1$ | A | X | n |
|---|---|---|---|
| $CH_2CH_3$ | $CH_2CH_2CH_2CH_2CH_2O$ | - | 0 |
| $(CH_2)_2CH_3$ | $CH_2CH_2CH_2CH_2CH_2O$ | - | 0 |
| $CH_2CH_3$ | $CH_2CH_2CH_2CH_2CH_2O$ | 4-Cl | 1 |
| $(CH_2)_2CH_3$ | $CH_2CH_2CH_2CH_2CH_2O$ | 4-Cl | 1 |
| $CH_2CH_3$ | $CH_2CH_2CH_2CH_2CH_2O$ | 4-F | 1 |
| $(CH_2)_2CH_3$ | $CH_2CH_2CH_2CH_2CH_2O$ | 4-F | 1 |

Tabelle 11

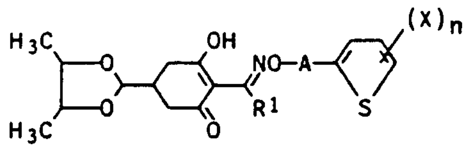

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 5-Cl | 1 |
| $(CH_2)_2CH_3$ | $CH_2CH_2OCH_2$ | 5-Cl | 1 |
| $CH_2CH_3$ | $CH_2CH_2OCH_2$ | $5-CH_3$ | 1 |
| $(CH_2)_2CH_3$ | $CH_2CH_2OCH_2$ | $5-CH_3$ | 1 |
| $CH_2CH_3$ | $CH_2CH_2OCH_2$ | $5-C_2H_5$ | 1 |
| $(CH_2)_2CH_3$ | $CH_2CH_2OCH_2$ | $5-C_2H_5$ | 1 |
| $CH_2CH_3$ | $CH_2CH_2CH_2OCH_2$ | - | 0 |
| $(CH_2)_2CH_3$ | $CH_2CH_2CH_2OCH_2$ | - | 0 |
| $CH_2CH_3$ | $CH_2CH_2CH_2OCH_2$ | 5-Cl | 1 |
| $(CH_2)_2CH_3$ | $CH_2CH_2CH_2OCH_2$ | 5-Cl | 1 |
| $CH_2CH_3$ | $CH_2CH_2CH_2OCH_2$ | $5-CH_3$ | 1 |
| $(CH_2)_2CH_3$ | $CH_2CH_2CH_2OCH_2$ | $5-CH_3$ | 1 |
| $CH_2CH_3$ | $CH_2CH_2CH_2OCH_2$ | $5-C_2H_5$ | 1 |
| $(CH_2)_2CH_3$ | $CH_2CH_2CH_2OCH_2$ | $5-C_2H_5$ | 1 |
| $CH_2CH_3$ | $CH_2CH_2CH_2OCH_2CH_2$ | - | 0 |
| $(CH_2)_2CH_3$ | $CH_2CH_2CH_2OCH_2CH_2$ | - | 0 |
| $CH_2CH_3$ | $CH_2CH_2CH_2OCH_2CH_2$ | 5-Cl | 1 |
| $(CH_2)_2CH_3$ | $CH_2CH_2CH_2OCH_2CH_2$ | 5-Cl | 1 |
| $CH_2CH_3$ | $CH_2CH_2CH_2OCH_2CH_2$ | $5-CH_3$ | 1 |
| $(CH_2)_2CH_3$ | $CH_2CH_2CH_2OCH_2CH_2$ | $5-CH_3$ | 1 |
| $CH_2CH_3$ | $CH_2CH_2CH_2OCH_2CH_2$ | $5-C_2H_5$ | 1 |
| $(CH_2)_2CH_3$ | $CH_2CH_2CH_2OCH_2CH_2$ | $5-C_2H_5$ | 1 |

Tabelle 11 (Fort.)

| $R^1$ | A | X | n |
|---|---|---|---|
| $CH_2CH_3$ | $CH_2CH_2CH_2CH_2OCH_2$ | - | 0 |
| $(CH_2)_2CH_3$ | $CH_2CH_2CH_2CH_2OCH_2$ | - | 0 |
| $CH_2CH_3$ | $CH_2CH_2CH_2CH_2OCH_2$ | 5-Cl | 1 |
| $(CH_2)_2CH_3$ | $CH_2CH_2CH_2CH_2OCH_2$ | 5-Cl | 1 |
| $CH_2CH_3$ | $CH_2CH_2CH_2CH_2OCH_2$ | 5-$CH_3$ | 1 |
| $(CH_2)_2CH_3$ | $CH_2CH_2CH_2CH_2OCH_2$ | 5-$CH_3$ | 1 |
| $CH_2CH_3$ | $CH_2CH_2CH_2CH_2OCH_2$ | 5-$C_2H_5$ | 1 |
| $(CH_2)_2CH_3$ | $CH_2CH_2CH_2CH_2OCH_2$ | 5-$C_2H_5$ | 1 |

Tabelle 12

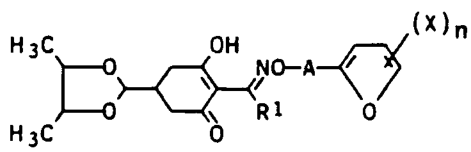

| R1 | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 5-$CH_3$ | 1 |
| $(CH_2)_2CH_3$ | $CH_2CH_2OCH_2$ | 5-$CH_3$ | 1 |
| $CH_2CH_3$ | $CH_2CH_2OCH_2$ | 5-$C_2H_5$ | 1 |
| $(CH_2)_2CH_3$ | $CH_2CH_2OCH_2$ | 5-$C_2H_5$ | 1 |
| $CH_2CH_3$ | $CH_2CH_2CH_2OCH_2$ | - | 0 |
| $(CH_2)_2CH_3$ | $CH_2CH_2CH_2OCH_2$ | - | 0 |
| $CH_2CH_3$ | $CH_2CH_2CH_2OCH_2$ | 5-$CH_3$ | 1 |
| $(CH_2)_2CH_3$ | $CH_2CH_2CH_2OCH_2$ | 5-$CH_3$ | 1 |
| $CH_2CH_3$ | $CH_2CH_2CH_2OCH_2$ | 5-$C_2H_5$ | 1 |
| $(CH_2)_2CH_3$ | $CH_2CH_2CH_2OCH_2$ | 5-$C_2H_5$ | 1 |
| $CH_2CH_3$ | $CH_2CH_2CH_2OCH_2CH_2$ | - | 0 |
| $(CH_2)_2CH_3$ | $CH_2CH_2CH_2OCH_2CH_2$ | - | 0 |
| $CH_2CH_3$ | $CH_2CH_2CH_2OCH_2CH_2$ | 5-$CH_3$ | 1 |
| $(CH_2)_2CH_3$ | $CH_2CH_2CH_2OCH_2CH_2$ | 5-$CH_3$ | 1 |
| $CH_2CH_3$ | $CH_2CH_2CH_2OCH_2CH_2$ | 5-$C_2H_5$ | 1 |
| $(CH_2)_2CH_3$ | $CH_2CH_2CH_2OCH_2CH_2$ | 5-$C_2H_5$ | 1 |
| $CH_2CH_3$ | $CH_2CH_2CH_2CH_2OCH_2$ | - | 0 |
| $(CH_2)_2CH_3$ | $CH_2CH_2CH_2CH_2OCH_2$ | - | 0 |
| $CH_2CH_3$ | $CH_2CH_2CH_2CH_2OCH_2$ | 5-$CH_3$ | 1 |
| $(CH_2)_2CH_3$ | $CH_2CH_2CH_2CH_2OCH_2$ | 5-$CH_3$ | 1 |
| $CH_2CH_3$ | $CH_2CH_2CH_2CH_2OCH_2$ | 5-$C_2H_5$ | 1 |
| $(CH_2)_2CH_3$ | $CH_2CH_2CH_2CH_2OCH_2$ | 5-$C_2H_5$ | 1 |

Tabelle 13

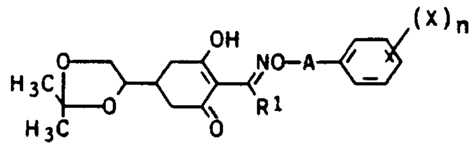

| $R^1$ | A | X | n |
|---|---|---|---|
| $CH_2CH_3$ | $CH_2CH_2O$ | - | 0 |
| $(CH_2)_2CH_3$ | $CH_2CH_2O$ | - | 0 |
| $CH_2CH_3$ | $CH_2CH_2O$ | 2-Cl | 1 |
| $(CH_2)_2CH_3$ | $CH_2CH_2O$ | 2-Cl | 1 |
| $CH_2CH_3$ | $CH_2CH_2O$ | 3-Cl | 1 |
| $(CH_2)_2CH_3$ | $CH_2CH_2O$ | 3-Cl | 1 |
| $CH_2CH_3$ | $CH_2CH_2O$ | 4-Cl | 1 |
| $(CH_2)_2CH_3$ | $CH_2CH_2O$ | 4-Cl | 1 |
| $CH_2CH_3$ | $CH_2CH_2O$ | 2-F | 1 |
| $(CH_2)_2CH_3$ | $CH_2CH_2O$ | 2-F | 1 |
| $CH_2CH_3$ | $CH_2CH_2O$ | 3-F | 1 |
| $(CH_2)_2CH_3$ | $CH_2CH_2O$ | 3-F | 1 |
| $CH_2CH_3$ | $CH_2CH_2O$ | 4-F | 1 |
| $(CH_2)_2CH_3$ | $CH_2CH_2O$ | 4-F | 1 |
| $CH_2CH_3$ | $CH_2CH_2O$ | $3-CF_3$ | 1 |
| $(CH_2)_2CH_3$ | $CH_2CH_2O$ | $3-CF_3$ | 1 |
| $CH_2CH_3$ | $CH_2CH_2CH_2O$ | - | 0 |
| $(CH_2)_2CH_3$ | $CH_2CH_2CH_2O$ | - | 0 |
| $CH_2CH_3$ | $CH_2CH_2CH_2O$ | 4-Cl | 1 |
| $(CH_2)_2CH_3$ | $CH_2CH_2CH_2O$ | 4-Cl | 1 |
| $CH_2CH_3$ | $CH_2CH_2CH_2O$ | 4-F | 1 |
| $(CH_2)_2CH_3$ | $CH_2CH_2CH_2O$ | 4-F | 1 |

Tabelle 13 (Fort.)

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 2-F | 1 |
| $(CH_2)_2CH_3$ | $CH_2CH_2OCH_2$ | 2-F | 1 |
| $CH_2CH_3$ | $CH_2CH_2OCH_2$ | 3-F | 1 |
| $(CH_2)_2CH_3$ | $CH_2CH_2OCH_2$ | 3-F | 1 |
| $CH_2CH_3$ | $CH_2CH_2OCH_2$ | 4-F | 1 |
| $(CH_2)_2CH_3$ | $CH_2CH_2OCH_2$ | 4-F | 1 |
| $CH_2CH_3$ | $CH_2CH_2OCH_2$ | 4-Cl | 1 |
| $(CH_2)_2CH_3$ | $CH_2CH_2OCH_2$ | 4-Cl | 1 |
| $CH_2CH_3$ | $CH_2CH_2OCH_2$ | 2-$CH_3$ | 1 |
| $(CH_2)_2CH_3$ | $CH_2CH_2OCH_2$ | 2-$CH_3$ | 1 |
| $CH_2CH_3$ | $CH_2CH_2OCH_2$ | 3-$CH_3$ | 1 |
| $(CH_2)_2CH_3$ | $CH_2CH_2OCH_2$ | 3-$CH_3$ | 1 |
| $CH_2CH_3$ | $CH_2CH_2OCH_2$ | 4-$CH_3$ | 1 |
| $(CH_2)_2CH_3$ | $CH_2CH_2OCH_2$ | 4-$CH_3$ | 1 |
| $CH_2CH_3$ | $CH_2CH_2OCH_2$ | 4-t$C_4H_9$ | 1 |
| $(CH_2)_2CH_3$ | $CH_2CH_2OCH_2$ | 4-t$C_4H_9$ | 1 |
| $CH_2CH_3$ | $CH_2CH_2SCH_2$ | - | 0 |
| $(CH_2)_2CH_3$ | $CH_2CH_2SCH_2$ | - | 0 |
| $CH_2CH_3$ | $CH_2CH_2SCH_2$ | 4-Cl | 1 |
| $(CH_2)_2CH_3$ | $CH_2CH_2SCH_2$ | 4-Cl | 1 |
| $CH_2CH_3$ | $CH_2CH_2SCH_2$ | 4-F | 1 |
| $(CH_2)_2CH_3$ | $CH_2CH_2SCH_2$ | 4-F | 1 |
| $CH_2CH_3$ | $CH_2CH_2CH_2CH_2O$ | - | 0 |
| $(CH_2)_2CH_3$ | $CH_2CH_2CH_2CH_2O$ | - | 0 |
| $CH_2CH_3$ | $CH_2CH_2CH_2CH_2O$ | 4-Cl | 1 |
| $(CH_2)_2CH_3$ | $CH_2CH_2CH_2CH_2O$ | 4-Cl | 1 |
| $CH_2CH_3$ | $CH_2CH_2CH_2CH_2O$ | 4-F | 1 |
| $(CH_2)_2CH_3$ | $CH_2CH_2CH_2CH_2O$ | 4-F | 1 |

Tabelle 13 (Fort.)

| $R^1$ | A | X | n |
|---|---|---|---|
| $CH_2CH_3$ | $CH_2CH_2CH_2CH_2CH_2O$ | - | 0 |
| $(CH_2)_2CH_3$ | $CH_2CH_2CH_2CH_2CH_2O$ | - | 0 |
| $CH_2CH_3$ | $CH_2CH_2CH_2CH_2CH_2O$ | 4-Cl | 1 |
| $(CH_2)_2CH_3$ | $CH_2CH_2CH_2CH_2CH_2O$ | 4-Cl | 1 |
| $CH_2CH_3$ | $CH_2CH_2CH_2CH_2CH_2O$ | 4-F | 1 |
| $(CH_2)_2CH_3$ | $CH_2CH_2CH_2CH_2CH_2O$ | 4-F | 1 |

Tabelle 14

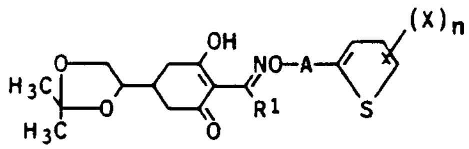

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 5-Cl | 1 |
| $(CH_2)_2CH_3$ | $CH_2CH_2OCH_2$ | 5-Cl | 1 |
| $CH_2CH_3$ | $CH_2CH_2OCH_2$ | 5-$CH_3$ | 1 |
| $(CH_2)_2CH_3$ | $CH_2CH_2OCH_2$ | 5-$CH_3$ | 1 |
| $CH_2CH_3$ | $CH_2CH_2OCH_2$ | 5-$C_2H_5$ | 1 |
| $(CH_2)_2CH_3$ | $CH_2CH_2OCH_2$ | 5-$C_2H_5$ | 1 |
| $CH_2CH_3$ | $CH_2CH_2CH_2OCH_2$ | - | 0 |
| $(CH_2)_2CH_3$ | $CH_2CH_2CH_2OCH_2$ | - | 0 |
| $CH_2CH_3$ | $CH_2CH_2CH_2OCH_2$ | 5-Cl | 1 |
| $(CH_2)_2CH_3$ | $CH_2CH_2CH_2OCH_2$ | 5-Cl | 1 |
| $CH_2CH_3$ | $CH_2CH_2CH_2OCH_2$ | 5-$CH_3$ | 1 |
| $(CH_2)_2CH_3$ | $CH_2CH_2CH_2OCH_2$ | 5-$CH_3$ | 1 |
| $CH_2CH_3$ | $CH_2CH_2CH_2OCH_2$ | 5-$C_2H_5$ | 1 |
| $(CH_2)_2CH_3$ | $CH_2CH_2CH_2OCH_2$ | 5-$C_2H_5$ | 1 |
| $CH_2CH_3$ | $CH_2CH_2CH_2OCH_2CH_2$ | - | 0 |
| $(CH_2)_2CH_3$ | $CH_2CH_2CH_2OCH_2CH_2$ | - | 0 |
| $CH_2CH_3$ | $CH_2CH_2CH_2OCH_2CH_2$ | 5-Cl | 1 |
| $(CH_2)_2CH_3$ | $CH_2CH_2CH_2OCH_2CH_2$ | 5-Cl | 1 |
| $CH_2CH_3$ | $CH_2CH_2CH_2OCH_2CH_2$ | 5-$CH_3$ | 1 |
| $(CH_2)_2CH_3$ | $CH_2CH_2CH_2OCH_2CH_2$ | 5-$CH_3$ | 1 |
| $CH_2CH_3$ | $CH_2CH_2CH_2OCH_2CH_2$ | 5-$C_2H_5$ | 1 |
| $(CH_2)_2CH_3$ | $CH_2CH_2CH_2OCH_2CH_2$ | 5-$C_2H_5$ | 1 |

Tabelle 14 (Fort.)

| $R^1$ | A | X | n |
|---|---|---|---|
| $CH_2CH_3$ | $CH_2CH_2CH_2CH_2OCH_2$ | - | 0 |
| $(CH_2)_2CH_3$ | $CH_2CH_2CH_2CH_2OCH_2$ | - | 0 |
| $CH_2CH_3$ | $CH_2CH_2CH_2CH_2OCH_2$ | 5-Cl | 1 |
| $(CH_2)_2CH_3$ | $CH_2CH_2CH_2CH_2OCH_2$ | 5-Cl | 1 |
| $CH_2CH_3$ | $CH_2CH_2CH_2CH_2OCH_2$ | 5-$CH_3$ | 1 |
| $(CH_2)_2CH_3$ | $CH_2CH_2CH_2CH_2OCH_2$ | 5-$CH_3$ | 1 |
| $CH_2CH_3$ | $CH_2CH_2CH_2CH_2OCH_2$ | 5-$C_2H_5$ | 1 |
| $(CH_2)_2CH_3$ | $CH_2CH_2CH_2CH_2OCH_2$ | 5-$C_2H_5$ | 1 |

Tabelle 15

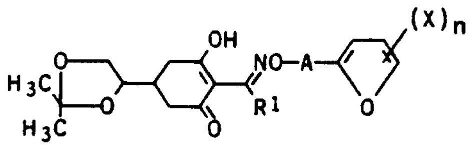

| R1 | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 5-$CH_3$ | 1 |
| $(CH_2)_2CH_3$ | $CH_2CH_2OCH_2$ | 5-$CH_3$ | 1 |
| $CH_2CH_3$ | $CH_2CH_2OCH_2$ | 5-$C_2H_5$ | 1 |
| $(CH_2)_2CH_3$ | $CH_2CH_2OCH_2$ | 5-$C_2H_5$ | 1 |
| $CH_2CH_3$ | $CH_2CH_2CH_2OCH_2$ | - | 0 |
| $(CH_2)_2CH_3$ | $CH_2CH_2CH_2OCH_2$ | - | 0 |
| $CH_2CH_3$ | $CH_2CH_2CH_2OCH_2$ | 5-$CH_3$ | 1 |
| $(CH_2)_2CH_3$ | $CH_2CH_2CH_2OCH_2$ | 5-$CH_3$ | 1 |
| $CH_2CH_3$ | $CH_2CH_2CH_2OCH_2$ | 5-$C_2H_5$ | 1 |
| $(CH_2)_2CH_3$ | $CH_2CH_2CH_2OCH_2$ | 5-$C_2H_5$ | 1 |
| $CH_2CH_3$ | $CH_2CH_2CH_2OCH_2CH_2$ | - | 0 |
| $(CH_2)_2CH_3$ | $CH_2CH_2CH_2OCH_2CH_2$ | - | 0 |
| $CH_2CH_3$ | $CH_2CH_2CH_2OCH_2CH_2$ | 5-$CH_3$ | 1 |
| $(CH_2)_2CH_3$ | $CH_2CH_2CH_2OCH_2CH_2$ | 5-$CH_3$ | 1 |
| $CH_2CH_3$ | $CH_2CH_2CH_2OCH_2CH_2$ | 5-$C_2H_5$ | 1 |
| $(CH_2)_2CH_3$ | $CH_2CH_2CH_2OCH_2CH_2$ | 5-$C_2H_5$ | 1 |
| $CH_2CH_3$ | $CH_2CH_2CH_2CH_2OCH_2$ | - | 0 |
| $(CH_2)_2CH_3$ | $CH_2CH_2CH_2CH_2OCH_2$ | - | 0 |
| $CH_2CH_3$ | $CH_2CH_2CH_2CH_2OCH_2$ | 5-$CH_3$ | 1 |
| $(CH_2)_2CH_3$ | $CH_2CH_2CH_2CH_2OCH_2$ | 5-$CH_3$ | 1 |
| $CH_2CH_3$ | $CH_2CH_2CH_2CH_2OCH_2$ | 5-$C_2H_5$ | 1 |
| $(CH_2)_2CH_3$ | $CH_2CH_2CH_2CH_2OCH_2$ | 5-$C_2H_5$ | 1 |

Tabelle 16

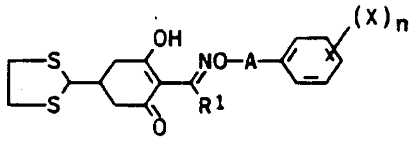

| $R^1$ | A | X | n |
|---|---|---|---|
| $CH_2CH_3$ | $CH_2CH_2O$ | - | 0 |
| $(CH_2)_2CH_3$ | $CH_2CH_2O$ | - | 0 |
| $CH_2CH_3$ | $CH_2CH_2O$ | 2-Cl | 1 |
| $(CH_2)_2CH_3$ | $CH_2CH_2O$ | 2-Cl | 1 |
| $CH_2CH_3$ | $CH_2CH_2O$ | 3-Cl | 1 |
| $(CH_2)_2CH_3$ | $CH_2CH_2O$ | 3-Cl | 1 |
| $CH_2CH_3$ | $CH_2CH_2O$ | 4-Cl | 1 |
| $(CH_2)_2CH_3$ | $CH_2CH_2O$ | 4-Cl | 1 |
| $CH_2CH_3$ | $CH_2CH_2O$ | 2-F | 1 |
| $(CH_2)_2CH_3$ | $CH_2CH_2O$ | 2-F | 1 |
| $CH_2CH_3$ | $CH_2CH_2O$ | 3-F | 1 |
| $(CH_2)_2CH_3$ | $CH_2CH_2O$ | 3-F | 1 |
| $CH_2CH_3$ | $CH_2CH_2O$ | 4-F | 1 |
| $(CH_2)_2CH_3$ | $CH_2CH_2O$ | 4-F | 1 |
| $CH_2CH_3$ | $CH_2CH_2O$ | $3-CF_3$ | 1 |
| $(CH_2)_2CH_3$ | $CH_2CH_2O$ | $3-CF_3$ | 1 |
| $CH_2CH_3$ | $CH_2CH_2CH_2O$ | - | 0 |
| $(CH_2)_2CH_3$ | $CH_2CH_2CH_2O$ | - | 0 |
| $CH_2CH_3$ | $CH_2CH_2CH_2O$ | 4-Cl | 1 |
| $(CH_2)_2CH_3$ | $CH_2CH_2CH_2O$ | 4-Cl | 1 |
| $CH_2CH_3$ | $CH_2CH_2CH_2O$ | 4-F | 1 |
| $(CH_2)_2CH_3$ | $CH_2CH_2CH_2O$ | 4-F | 1 |

Tabelle 16 (Fort.)

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 2-F | 1 |
| $(CH_2)_2CH_3$ | $CH_2CH_2OCH_2$ | 2-F | 1 |
| $CH_2CH_3$ | $CH_2CH_2OCH_2$ | 3-F | 1 |
| $(CH_2)_2CH_3$ | $CH_2CH_2OCH_2$ | 3-F | 1 |
| $CH_2CH_3$ | $CH_2CH_2OCH_2$ | 4-F | 1 |
| $(CH_2)_2CH_3$ | $CH_2CH_2OCH_2$ | 4-F | 1 |
| $CH_2CH_3$ | $CH_2CH_2OCH_2$ | 4-Cl | 1 |
| $(CH_2)_2CH_3$ | $CH_2CH_2OCH_2$ | 4-Cl | 1 |
| $CH_2CH_3$ | $CH_2CH_2OCH_2$ | 2-$CH_3$ | 1 |
| $(CH_2)_2CH_3$ | $CH_2CH_2OCH_2$ | 2-$CH_3$ | 1 |
| $CH_2CH_3$ | $CH_2CH_2OCH_2$ | 3-$CH_3$ | 1 |
| $(CH_2)_2CH_3$ | $CH_2CH_2OCH_2$ | 3-$CH_3$ | 1 |
| $CH_2CH_3$ | $CH_2CH_2OCH_2$ | 4-$CH_3$ | 1 |
| $(CH_2)_2CH_3$ | $CH_2CH_2OCH_2$ | 4-$CH_3$ | 1 |
| $CH_2CH_3$ | $CH_2CH_2OCH_2$ | 4-t$C_4H_9$ | 1 |
| $(CH_2)_2CH_3$ | $CH_2CH_2OCH_2$ | 4-t$C_4H_9$ | 1 |
| $CH_2CH_3$ | $CH_2CH_2SCH_2$ | - | 0 |
| $(CH_2)_2CH_3$ | $CH_2CH_2SCH_2$ | - | 0 |
| $CH_2CH_3$ | $CH_2CH_2SCH_2$ | 4-Cl | 1 |
| $(CH_2)_2CH_3$ | $CH_2CH_2SCH_2$ | 4-Cl | 1 |
| $CH_2CH_3$ | $CH_2CH_2SCH_2$ | 4-F | 1 |
| $(CH_2)_2CH_3$ | $CH_2CH_2SCH_2$ | 4-F | 1 |
| $CH_2CH_3$ | $CH_2CH_2CH_2CH_2O$ | - | 0 |
| $(CH_2)_2CH_3$ | $CH_2CH_2CH_2CH_2O$ | - | 0 |
| $CH_2CH_3$ | $CH_2CH_2CH_2CH_2O$ | 4-Cl | 1 |
| $(CH_2)_2CH_3$ | $CH_2CH_2CH_2CH_2O$ | 4-Cl | 1 |
| $CH_2CH_3$ | $CH_2CH_2CH_2CH_2O$ | 4-F | 1 |
| $(CH_2)_2CH_3$ | $CH_2CH_2CH_2CH_2O$ | 4-F | 1 |

Tabelle 16 (Fort.)

| $R^1$ | A | X | n |
|---|---|---|---|
| $CH_2CH_3$ | $CH_2CH_2CH_2CH_2CH_2O$ | - | 0 |
| $(CH_2)_2CH_3$ | $CH_2CH_2CH_2CH_2CH_2O$ | - | 0 |
| $CH_2CH_3$ | $CH_2CH_2CH_2CH_2CH_2O$ | 4-Cl | 1 |
| $(CH_2)_2CH_3$ | $CH_2CH_2CH_2CH_2CH_2O$ | 4-Cl | 1 |
| $CH_2CH_3$ | $CH_2CH_2CH_2CH_2CH_2O$ | 4-F | 1 |
| $(CH_2)_2CH_3$ | $CH_2CH_2CH_2CH_2CH_2O$ | 4-F | 1 |

Tabelle 17

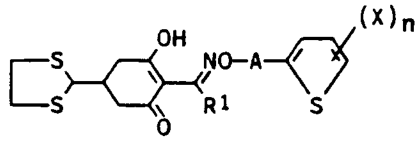

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 5-Cl | 1 |
| $(CH_2)_2CH_3$ | $CH_2CH_2OCH_2$ | 5-Cl | 1 |
| $CH_2CH_3$ | $CH_2CH_2OCH_2$ | $5-CH_3$ | 1 |
| $(CH_2)_2CH_3$ | $CH_2CH_2OCH_2$ | $5-CH_3$ | 1 |
| $CH_2CH_3$ | $CH_2CH_2OCH_2$ | $5-C_2H_5$ | 1 |
| $(CH_2)_2CH_3$ | $CH_2CH_2OCH_2$ | $5-C_2H_5$ | 1 |
| $CH_2CH_3$ | $CH_2CH_2CH_2OCH_2$ | - | 0 |
| $(CH_2)_2CH_3$ | $CH_2CH_2CH_2OCH_2$ | - | 0 |
| $CH_2CH_3$ | $CH_2CH_2CH_2OCH_2$ | 5-Cl | 1 |
| $(CH_2)_2CH_3$ | $CH_2CH_2CH_2OCH_2$ | 5-Cl | 1 |
| $CH_2CH_3$ | $CH_2CH_2CH_2OCH_2$ | $5-CH_3$ | 1 |
| $(CH_2)_2CH_3$ | $CH_2CH_2CH_2OCH_2$ | $5-CH_3$ | 1 |
| $CH_2CH_3$ | $CH_2CH_2CH_2OCH_2$ | $5-C_2H_5$ | 1 |
| $(CH_2)_2CH_3$ | $CH_2CH_2CH_2OCH_2$ | $5-C_2H_5$ | 1 |
| $CH_2CH_3$ | $CH_2CH_2CH_2OCH_2CH_2$ | - | 0 |
| $(CH_2)_2CH_3$ | $CH_2CH_2CH_2OCH_2CH_2$ | - | 0 |
| $CH_2CH_3$ | $CH_2CH_2CH_2OCH_2CH_2$ | 5-Cl | 1 |
| $(CH_2)_2CH_3$ | $CH_2CH_2CH_2OCH_2CH_2$ | 5-Cl | 1 |
| $CH_2CH_3$ | $CH_2CH_2CH_2OCH_2CH_2$ | $5-CH_3$ | 1 |
| $(CH_2)_2CH_3$ | $CH_2CH_2CH_2OCH_2CH_2$ | $5-CH_3$ | 1 |
| $CH_2CH_3$ | $CH_2CH_2CH_2OCH_2CH_2$ | $5-C_2H_5$ | 1 |
| $(CH_2)_2CH_3$ | $CH_2CH_2CH_2OCH_2CH_2$ | $5-C_2H_5$ | 1 |

Tabelle 17 (Fort.)

| $R^1$ | A | X | n |
|---|---|---|---|
| $CH_2CH_3$ | $CH_2CH_2CH_2CH_2OCH_2$ | - | 0 |
| $(CH_2)_2CH_3$ | $CH_2CH_2CH_2CH_2OCH_2$ | - | 0 |
| $CH_2CH_3$ | $CH_2CH_2CH_2CH_2OCH_2$ | 5-Cl | 1 |
| $(CH_2)_2CH_3$ | $CH_2CH_2CH_2CH_2OCH_2$ | 5-Cl | 1 |
| $CH_2CH_3$ | $CH_2CH_2CH_2CH_2OCH_2$ | 5-$CH_3$ | 1 |
| $(CH_2)_2CH_3$ | $CH_2CH_2CH_2CH_2OCH_2$ | 5-$CH_3$ | 1 |
| $CH_2CH_3$ | $CH_2CH_2CH_2CH_2OCH_2$ | 5-$C_2H_5$ | 1 |
| $(CH_2)_2CH_3$ | $CH_2CH_2CH_2CH_2OCH_2$ | 5-$C_2H_5$ | 1 |

Tabelle 18

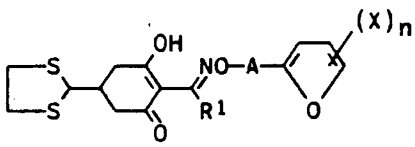

| R1 | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 5-$CH_3$ | 1 |
| $(CH_2)_2CH_3$ | $CH_2CH_2OCH_2$ | 5-$CH_3$ | 1 |
| $CH_2CH_3$ | $CH_2CH_2OCH_2$ | 5-$C_2H_5$ | 1 |
| $(CH_2)_2CH_3$ | $CH_2CH_2OCH_2$ | 5-$C_2H_5$ | 1 |
| $CH_2CH_3$ | $CH_2CH_2CH_2OCH_2$ | - | 0 |
| $(CH_2)_2CH_3$ | $CH_2CH_2CH_2OCH_2$ | - | 0 |
| $CH_2CH_3$ | $CH_2CH_2CH_2OCH_2$ | 5-$CH_3$ | 1 |
| $(CH_2)_2CH_3$ | $CH_2CH_2CH_2OCH_2$ | 5-$CH_3$ | 1 |
| $CH_2CH_3$ | $CH_2CH_2CH_2OCH_2$ | 5-$C_2H_5$ | 1 |
| $(CH_2)_2CH_3$ | $CH_2CH_2CH_2OCH_2$ | 5-$C_2H_5$ | 1 |
| $CH_2CH_3$ | $CH_2CH_2CH_2OCH_2CH_2$ | - | 0 |
| $(CH_2)_2CH_3$ | $CH_2CH_2CH_2OCH_2CH_2$ | - | 0 |
| $CH_2CH_3$ | $CH_2CH_2CH_2OCH_2CH_2$ | 5-$CH_3$ | 1 |
| $(CH_2)_2CH_3$ | $CH_2CH_2CH_2OCH_2CH_2$ | 5-$CH_3$ | 1 |
| $CH_2CH_3$ | $CH_2CH_2CH_2OCH_2CH_2$ | 5-$C_2H_5$ | 1 |
| $(CH_2)_2CH_3$ | $CH_2CH_2CH_2OCH_2CH_2$ | 5-$C_2H_5$ | 1 |
| $CH_2CH_3$ | $CH_2CH_2CH_2CH_2OCH_2$ | - | 0 |
| $(CH_2)_2CH_3$ | $CH_2CH_2CH_2CH_2OCH_2$ | - | 0 |
| $CH_2CH_3$ | $CH_2CH_2CH_2CH_2OCH_2$ | 5-$CH_3$ | 1 |
| $(CH_2)_2CH_3$ | $CH_2CH_2CH_2CH_2OCH_2$ | 5-$CH_3$ | 1 |
| $CH_2CH_3$ | $CH_2CH_2CH_2CH_2OCH_2$ | 5-$C_2H_5$ | 1 |
| $(CH_2)_2CH_3$ | $CH_2CH_2CH_2CH_2OCH_2$ | 5-$C_2H_5$ | 1 |

Tabelle 19

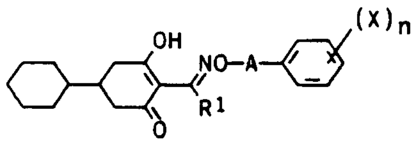

| R1 | A | X | n |
|---|---|---|---|
| $CH_2CH_3$ | $CH_2CH_2O$ | - | 0 |
| $(CH_2)_2CH_3$ | $CH_2CH_2O$ | - | 0 |
| $CH_2CH_3$ | $CH_2CH_2O$ | 2-Cl | 1 |
| $(CH_2)_2CH_3$ | $CH_2CH_2O$ | 2-Cl | 1 |
| $CH_2CH_3$ | $CH_2CH_2O$ | 3-Cl | 1 |
| $(CH_2)_2CH_3$ | $CH_2CH_2O$ | 3-Cl | 1 |
| $CH_2CH_3$ | $CH_2CH_2O$ | 4-Cl | 1 |
| $(CH_2)_2CH_3$ | $CH_2CH_2O$ | 4-Cl | 1 |
| $CH_2CH_3$ | $CH_2CH_2O$ | 2-F | 1 |
| $(CH_2)_2CH_3$ | $CH_2CH_2O$ | 2-F | 1 |
| $CH_2CH_3$ | $CH_2CH_2O$ | 3-F | 1 |
| $(CH_2)_2CH_3$ | $CH_2CH_2O$ | 3-F | 1 |
| $CH_2CH_3$ | $CH_2CH_2O$ | 4-F | 1 |
| $(CH_2)_2CH_3$ | $CH_2CH_2O$ | 4-F | 1 |
| $CH_2CH_3$ | $CH_2CH_2O$ | $3-CF_3$ | 1 |
| $(CH_2)_2CH_3$ | $CH_2CH_2O$ | $3-CF_3$ | 1 |
| $CH_2CH_3$ | $CH_2CH_2CH_2O$ | - | 0 |
| $(CH_2)_2CH_3$ | $CH_2CH_2CH_2O$ | - | 0 |
| $CH_2CH_3$ | $CH_2CH_2CH_2O$ | 4-Cl | 1 |
| $(CH_2)_2CH_3$ | $CH_2CH_2CH_2O$ | 4-Cl | 1 |
| $CH_2CH_3$ | $CH_2CH_2CH_2O$ | 4-F | 1 |
| $(CH_2)_2CH_3$ | $CH_2CH_2CH_2O$ | 4-F | 1 |

Tabelle 19 (Fort.)

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 2-F | 1 |
| $(CH_2)_2CH_3$ | $CH_2CH_2OCH_2$ | 2-F | 1 |
| $CH_2CH_3$ | $CH_2CH_2OCH_2$ | 3-F | 1 |
| $(CH_2)_2CH_3$ | $CH_2CH_2OCH_2$ | 3-F | 1 |
| $CH_2CH_3$ | $CH_2CH_2OCH_2$ | 4-F | 1 |
| $(CH_2)_2CH_3$ | $CH_2CH_2OCH_2$ | 4-F | 1 |
| $CH_2CH_3$ | $CH_2CH_2OCH_2$ | 4-Cl | 1 |
| $(CH_2)_2CH_3$ | $CH_2CH_2OCH_2$ | 4-Cl | 1 |
| $CH_2CH_3$ | $CH_2CH_2OCH_2$ | 2-$CH_3$ | 1 |
| $(CH_2)_2CH_3$ | $CH_2CH_2OCH_2$ | 2-$CH_3$ | 1 |
| $CH_2CH_3$ | $CH_2CH_2OCH_2$ | 3-$CH_3$ | 1 |
| $(CH_2)_2CH_3$ | $CH_2CH_2OCH_2$ | 3-$CH_3$ | 1 |
| $CH_2CH_3$ | $CH_2CH_2OCH_2$ | 4-$CH_3$ | 1 |
| $(CH_2)_2CH_3$ | $CH_2CH_2OCH_2$ | 4-$CH_3$ | 1 |
| $CH_2CH_3$ | $CH_2CH_2OCH_2$ | 4-t$C_4H_9$ | 1 |
| $(CH_2)_2CH_3$ | $CH_2CH_2OCH_2$ | 4-t$C_4H_9$ | 1 |
| $CH_2CH_3$ | $CH_2CH_2SCH_2$ | - | 0 |
| $(CH_2)_2CH_3$ | $CH_2CH_2SCH_2$ | - | 0 |
| $CH_2CH_3$ | $CH_2CH_2SCH_2$ | 4-Cl | 1 |
| $(CH_2)_2CH_3$ | $CH_2CH_2SCH_2$ | 4-Cl | 1 |
| $CH_2CH_3$ | $CH_2CH_2SCH_2$ | 4-F | 1 |
| $(CH_2)_2CH_3$ | $CH_2CH_2SCH_2$ | 4-F | 1 |
| $CH_2CH_3$ | $CH_2CH_2CH_2CH_2O$ | - | 0 |
| $(CH_2)_2CH_3$ | $CH_2CH_2CH_2CH_2O$ | - | 0 |
| $CH_2CH_3$ | $CH_2CH_2CH_2CH_2O$ | 4-Cl | 1 |
| $(CH_2)_2CH_3$ | $CH_2CH_2CH_2CH_2O$ | 4-Cl | 1 |
| $CH_2CH_3$ | $CH_2CH_2CH_2CH_2O$ | 4-F | 1 |
| $(CH_2)_2CH_3$ | $CH_2CH_2CH_2CH_2O$ | 4-F | 1 |

Tabelle 19 (Fort.)

| $R^1$ | A | X | n |
|---|---|---|---|
| $CH_2CH_3$ | $CH_2CH_2CH_2CH_2CH_2O$ | - | 0 |
| $(CH_2)_2CH_3$ | $CH_2CH_2CH_2CH_2CH_2O$ | - | 0 |
| $CH_2CH_3$ | $CH_2CH_2CH_2CH_2CH_2O$ | 4-Cl | 1 |
| $(CH_2)_2CH_3$ | $CH_2CH_2CH_2CH_2CH_2O$ | 4-Cl | 1 |
| $CH_2CH_3$ | $CH_2CH_2CH_2CH_2CH_2O$ | 4-F | 1 |
| $(CH_2)_2CH_3$ | $CH_2CH_2CH_2CH_2CH_2O$ | 4-F | 1 |

Tabelle 20

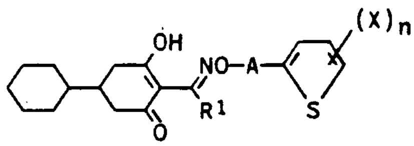

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 5-Cl | 1 |
| $(CH_2)_2CH_3$ | $CH_2CH_2OCH_2$ | 5-Cl | 1 |
| $CH_2CH_3$ | $CH_2CH_2OCH_2$ | 5-$CH_3$ | 1 |
| $(CH_2)_2CH_3$ | $CH_2CH_2OCH_2$ | 5-$CH_3$ | 1 |
| $CH_2CH_3$ | $CH_2CH_2OCH_2$ | 5-$C_2H_5$ | 1 |
| $(CH_2)_2CH_3$ | $CH_2CH_2OCH_2$ | 5-$C_2H_5$ | 1 |
| $CH_2CH_3$ | $CH_2CH_2CH_2OCH_2$ | - | 0 |
| $(CH_2)_2CH_3$ | $CH_2CH_2CH_2OCH_2$ | - | 0 |
| $CH_2CH_3$ | $CH_2CH_2CH_2OCH_2$ | 5-Cl | 1 |
| $(CH_2)_2CH_3$ | $CH_2CH_2CH_2OCH_2$ | 5-Cl | 1 |
| $CH_2CH_3$ | $CH_2CH_2CH_2OCH_2$ | 5-$CH_3$ | 1 |
| $(CH_2)_2CH_3$ | $CH_2CH_2CH_2OCH_2$ | 5-$CH_3$ | 1 |
| $CH_2CH_3$ | $CH_2CH_2CH_2OCH_2$ | 5-$C_2H_5$ | 1 |
| $(CH_2)_2CH_3$ | $CH_2CH_2CH_2OCH_2$ | 5-$C_2H_5$ | 1 |
| $CH_2CH_3$ | $CH_2CH_2CH_2OCH_2CH_2$ | - | 0 |
| $(CH_2)_2CH_3$ | $CH_2CH_2CH_2OCH_2CH_2$ | - | 0 |
| $CH_2CH_3$ | $CH_2CH_2CH_2OCH_2CH_2$ | 5-Cl | 1 |
| $(CH_2)_2CH_3$ | $CH_2CH_2CH_2OCH_2CH_2$ | 5-Cl | 1 |
| $CH_2CH_3$ | $CH_2CH_2CH_2OCH_2CH_2$ | 5-$CH_3$ | 1 |
| $(CH_2)_2CH_3$ | $CH_2CH_2CH_2OCH_2CH_2$ | 5-$CH_3$ | 1 |
| $CH_2CH_3$ | $CH_2CH_2CH_2OCH_2CH_2$ | 5-$C_2H_5$ | 1 |
| $(CH_2)_2CH_3$ | $CH_2CH_2CH_2OCH_2CH_2$ | 5-$C_2H_5$ | 1 |

Tabelle 20 (Fort.)

| $R^1$ | A | X | n |
|---|---|---|---|
| $CH_2CH_3$ | $CH_2CH_2CH_2CH_2OCH_2$ | - | 0 |
| $(CH_2)_2CH_3$ | $CH_2CH_2CH_2CH_2OCH_2$ | - | 0 |
| $CH_2CH_3$ | $CH_2CH_2CH_2CH_2OCH_2$ | 5-Cl | 1 |
| $(CH_2)_2CH_3$ | $CH_2CH_2CH_2CH_2OCH_2$ | 5-Cl | 1 |
| $CH_2CH_3$ | $CH_2CH_2CH_2CH_2OCH_2$ | 5-$CH_3$ | 1 |
| $(CH_2)_2CH_3$ | $CH_2CH_2CH_2CH_2OCH_2$ | 5-$CH_3$ | 1 |
| $CH_2CH_3$ | $CH_2CH_2CH_2CH_2OCH_2$ | 5-$C_2H_5$ | 1 |
| $(CH_2)_2CH_3$ | $CH_2CH_2CH_2CH_2OCH_2$ | 5-$C_2H_5$ | 1 |

Tabelle 21

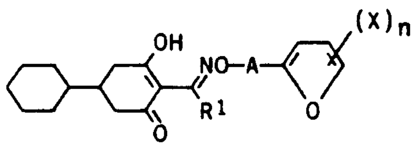

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 5-$CH_3$ | 1 |
| $(CH_2)_2CH_3$ | $CH_2CH_2OCH_2$ | 5-$CH_3$ | 1 |
| $CH_2CH_3$ | $CH_2CH_2OCH_2$ | 5-$C_2H_5$ | 1 |
| $(CH_2)_2CH_3$ | $CH_2CH_2OCH_2$ | 5-$C_2H_5$ | 1 |
| $CH_2CH_3$ | $CH_2CH_2CH_2OCH_2$ | - | 0 |
| $(CH_2)_2CH_3$ | $CH_2CH_2CH_2OCH_2$ | - | 0 |
| $CH_2CH_3$ | $CH_2CH_2CH_2OCH_2$ | 5-$CH_3$ | 1 |
| $(CH_2)_2CH_3$ | $CH_2CH_2CH_2OCH_2$ | 5-$CH_3$ | 1 |
| $CH_2CH_3$ | $CH_2CH_2CH_2OCH_2$ | 5-$C_2H_5$ | 1 |
| $(CH_2)_2CH_3$ | $CH_2CH_2CH_2OCH_2$ | 5-$C_2H_5$ | 1 |
| $CH_2CH_3$ | $CH_2CH_2CH_2OCH_2CH_2$ | - | 0 |
| $(CH_2)_2CH_3$ | $CH_2CH_2CH_2OCH_2CH_2$ | - | 0 |
| $CH_2CH_3$ | $CH_2CH_2CH_2OCH_2CH_2$ | 5-$CH_3$ | 1 |
| $(CH_2)_2CH_3$ | $CH_2CH_2CH_2OCH_2CH_2$ | 5-$CH_3$ | 1 |
| $CH_2CH_3$ | $CH_2CH_2CH_2OCH_2CH_2$ | 5-$C_2H_5$ | 1 |
| $(CH_2)_2CH_3$ | $CH_2CH_2CH_2OCH_2CH_2$ | 5-$C_2H_5$ | 1 |
| $CH_2CH_3$ | $CH_2CH_2CH_2CH_2OCH_2$ | - | 0 |
| $(CH_2)_2CH_3$ | $CH_2CH_2CH_2CH_2OCH_2$ | - | 0 |
| $CH_2CH_3$ | $CH_2CH_2CH_2CH_2OCH_2$ | 5-$CH_3$ | 1 |
| $(CH_2)_2CH_3$ | $CH_2CH_2CH_2CH_2OCH_2$ | 5-$CH_3$ | 1 |
| $CH_2CH_3$ | $CH_2CH_2CH_2CH_2OCH_2$ | 5-$C_2H_5$ | 1 |
| $(CH_2)_2CH_3$ | $CH_2CH_2CH_2CH_2OCH_2$ | 5-$C_2H_5$ | 1 |

Tabelle 22

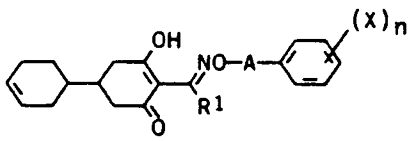

| R1 | A | X | n |
|---|---|---|---|
| $CH_2CH_3$ | $CH_2CH_2O$ | - | 0 |
| $(CH_2)_2CH_3$ | $CH_2CH_2O$ | - | 0 |
| $CH_2CH_3$ | $CH_2CH_2O$ | 2-Cl | 1 |
| $(CH_2)_2CH_3$ | $CH_2CH_2O$ | 2-Cl | 1 |
| $CH_2CH_3$ | $CH_2CH_2O$ | 3-Cl | 1 |
| $(CH_2)_2CH_3$ | $CH_2CH_2O$ | 3-Cl | 1 |
| $CH_2CH_3$ | $CH_2CH_2O$ | 4-Cl | 1 |
| $(CH_2)_2CH_3$ | $CH_2CH_2O$ | 4-Cl | 1 |
| $CH_2CH_3$ | $CH_2CH_2O$ | 2-F | 1 |
| $(CH_2)_2CH_3$ | $CH_2CH_2O$ | 2-F | 1 |
| $CH_2CH_3$ | $CH_2CH_2O$ | 3-F | 1 |
| $(CH_2)_2CH_3$ | $CH_2CH_2O$ | 3-F | 1 |
| $CH_2CH_3$ | $CH_2CH_2O$ | 4-F | 1 |
| $(CH_2)_2CH_3$ | $CH_2CH_2O$ | 4-F | 1 |
| $CH_2CH_3$ | $CH_2CH_2O$ | 3-$CF_3$ | 1 |
| $(CH_2)_2CH_3$ | $CH_2CH_2O$ | 3-$CF_3$ | 1 |
| $CH_2CH_3$ | $CH_2CH_2CH_2O$ | - | 0 |
| $(CH_2)_2CH_3$ | $CH_2CH_2CH_2O$ | - | 0 |
| $CH_2CH_3$ | $CH_2CH_2CH_2O$ | 4-Cl | 1 |
| $(CH_2)_2CH_3$ | $CH_2CH_2CH_2O$ | 4-Cl | 1 |
| $CH_2CH_3$ | $CH_2CH_2CH_2O$ | 4-F | 1 |
| $(CH_2)_2CH_3$ | $CH_2CH_2CH_2O$ | 4-F | 1 |

Tabelle 22 (Fort.)

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 2-F | 1 |
| $(CH_2)_2CH_3$ | $CH_2CH_2OCH_2$ | 2-F | 1 |
| $CH_2CH_3$ | $CH_2CH_2OCH_2$ | 3-F | 1 |
| $(CH_2)_2CH_3$ | $CH_2CH_2OCH_2$ | 3-F | 1 |
| $CH_2CH_3$ | $CH_2CH_2OCH_2$ | 4-F | 1 |
| $(CH_2)_2CH_3$ | $CH_2CH_2OCH_2$ | 4-F | 1 |
| $CH_2CH_3$ | $CH_2CH_2OCH_2$ | 4-Cl | 1 |
| $(CH_2)_2CH_3$ | $CH_2CH_2OCH_2$ | 4-Cl | 1 |
| $CH_2CH_3$ | $CH_2CH_2OCH_2$ | 2-$CH_3$ | 1 |
| $(CH_2)_2CH_3$ | $CH_2CH_2OCH_2$ | 2-$CH_3$ | 1 |
| $CH_2CH_3$ | $CH_2CH_2OCH_2$ | 3-$CH_3$ | 1 |
| $(CH_2)_2CH_3$ | $CH_2CH_2OCH_2$ | 3-$CH_3$ | 1 |
| $CH_2CH_3$ | $CH_2CH_2OCH_2$ | 4-$CH_3$ | 1 |
| $(CH_2)_2CH_3$ | $CH_2CH_2OCH_2$ | 4-$CH_3$ | 1 |
| $CH_2CH_3$ | $CH_2CH_2OCH_2$ | 4-t$C_4H_9$ | 1 |
| $(CH_2)_2CH_3$ | $CH_2CH_2OCH_2$ | 4-t$C_4H_9$ | 1 |
| $CH_2CH_3$ | $CH_2CH_2SCH_2$ | - | 0 |
| $(CH_2)_2CH_3$ | $CH_2CH_2SCH_2$ | - | 0 |
| $CH_2CH_3$ | $CH_2CH_2SCH_2$ | 4-Cl | 1 |
| $(CH_2)_2CH_3$ | $CH_2CH_2SCH_2$ | 4-Cl | 1 |
| $CH_2CH_3$ | $CH_2CH_2SCH_2$ | 4-F | 1 |
| $(CH_2)_2CH_3$ | $CH_2CH_2SCH_2$ | 4-F | 1 |
| $CH_2CH_3$ | $CH_2CH_2CH_2CH_2O$ | - | 0 |
| $(CH_2)_2CH_3$ | $CH_2CH_2CH_2CH_2O$ | - | 0 |
| $CH_2CH_3$ | $CH_2CH_2CH_2CH_2O$ | 4-Cl | 1 |
| $(CH_2)_2CH_3$ | $CH_2CH_2CH_2CH_2O$ | 4-Cl | 1 |
| $CH_2CH_3$ | $CH_2CH_2CH_2CH_2O$ | 4-F | 1 |
| $(CH_2)_2CH_3$ | $CH_2CH_2CH_2CH_2O$ | 4-F | 1 |

Tabelle 22 (Fort.)

| $R^1$ | A | X | n |
|---|---|---|---|
| $CH_2CH_3$ | $CH_2CH_2CH_2CH_2CH_2O$ | - | 0 |
| $(CH_2)_2CH_3$ | $CH_2CH_2CH_2CH_2CH_2O$ | - | 0 |
| $CH_2CH_3$ | $CH_2CH_2CH_2CH_2CH_2O$ | 4-Cl | 1 |
| $(CH_2)_2CH_3$ | $CH_2CH_2CH_2CH_2CH_2O$ | 4-Cl | 1 |
| $CH_2CH_3$ | $CH_2CH_2CH_2CH_2CH_2O$ | 4-F | 1 |
| $(CH_2)_2CH_3$ | $CH_2CH_2CH_2CH_2CH_2O$ | 4-F | 1 |

Tabelle 23

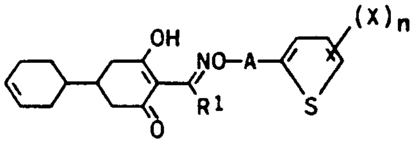

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 5-Cl | 1 |
| $(CH_2)_2CH_3$ | $CH_2CH_2OCH_2$ | 5-Cl | 1 |
| $CH_2CH_3$ | $CH_2CH_2OCH_2$ | $5-CH_3$ | 1 |
| $(CH_2)_2CH_3$ | $CH_2CH_2OCH_2$ | $5-CH_3$ | 1 |
| $CH_2CH_3$ | $CH_2CH_2OCH_2$ | $5-C_2H_5$ | 1 |
| $(CH_2)_2CH_3$ | $CH_2CH_2OCH_2$ | $5-C_2H_5$ | 1 |
| $CH_2CH_3$ | $CH_2CH_2CH_2OCH_2$ | - | 0 |
| $(CH_2)_2CH_3$ | $CH_2CH_2CH_2OCH_2$ | - | 0 |
| $CH_2CH_3$ | $CH_2CH_2CH_2OCH_2$ | 5-Cl | 1 |
| $(CH_2)_2CH_3$ | $CH_2CH_2CH_2OCH_2$ | 5-Cl | 1 |
| $CH_2CH_3$ | $CH_2CH_2CH_2OCH_2$ | $5-CH_3$ | 1 |
| $(CH_2)_2CH_3$ | $CH_2CH_2CH_2OCH_2$ | $5-CH_3$ | 1 |
| $CH_2CH_3$ | $CH_2CH_2CH_2OCH_2$ | $5-C_2H_5$ | 1 |
| $(CH_2)_2CH_3$ | $CH_2CH_2CH_2OCH_2$ | $5-C_2H_5$ | 1 |
| $CH_2CH_3$ | $CH_2CH_2CH_2OCH_2CH_2$ | - | 0 |
| $(CH_2)_2CH_3$ | $CH_2CH_2CH_2OCH_2CH_2$ | - | 0 |
| $CH_2CH_3$ | $CH_2CH_2CH_2OCH_2CH_2$ | 5-Cl | 1 |
| $(CH_2)_2CH_3$ | $CH_2CH_2CH_2OCH_2CH_2$ | 5-Cl | 1 |
| $CH_2CH_3$ | $CH_2CH_2CH_2OCH_2CH_2$ | $5-CH_3$ | 1 |
| $(CH_2)_2CH_3$ | $CH_2CH_2CH_2OCH_2CH_2$ | $5-CH_3$ | 1 |
| $CH_2CH_3$ | $CH_2CH_2CH_2OCH_2CH_2$ | $5-C_2H_5$ | 1 |
| $(CH_2)_2CH_3$ | $CH_2CH_2CH_2OCH_2CH_2$ | $5-C_2H_5$ | 1 |

Tabelle 23 (Fort.)

| $R^1$ | A | X | n |
|---|---|---|---|
| $CH_2CH_3$ | $CH_2CH_2CH_2CH_2OCH_2$ | - | 0 |
| $(CH_2)_2CH_3$ | $CH_2CH_2CH_2CH_2OCH_2$ | - | 0 |
| $CH_2CH_3$ | $CH_2CH_2CH_2CH_2OCH_2$ | 5-Cl | 1 |
| $(CH_2)_2CH_3$ | $CH_2CH_2CH_2CH_2OCH_2$ | 5-Cl | 1 |
| $CH_2CH_3$ | $CH_2CH_2CH_2CH_2OCH_2$ | 5-$CH_3$ | 1 |
| $(CH_2)_2CH_3$ | $CH_2CH_2CH_2CH_2OCH_2$ | 5-$CH_3$ | 1 |
| $CH_2CH_3$ | $CH_2CH_2CH_2CH_2OCH_2$ | 5-$C_2H_5$ | 1 |
| $(CH_2)_2CH_3$ | $CH_2CH_2CH_2CH_2OCH_2$ | 5-$C_2H_5$ | 1 |

Tabelle 24

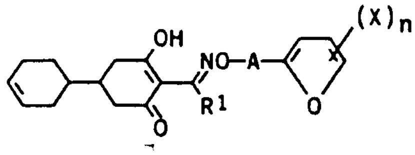

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 5-$CH_3$ | 1 |
| $(CH_2)_2CH_3$ | $CH_2CH_2OCH_2$ | 5-$CH_3$ | 1 |
| $CH_2CH_3$ | $CH_2CH_2OCH_2$ | 5-$C_2H_5$ | 1 |
| $(CH_2)_2CH_3$ | $CH_2CH_2OCH_2$ | 5-$C_2H_5$ | 1 |
| $CH_2CH_3$ | $CH_2CH_2CH_2OCH_2$ | - | 0 |
| $(CH_2)_2CH_3$ | $CH_2CH_2CH_2OCH_2$ | - | 0 |
| $CH_2CH_3$ | $CH_2CH_2CH_2OCH_2$ | 5-$CH_3$ | 1 |
| $(CH_2)_2CH_3$ | $CH_2CH_2CH_2OCH_2$ | 5-$CH_3$ | 1 |
| $CH_2CH_3$ | $CH_2CH_2CH_2OCH_2$ | 5-$C_2H_5$ | 1 |
| $(CH_2)_2CH_3$ | $CH_2CH_2CH_2OCH_2$ | 5-$C_2H_5$ | 1 |
| $CH_2CH_3$ | $CH_2CH_2CH_2OCH_2CH_2$ | - | 0 |
| $(CH_2)_2CH_3$ | $CH_2CH_2CH_2OCH_2CH_2$ | - | 0 |
| $CH_2CH_3$ | $CH_2CH_2CH_2OCH_2CH_2$ | 5-$CH_3$ | 1 |
| $(CH_2)_2CH_3$ | $CH_2CH_2CH_2OCH_2CH_2$ | 5-$CH_3$ | 1 |
| $CH_2CH_3$ | $CH_2CH_2CH_2OCH_2CH_2$ | 5-$C_2H_5$ | 1 |
| $(CH_2)_2CH_3$ | $CH_2CH_2CH_2OCH_2CH_2$ | 5-$C_2H_5$ | 1 |
| $CH_2CH_3$ | $CH_2CH_2CH_2CH_2OCH_2$ | - | 0 |
| $(CH_2)_2CH_3$ | $CH_2CH_2CH_2CH_2OCH_2$ | - | 0 |
| $CH_2CH_3$ | $CH_2CH_2CH_2CH_2OCH_2$ | 5-$CH_3$ | 1 |
| $(CH_2)_2CH_3$ | $CH_2CH_2CH_2CH_2OCH_2$ | 5-$CH_3$ | 1 |
| $CH_2CH_3$ | $CH_2CH_2CH_2CH_2OCH_2$ | 5-$C_2H_5$ | 1 |
| $(CH_2)_2CH_3$ | $CH_2CH_2CH_2CH_2OCH_2$ | 5-$C_2H_5$ | 1 |

Tabelle 25

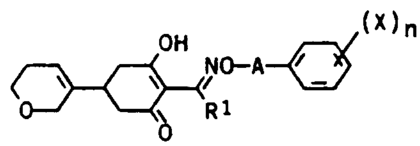

| $R^1$ | A | X | n |
|---|---|---|---|
| $CH_2CH_3$ | $CH_2CH_2O$ | - | 0 |
| $(CH_2)_2CH_3$ | $CH_2CH_2O$ | - | 0 |
| $CH_2CH_3$ | $CH_2CH_2O$ | 2-Cl | 1 |
| $(CH_2)_2CH_3$ | $CH_2CH_2O$ | 2-Cl | 1 |
| $CH_2CH_3$ | $CH_2CH_2O$ | 3-Cl | 1 |
| $(CH_2)_2CH_3$ | $CH_2CH_2O$ | 3-Cl | 1 |
| $CH_2CH_3$ | $CH_2CH_2O$ | 4-Cl | 1 |
| $(CH_2)_2CH_3$ | $CH_2CH_2O$ | 4-Cl | 1 |
| $CH_2CH_3$ | $CH_2CH_2O$ | 2-F | 1 |
| $(CH_2)_2CH_3$ | $CH_2CH_2O$ | 2-F | 1 |
| $CH_2CH_3$ | $CH_2CH_2O$ | 3-F | 1 |
| $(CH_2)_2CH_3$ | $CH_2CH_2O$ | 3-F | 1 |
| $CH_2CH_3$ | $CH_2CH_2O$ | 4-F | 1 |
| $(CH_2)_2CH_3$ | $CH_2CH_2O$ | 4-F | 1 |
| $CH_2CH_3$ | $CH_2CH_2O$ | $3-CF_3$ | 1 |
| $(CH_2)_2CH_3$ | $CH_2CH_2O$ | $3-CF_3$ | 1 |
| $CH_2CH_3$ | $CH_2CH_2CH_2O$ | - | 0 |
| $(CH_2)_2CH_3$ | $CH_2CH_2CH_2O$ | - | 0 |
| $CH_2CH_3$ | $CH_2CH_2CH_2O$ | 4-Cl | 1 |
| $(CH_2)_2CH_3$ | $CH_2CH_2CH_2O$ | 4-Cl | 1 |
| $CH_2CH_3$ | $CH_2CH_2CH_2O$ | 4-F | 1 |
| $(CH_2)_2CH_3$ | $CH_2CH_2CH_2O$ | 4-F | 1 |

Tabelle 25 (Fort.)

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 2-F | 1 |
| $(CH_2)_2CH_3$ | $CH_2CH_2OCH_2$ | 2-F | 1 |
| $CH_2CH_3$ | $CH_2CH_2OCH_2$ | 3-F | 1 |
| $(CH_2)_2CH_3$ | $CH_2CH_2OCH_2$ | 3-F | 1 |
| $CH_2CH_3$ | $CH_2CH_2OCH_2$ | 4-F | 1 |
| $(CH_2)_2CH_3$ | $CH_2CH_2OCH_2$ | 4-F | 1 |
| $CH_2CH_3$ | $CH_2CH_2OCH_2$ | 4-Cl | 1 |
| $(CH_2)_2CH_3$ | $CH_2CH_2OCH_2$ | 4-Cl | 1 |
| $CH_2CH_3$ | $CH_2CH_2OCH_2$ | $2-CH_3$ | 1 |
| $(CH_2)_2CH_3$ | $CH_2CH_2OCH_2$ | $2-CH_3$ | 1 |
| $CH_2CH_3$ | $CH_2CH_2OCH_2$ | $3-CH_3$ | 1 |
| $(CH_2)_2CH_3$ | $CH_2CH_2OCH_2$ | $3-CH_3$ | 1 |
| $CH_2CH_3$ | $CH_2CH_2OCH_2$ | $4-CH_3$ | 1 |
| $(CH_2)_2CH_3$ | $CH_2CH_2OCH_2$ | $4-CH_3$ | 1 |
| $CH_2CH_3$ | $CH_2CH_2OCH_2$ | $4-tC_4H_9$ | 1 |
| $(CH_2)_2CH_3$ | $CH_2CH_2OCH_2$ | $4-tC_4H_9$ | 1 |
| $CH_2CH_3$ | $CH_2CH_2SCH_2$ | - | 0 |
| $(CH_2)_2CH_3$ | $CH_2CH_2SCH_2$ | - | 0 |
| $CH_2CH_3$ | $CH_2CH_2SCH_2$ | 4-Cl | 1 |
| $(CH_2)_2CH_3$ | $CH_2CH_2SCH_2$ | 4-Cl | 1 |
| $CH_2CH_3$ | $CH_2CH_2SCH_2$ | 4-F | 1 |
| $(CH_2)_2CH_3$ | $CH_2CH_2SCH_2$ | 4-F | 1 |
| $CH_2CH_3$ | $CH_2CH_2CH_2CH_2O$ | - | 0 |
| $(CH_2)_2CH_3$ | $CH_2CH_2CH_2CH_2O$ | - | 0 |
| $CH_2CH_3$ | $CH_2CH_2CH_2CH_2O$ | 4-Cl | 1 |
| $(CH_2)_2CH_3$ | $CH_2CH_2CH_2CH_2O$ | 4-Cl | 1 |
| $CH_2CH_3$ | $CH_2CH_2CH_2CH_2O$ | 4-F | 1 |
| $(CH_2)_2CH_3$ | $CH_2CH_2CH_2CH_2O$ | 4-F | 1 |

Tabelle 26 (Fort.)

| $R^1$ | A | X | n |
|---|---|---|---|
| $CH_2CH_3$ | $CH_2CH_2CH_2CH_2CH_2O$ | - | 0 |
| $(CH_2)_2CH_3$ | $CH_2CH_2CH_2CH_2CH_2O$ | - | 0 |
| $CH_2CH_3$ | $CH_2CH_2CH_2CH_2CH_2O$ | 4-Cl | 1 |
| $(CH_2)_2CH_3$ | $CH_2CH_2CH_2CH_2CH_2O$ | 4-Cl | 1 |
| $CH_2CH_3$ | $CH_2CH_2CH_2CH_2CH_2O$ | 4-F | 1 |
| $(CH_2)_2CH_3$ | $CH_2CH_2CH_2CH_2CH_2O$ | 4-F | 1 |

Tabelle 26

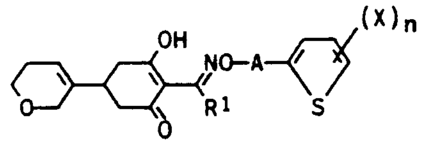

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 5-Cl | 1 |
| $(CH_2)_2CH_3$ | $CH_2CH_2OCH_2$ | 5-Cl | 1 |
| $CH_2CH_3$ | $CH_2CH_2OCH_2$ | 5-$CH_3$ | 1 |
| $(CH_2)_2CH_3$ | $CH_2CH_2OCH_2$ | 5-$CH_3$ | 1 |
| $CH_2CH_3$ | $CH_2CH_2OCH_2$ | 5-$C_2H_5$ | 1 |
| $(CH_2)_2CH_3$ | $CH_2CH_2OCH_2$ | 5-$C_2H_5$ | 1 |
| $CH_2CH_3$ | $CH_2CH_2CH_2OCH_2$ | - | 0 |
| $(CH_2)_2CH_3$ | $CH_2CH_2CH_2OCH_2$ | - | 1 |
| $CH_2CH_3$ | $CH_2CH_2CH_2OCH_2$ | 5-Cl | 1 |
| $(CH_2)_2CH_3$ | $CH_2CH_2CH_2OCH_2$ | 5-Cl | 1 |
| $CH_2CH_3$ | $CH_2CH_2CH_2OCH_2$ | 5-$CH_3$ | 1 |
| $(CH_2)_2CH_3$ | $CH_2CH_2CH_2OCH_2$ | 5-$CH_3$ | 1 |
| $CH_2CH_3$ | $CH_2CH_2CH_2OCH_2$ | 5-$C_2H_5$ | 1 |
| $(CH_2)_2CH_3$ | $CH_2CH_2CH_2OCH_2$ | 5-$C_2H_5$ | 1 |
| $CH_2CH_3$ | $CH_2CH_2CH_2OCH_2CH_2$ | - | 0 |
| $(CH_2)_2CH_3$ | $CH_2CH_2CH_2OCH_2CH_2$ | - | 0 |
| $CH_2CH_3$ | $CH_2CH_2CH_2OCH_2CH_2$ | 5-Cl | 1 |
| $(CH_2)_2CH_3$ | $CH_2CH_2CH_2OCH_2CH_2$ | 5-Cl | 1 |
| $CH_2CH_3$ | $CH_2CH_2CH_2OCH_2CH_2$ | 5-$CH_3$ | 1 |
| $(CH_2)_2CH_3$ | $CH_2CH_2CH_2OCH_2CH_2$ | 5-$CH_3$ | 1 |
| $CH_2CH_3$ | $CH_2CH_2CH_2OCH_2CH_2$ | 5-$C_2H_5$ | 1 |
| $(CH_2)_2CH_3$ | $CH_2CH_2CH_2OCH_2CH_2$ | 5-$C_2H_5$ | 1 |

Tabelle 26 (Fort.)

| $R^1$ | A | X | n |
|---|---|---|---|
| $CH_2CH_3$ | $CH_2CH_2CH_2CH_2OCH_2$ | - | 0 |
| $(CH_2)_2CH_3$ | $CH_2CH_2CH_2CH_2OCH_2$ | - | 0 |
| $CH_2CH_3$ | $CH_2CH_2CH_2CH_2OCH_2$ | 5-Cl | 1 |
| $(CH_2)_2CH_3$ | $CH_2CH_2CH_2CH_2OCH_2$ | 5-Cl | 1 |
| $CH_2CH_3$ | $CH_2CH_2CH_2CH_2OCH_2$ | 5-$CH_3$ | 1 |
| $(CH_2)_2CH_3$ | $CH_2CH_2CH_2CH_2OCH_2$ | 5-$CH_3$ | 1 |
| $CH_2CH_3$ | $CH_2CH_2CH_2CH_2OCH_2$ | 5-$C_2H_5$ | 1 |
| $(CH_2)_2CH_3$ | $CH_2CH_2CH_2CH_2OCH_2$ | 5-$C_2H_5$ | 1 |

Tabelle 27

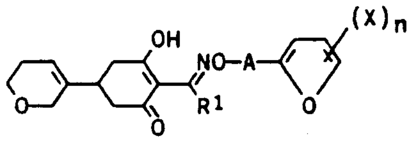

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 5-$CH_3$ | 1 |
| $(CH_2)_2CH_3$ | $CH_2CH_2OCH_2$ | 5-$CH_3$ | 1 |
| $CH_2CH_3$ | $CH_2CH_2OCH_2$ | 5-$C_2H_5$ | 1 |
| $(CH_2)_2CH_3$ | $CH_2CH_2OCH_2$ | 5-$C_2H_5$ | 1 |
| $CH_2CH_3$ | $CH_2CH_2CH_2OCH_2$ | - | 0 |
| $(CH_2)_2CH_3$ | $CH_2CH_2CH_2OCH_2$ | - | 0 |
| $CH_2CH_3$ | $CH_2CH_2CH_2OCH_2$ | 5-$CH_3$ | 1 |
| $(CH_2)_2CH_3$ | $CH_2CH_2CH_2OCH_2$ | 5-$CH_3$ | 1 |
| $CH_2CH_3$ | $CH_2CH_2CH_2OCH_2$ | 5-$C_2H_5$ | 1 |
| $(CH_2)_2CH_3$ | $CH_2CH_2CH_2OCH_2$ | 5-$C_2H_5$ | 1 |
| $CH_2CH_3$ | $CH_2CH_2CH_2OCH_2CH_2$ | - | 0 |
| $(CH_2)_2CH_3$ | $CH_2CH_2CH_2OCH_2CH_2$ | - | 0 |
| $CH_2CH_3$ | $CH_2CH_2CH_2OCH_2CH_2$ | 5-$CH_3$ | 1 |
| $(CH_2)_2CH_3$ | $CH_2CH_2CH_2OCH_2CH_2$ | 5-$CH_3$ | 1 |
| $CH_2CH_3$ | $CH_2CH_2CH_2OCH_2CH_2$ | 5-$C_2H_5$ | 1 |
| $(CH_2)_2CH_3$ | $CH_2CH_2CH_2OCH_2CH_2$ | 5-$C_2H_5$ | 1 |
| $CH_2CH_3$ | $CH_2CH_2CH_2CH_2OCH_2$ | - | 0 |
| $(CH_2)_2CH_3$ | $CH_2CH_2CH_2CH_2OCH_2$ | - | 0 |
| $CH_2CH_3$ | $CH_2CH_2CH_2CH_2OCH_2$ | 5-$CH_3$ | 1 |
| $(CH_2)_2CH_3$ | $CH_2CH_2CH_2CH_2OCH_2$ | 5-$CH_3$ | 1 |
| $CH_2CH_3$ | $CH_2CH_2CH_2CH_2OCH_2$ | 5-$C_2H_5$ | 1 |
| $(CH_2)_2CH_3$ | $CH_2CH_2CH_2CH_2OCH_2$ | 5-$C_2H_5$ | 1 |

Tabelle 28

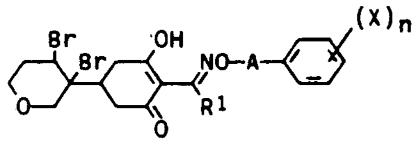

| $R^1$ | A | X | n |
|---|---|---|---|
| $CH_2CH_3$ | $CH_2CH_2O$ | - | 0 |
| $(CH_2)_2CH_3$ | $CH_2CH_2O$ | - | 0 |
| $CH_2CH_3$ | $CH_2CH_2O$ | 2-Cl | 1 |
| $(CH_2)_2CH_3$ | $CH_2CH_2O$ | 2-Cl | 1 |
| $CH_2CH_3$ | $CH_2CH_2O$ | 3-Cl | 1 |
| $(CH_2)_2CH_3$ | $CH_2CH_2O$ | 3-Cl | 1 |
| $CH_2CH_3$ | $CH_2CH_2O$ | 4-Cl | 1 |
| $(CH_2)_2CH_3$ | $CH_2CH_2O$ | 4-Cl | 1 |
| $CH_2CH_3$ | $CH_2CH_2O$ | 2-F | 1 |
| $(CH_2)_2CH_3$ | $CH_2CH_2O$ | 2-F | 1 |
| $CH_2CH_3$ | $CH_2CH_2O$ | 3-F | 1 |
| $(CH_2)_2CH_3$ | $CH_2CH_2O$ | 3-F | 1 |
| $CH_2CH_3$ | $CH_2CH_2O$ | 4-F | 1 |
| $(CH_2)_2CH_3$ | $CH_2CH_2O$ | 4-F | 1 |
| $CH_2CH_3$ | $CH_2CH_2O$ | $3-CF_3$ | 1 |
| $(CH_2)_2CH_3$ | $CH_2CH_2O$ | $3-CF_3$ | 1 |
| $CH_2CH_3$ | $CH_2CH_2CH_2O$ | - | 0 |
| $(CH_2)_2CH_3$ | $CH_2CH_2CH_2O$ | - | 0 |
| $CH_2CH_3$ | $CH_2CH_2CH_2O$ | 4-Cl | 1 |
| $(CH_2)_2CH_3$ | $CH_2CH_2CH_2O$ | 4-Cl | 1 |
| $CH_2CH_3$ | $CH_2CH_2CH_2O$ | 4-F | 1 |
| $(CH_2)_2CH_3$ | $CH_2CH_2CH_2O$ | 4-F | 1 |

Tabelle 28 (Fort.)

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 2-F | 1 |
| $(CH_2)_2CH_3$ | $CH_2CH_2OCH_2$ | 2-F | 1 |
| $CH_2CH_3$ | $CH_2CH_2OCH_2$ | 3-F | 1 |
| $(CH_2)_2CH_3$ | $CH_2CH_2OCH_2$ | 3-F | 1 |
| $CH_2CH_3$ | $CH_2CH_2OCH_2$ | 4-F | 1 |
| $(CH_2)_2CH_3$ | $CH_2CH_2OCH_2$ | 4-F | 1 |
| $CH_2CH_3$ | $CH_2CH_2OCH_2$ | 4-Cl | 1 |
| $(CH_2)_2CH_3$ | $CH_2CH_2OCH_2$ | 4-Cl | 1 |
| $CH_2CH_3$ | $CH_2CH_2OCH_2$ | 2-$CH_3$ | 1 |
| $(CH_2)_2CH_3$ | $CH_2CH_2OCH_2$ | 2-$CH_3$ | 1 |
| $CH_2CH_3$ | $CH_2CH_2OCH_2$ | 3-$CH_3$ | 1 |
| $(CH_2)_2CH_3$ | $CH_2CH_2OCH_2$ | 3-$CH_3$ | 1 |
| $CH_2CH_3$ | $CH_2CH_2OCH_2$ | 4-$CH_3$ | 1 |
| $(CH_2)_2CH_3$ | $CH_2CH_2OCH_2$ | 4-$CH_3$ | 1 |
| $CH_2CH_3$ | $CH_2CH_2OCH_2$ | 4-t$C_4H_9$ | 1 |
| $(CH_2)_2CH_3$ | $CH_2CH_2OCH_2$ | 4-t$C_4H_9$ | 1 |
| $CH_2CH_3$ | $CH_2CH_2SCH_2$ | - | 0 |
| $(CH_2)_2CH_3$ | $CH_2CH_2SCH_2$ | - | 0 |
| $CH_2CH_3$ | $CH_2CH_2SCH_2$ | 4-Cl | 1 |
| $(CH_2)_2CH_3$ | $CH_2CH_2SCH_2$ | 4-Cl | 1 |
| $CH_2CH_3$ | $CH_2CH_2SCH_2$ | 4-F | 1 |
| $(CH_2)_2CH_3$ | $CH_2CH_2SCH_2$ | 4-F | 1 |
| $CH_2CH_3$ | $CH_2CH_2CH_2CH_2O$ | - | 0 |
| $(CH_2)_2CH_3$ | $CH_2CH_2CH_2CH_2O$ | - | 0 |
| $CH_2CH_3$ | $CH_2CH_2CH_2CH_2O$ | 4-Cl | 1 |
| $(CH_2)_2CH_3$ | $CH_2CH_2CH_2CH_2O$ | 4-Cl | 1 |
| $CH_2CH_3$ | $CH_2CH_2CH_2CH_2O$ | 4-F | 1 |
| $(CH_2)_2CH_3$ | $CH_2CH_2CH_2CH_2O$ | 4-F | 1 |

Tabelle 28 (Fort.)

| $R^1$ | A | X | n |
|---|---|---|---|
| $CH_2CH_3$ | $CH_2CH_2CH_2CH_2CH_2O$ | - | 0 |
| $(CH_2)_2CH_3$ | $CH_2CH_2CH_2CH_2CH_2O$ | - | 0 |
| $CH_2CH_3$ | $CH_2CH_2CH_2CH_2CH_2O$ | 4-Cl | 1 |
| $(CH_2)_2CH_3$ | $CH_2CH_2CH_2CH_2CH_2O$ | 4-Cl | 1 |
| $CH_2CH_3$ | $CH_2CH_2CH_2CH_2CH_2O$ | 4-F | 1 |
| $(CH_2)_2CH_3$ | $CH_2CH_2CH_2CH_2CH_2O$ | 4-F | 1 |

Tabelle 29

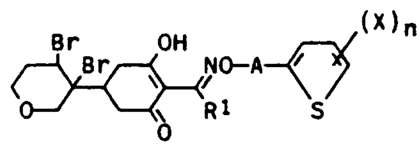

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 5-Cl | 1 |
| $(CH_2)_2CH_3$ | $CH_2CH_2OCH_2$ | 5-Cl | 1 |
| $CH_2CH_3$ | $CH_2CH_2OCH_2$ | 5-$CH_3$ | 1 |
| $(CH_2)_2CH_3$ | $CH_2CH_2OCH_2$ | 5-$CH_3$ | 1 |
| $CH_2CH_3$ | $CH_2CH_2OCH_2$ | 5-$C_2H_5$ | 1 |
| $(CH_2)_2CH_3$ | $CH_2CH_2OCH_2$ | 5-$C_2H_5$ | 1 |
| $CH_2CH_3$ | $CH_2CH_2CH_2OCH_2$ | - | 0 |
| $(CH_2)_2CH_3$ | $CH_2CH_2CH_2OCH_2$ | - | 0 |
| $CH_2CH_3$ | $CH_2CH_2CH_2OCH_2$ | 5-Cl | 1 |
| $(CH_2)_2CH_3$ | $CH_2CH_2CH_2OCH_2$ | 5-Cl | 1 |
| $CH_2CH_3$ | $CH_2CH_2CH_2OCH_2$ | 5-$CH_3$ | 1 |
| $(CH_2)_2CH_3$ | $CH_2CH_2CH_2OCH_2$ | 5-$CH_3$ | 1 |
| $CH_2CH_3$ | $CH_2CH_2CH_2OCH_2$ | 5-$C_2H_5$ | 1 |
| $(CH_2)_2CH_3$ | $CH_2CH_2CH_2OCH_2$ | 5-$C_2H_5$ | 1 |
| $CH_2CH_3$ | $CH_2CH_2CH_2OCH_2CH_2$ | - | 0 |
| $(CH_2)_2CH_3$ | $CH_2CH_2CH_2OCH_2CH_2$ | - | 0 |
| $CH_2CH_3$ | $CH_2CH_2CH_2OCH_2CH_2$ | 5-Cl | 1 |
| $(CH_2)_2CH_3$ | $CH_2CH_2CH_2OCH_2CH_2$ | 5-Cl | 1 |
| $CH_2CH_3$ | $CH_2CH_2CH_2OCH_2CH_2$ | 5-$CH_3$ | 1 |
| $(CH_2)_2CH_3$ | $CH_2CH_2CH_2OCH_2CH_2$ | 5-$CH_3$ | 1 |
| $CH_2CH_3$ | $CH_2CH_2CH_2OCH_2CH_2$ | 5-$C_2H_5$ | 1 |
| $(CH_2)_2CH_3$ | $CH_2CH_2CH_2OCH_2CH_2$ | 5-$C_2H_5$ | 1 |

Tabelle 29 (Fort.)

| $R^1$ | A | X | n |
|---|---|---|---|
| $CH_2CH_3$ | $CH_2CH_2CH_2CH_2OCH_2$ | - | 0 |
| $(CH_2)_2CH_3$ | $CH_2CH_2CH_2CH_2OCH_2$ | - | 0 |
| $CH_2CH_3$ | $CH_2CH_2CH_2CH_2OCH_2$ | 5-Cl | 1 |
| $(CH_2)_2CH_3$ | $CH_2CH_2CH_2CH_2OCH_2$ | 5-Cl | 1 |
| $CH_2CH_3$ | $CH_2CH_2CH_2CH_2OCH_2$ | 5-$CH_3$ | 1 |
| $(CH_2)_2CH_3$ | $CH_2CH_2CH_2CH_2OCH_2$ | 5-$CH_3$ | 1 |
| $CH_2CH_3$ | $CH_2CH_2CH_2CH_2OCH_2$ | 5-$C_2H_5$ | 1 |
| $(CH_2)_2CH_3$ | $CH_2CH_2CH_2CH_2OCH_2$ | 5-$C_2H_5$ | 1 |

Tabelle 30

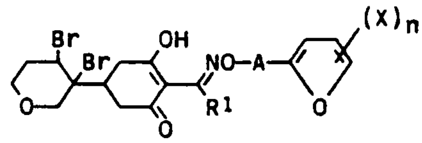

| $R^1$ | A | X | n |
|---|---|---|---|
| $CH_2CH_3$ | $CH_2CH_2OCH_2$ | - | 0 |
| $(CH_2)_2CH_3$ | $CH_2CH_2OCH_2$ | - | 0 |
| $CH_2CH_3$ | $CH_2CH_2OCH_2$ | 5-$CH_3$ | 1 |
| $(CH_2)_2CH_3$ | $CH_2CH_2OCH_2$ | 5-$CH_3$ | 1 |
| $CH_2CH_3$ | $CH_2CH_2OCH_2$ | 5-$C_2H_5$ | 1 |
| $(CH_2)_2CH_3$ | $CH_2CH_2OCH_2$ | 5-$C_2H_5$ | 1 |
| $CH_2CH_3$ | $CH_2CH_2CH_2OCH_2$ | - | 0 |
| $(CH_2)_2CH_3$ | $CH_2CH_2CH_2OCH_2$ | - | 0 |
| $CH_2CH_3$ | $CH_2CH_2CH_2OCH_2$ | 5-$CH_3$ | 1 |
| $(CH_2)_2CH_3$ | $CH_2CH_2CH_2OCH_2$ | 5-$CH_3$ | 1 |
| $CH_2CH_3$ | $CH_2CH_2CH_2OCH_2$ | 5-$C_2H_5$ | 1 |
| $(CH_2)_2CH_3$ | $CH_2CH_2CH_2OCH_2$ | 5-$C_2H_5$ | 1 |
| $CH_2CH_3$ | $CH_2CH_2CH_2OCH_2CH_2$ | - | 0 |
| $(CH_2)_2CH_3$ | $CH_2CH_2CH_2OCH_2CH_2$ | - | 0 |
| $CH_2CH_3$ | $CH_2CH_2CH_2OCH_2CH_2$ | 5-$CH_3$ | 1 |
| $(CH_2)_2CH_3$ | $CH_2CH_2CH_2OCH_2CH_2$ | 5-$CH_3$ | 1 |
| $CH_2CH_3$ | $CH_2CH_2CH_2OCH_2CH_2$ | 5-$C_2H_5$ | 1 |
| $(CH_2)_2CH_3$ | $CH_2CH_2CH_2OCH_2CH_2$ | 5-$C_2H_5$ | 1 |
| $CH_2CH_3$ | $CH_2CH_2CH_2CH_2OCH_2$ | - | 0 |
| $(CH_2)_2CH_3$ | $CH_2CH_2CH_2CH_2OCH_2$ | - | 0 |
| $CH_2CH_3$ | $CH_2CH_2CH_2CH_2OCH_2$ | 5-$CH_3$ | 1 |
| $(CH_2)_2CH_3$ | $CH_2CH_2CH_2CH_2OCH_2$ | 5-$CH_3$ | 1 |
| $CH_2CH_3$ | $CH_2CH_2CH_2CH_2OCH_2$ | 5-$C_2H_5$ | 1 |
| $(CH_2)_2CH_3$ | $CH_2CH_2CH_2CH_2OCH_2$ | 5-$C_2H_5$ | 1 |

Cyclohexenonoximether I, wobei Z die Phenylgruppe bedeutet, sind ganz besonders bevorzugt.

Die Cyclohexenonoximether I eignen sich als Herbizide, insbesondere zur Bekämpfung von Pflanzenarten aus der Familie der Gramineen (Gräser).

Die Cyclohexenonoximether I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin-und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach GC-/HPLC- oder NMR-Spektrum) eingesetzt.

Die erfindungsgemäßen Verbindungen I können beispielsweise wie folgt formuliert werden:

I. Man vermischt 90 Gewichtsteile der Verbindung Nr. 7.21 mit 10 Gewichtsteilen N-Methyl-$\alpha$-pyrrolidon und erhält eine Lösung, die zur Anwendung in Form kleinster Tropfen geeignet ist.

II. 20 Gewichtsteile der Verbindung Nr. 7.23 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 7.21 werden in einer Mischung gelöst, die aus 40 Gewichtstellen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 7.23 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 7.23 werden mit 3 Gewichtsteilen des Natriumsalzes der Diisobutylnaphthalin-$\alpha$-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 7.15 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 7.21 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 7.23 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der Mittel kann im Vorauflauf- oder im Nachauflaufverfahren erfolgen. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,001 bis 3 kg/ha, vorzugsweise 0,01 bis 2,0 kg/ha.

In Anbetracht des erfaßbaren Wirkungsspektrums zur Unkrautbekämpfung, der Verträglichkeit für Kulturpflanzen oder der erwünschten Beeinflussung des Wachstums derselben sowie angesichts der Vielfalt der Applikationsmethoden können die erfindungsgemäßen Verbindungen in einer großen Zahl von Kulturpflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |

| Botanischer Name | Deutscher Name |
|---|---|
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |

| Botanischer Name | Deutscher Name |
|---|---|
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Cyclohexenonderivate der Formel I sowohl untereinander als auch mit Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Chinolincarbonsäuren, Sulfonylharnstoffderivate, Cyclohexenone, (Hetero)-aryloxypropionsäuren, deren Salze, Ester und Amide und andere in Betracht.

Außerdem kann es von Nutzen sein, die Cyclohexenonderivate der Formel I bzw. sie enthaltende herbizide Mittel allein oder in Kombination mit anderen Herbiziden oder auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die in den nachstehenden Synthesebeispielen wiedergegebene Vorschrift wurde unter entsprechender Abwandlung der Ausgangsverbindung zur Gewinnung weiterer Hydroxylamine der Formel III und Cyclohexenonoximether der Formel 1 benutzt; die erhaltenen Verbindungen sind in den nachfolgenden Tabellen mit physikalischen Angaben aufgeführt.

Synthesebeispiele zur Herstellung der Hydroxylamine III

N-[2-(2-Fluorbenzyloxy)ethoxy]phthalimid

Zu einer Mischung aus 165 g (0,71 mol) 1-Brom-2-(2-fluorbenzyloxy)ethan, 116 g (0,7 mol) N-Hydroxyphthalimid und 710 ml N-Methyl-2-pyrrolidinon tropfte man bei 20-25°C innerhalb 1 h 108 ml Triethylamin. Nach 5 h bei 60°C goß man die kalte Reaktionsmischung in 2000 ml Eiswasser, saugte den Niederschlag ab, wusch mit Wasser und Isopropanol und trocknete i. Vak. über Phosphorpentoxid. Ausbeute: 185 g (82 %) Phthalimidether, Fp. 62-64°C - $^1$H-NMR (250 MHZ, $d_6$-DMSO): $\delta$ = 3,85 (m,2H), 4,35 (m,2H), 4,54 (s,2H), 7,10-7,40 (m,4H), 7,88 (s,4H).

O-[2-(2-Fluorbenzyloxy)ethyl]hydroxylamin

184 g (0,58 mol) des oben hergestellten Phthalimidethers wurden portionsweise in 270 ml Ethanolamin eingetragen. Nach 3 h bei 60 °C goß man die kalte Reaktionsmischung in 1000 ml Eiswasser. Das Hydrolysat wurde dreimal mit je 800 ml Dichlormethan extrahiert. Die vereinigten organischen Phasen wurden mit 200 ml ges. Kochsalz-Lösung gewaschen, über Magnesiumsulfat getrocknet und i. Vak. eingeengt. 98 g (91 %) Hydroxylamin. $^1$H-NMR (250 MHZ, $CDCl_3$): $\delta$ = 3,70 (dd,2H), 3,85 (dd,2H), 4,54 (s,2H), 5,50 (bs,2H), 7,00-7,50 (m,4H).

Synthesebeispiel zur Herstellung der Cyclohexenonoximether I

2-[1-[[2-(2-Fluorbenzyloxy)ethoxy]imino]butyl]-3-hydroxy-5-(2H-tetrahydropyran-4-yl)-2-cyclohexen-1-on

Eine Mischung aus 4,0 g (10 mmol) 2-Butyryl-3-hydroxy-5-(2H-tetrahydropyran-4-yl)-2-cyclohexen-1-on und 2,6 g (14 mmol) O-[2-(2-Fluorbenzyloxy)-ethyl]hydroxylamin in 100 ml Methanol wurde 24 h gerührt. Man engte i. Vak. ein und chromatographierte das Rohprodukt an 100 g Kieselgel/Säule 30 x 4 cm. (Laufmittel: Ether). 3,5 g (54 %) Cyclohexenonoximether. - $^1$H-NMR (300 MHZ, $CDCl_3$): $\delta$ = 0,93 (t,3H), 1,20-1,77 (m,7H), 1,90 (m,1H), 2,23 (m,2H), 2,58 (m,2H), 2,92 (m,2H), 3,38 (t,2H), 3,80 (m,2H), 4,03 (m,2H), 4,25 (m,2H), 4,68 (s,2H), 6,93-7,50 (m,4H), 14,30 (s,1H).

Tabelle A

$H_2N-O-A-Z-X_n$ III

| Nr. | A | Z | X | n | phys. Daten / $^1H$-NMR [δ in ppm] |
|---|---|---|---|---|---|
| 1.01 | $CH_2CH_2O$ | Phenyl | - | 0 | 6.90 (m, 3H), 7,27 (m, 2H) |
| 1.02 | $CH_2CH_2O$ | Phenyl | 2-F | 1 | 6.85-7.15 (m, 4H) |
| 1.03 | $CH_2CH_2O$ | Phenyl | 3-F | 1 | 6.70 (m, 3H), 7,25 (m, 1H) |
| 1.04 | $CH_2CH_2O$ | Phenyl | 4-F | 1 | 6.80-7.00 (m, 4H) |
| 1.05 | $CH_2CH_2O$ | Phenyl | 2-Cl | 1 | |
| 1.06 | $CH_2CH_2O$ | Phenyl | 3-Cl | 1 | |
| 1.07 | $CH_2CH_2O$ | Phenyl | 4-Cl | 1 | 6.85 (m, 2H), 7.25 (m, 2H) |
| 1.08 | $CH_2CH_2O$ | Phenyl | 2-$CF_3$ | 1 | |
| 1.09 | $CH_2CH_2O$ | Phenyl | 3-$CF_3$ | 1 | |
| 1.10 | $CH_2CH_2O$ | Phenyl | 4-$CF_3$ | 1 | |
| 1.11 | $CH_2CH_2O$ | Phenyl | 2,4-$Cl_2$ | 2 | 6.83 (d, 1H), 7.15 (dd, 1H), 7.85 (d, 1H) |
| 1.12 | $CH_2CH_2O$ | Phenyl | 2,4,6-$Cl_3$ | 3 | 7.30 (s, 2H) |
| 1.13 | $CH_2CH_2O$ | Phenyl | 4-$NO_2$ | 1 | 7.00 (d, 2H), 8.20 (d, 2H) |
| 1.14 | $CH_2CH(CH_3)O$ | Phenyl | - | 0 | |
| 1.15 | $CH_2CH(CH_3)O$ | Phenyl | 4-F | 1 | |
| 1.16 | $CH_2CH(CH_3)O$ | Phenyl | 4-Cl | 1 | |
| 1.17 | $CH_2CH_2S$ | Phenyl | - | 0 | 7.17-7.43 (m, 5H) |
| 1.18 | $CH_2CH_2S$ | Phenyl | 4-F | 1 | 7.00 (m, 2H), 7.40 (m, 2H) |

Tabelle A (Fort.)

| Nr. | A | Z | X | n | phys. Daten / $^1$H-NMR [δ in ppm] |
|---|---|---|---|---|---|
| 1.19 | $CH_2CH_2S$ | Phenyl | 2-Cl | 1 | 7.06-7.40 (m, 4H) |
| 1.20 | $CH_2CH_2S$ | Phenyl | 4-Cl | 1 | 7.30 (m, 4H) |
| 1.21 | $CH_2CH_2S$ | Phenyl | 2,6-$Cl_2$ | 2 | 7.15 (t, 1H), 7,33 (d, 2H) |
| 1.22 | $CH_2CH_2CH_2O$ | Phenyl | - | 0 | 6.90 (m, 3H), 7.27 (m, 2H) |
| 1.23 | $CH_2CH_2CH_2O$ | Phenyl | 2-F | 1 | 6.80-7.15 (m, 4H) |
| 1.24 | $CH_2CH_2CH_2O$ | Phenyl | 3-F | 1 | 6.70 (m, 3H), 7.25 (m, 1H) |
| 1.25 | $CH_2CH_2CH_2O$ | Phenyl | 4-F | 1 | 6.80-7.00 (m, 4H) |
| 1.26 | $CH_2CH_2CH_2O$ | Phenyl | 2-Cl | 1 | 6.80-7.00 (m, 2H), 7.20 (m, 1H), 7.35 (m, 1H) |
| 1.27 | $CH_2CH_2CH_2O$ | Phenyl | 3-Cl | 1 | 6.80 (m, 1H), 6.95 (m, 2H), 7.20 (m, 1H) |
| 1.28 | $CH_2CH_2CH_2O$ | Phenyl | 4-Cl | 1 | 6.80 (m, 2H), 7.20 (m, 2H) |
| 1.29 | $CH_2CH_2CH_2O$ | Phenyl | 4-$NO_2$ | 1 | 7.00 (d, 2H), 8.15 (d, 2H) |
| 1.30 | $CH_2CH_2CH_2S$ | Phenyl | - | 0 | 7.15-7.40 (m, 5H) |
| 1.31 | $CH_2CH_2CH_2S$ | Phenyl | 4-F | 1 | 7.00 (m, 2H), 7.40 (m, 2H) |
| 1.32 | $CH_2CH_2CH_2S$ | Phenyl | 2-Cl | 1 | 7.07-7.40 (m, 4H) |
| 1.33 | $CH_2CH_2CH_2S$ | Phenyl | 3-Cl | 1 | 7.17 (m, 3H), 7.30 (m, 1H) |
| 1.34 | $CH_2CH_2CH_2S$ | Phenyl | 4-Cl | 1 | 7.25 (m, 4H) |
| 1.35 | $CH_2CH_2CH_2S$ | Phenyl | 2,5-$Cl_2$ | 2 | 7.03 (dd, 1H), 7.20 (d, 1H), 7.23 (d, 1H) |
| 1.36 | $CH_2CH_2CH_2S$ | Phenyl | 2,6-$Cl_2$ | 2 | 7.15 (t, 1H), 7.33 (d, 2H) |

Tabelle A (Fort.)

| Nr. | A | Z | X | n | phys. Daten / $^1$H-NMR [$\delta$ in ppm] |
|---|---|---|---|---|---|
| 1.37 | $CH_2CH_2OCH_2$ | Phenyl | - | 0 | 7.25 (m, 5H) |
| 1.38 | $CH_2CH_2OCH_2$ | Phenyl | 2-F | 1 | 3,70 (dd, 2H), 3,85 (dd, 2H), 4,54 (s, 2H), 5,50 (bs, 2H), 7,00-7,50 (m, 4H) |
| 1.39 | $CH_2CH_2OCH_2$ | Phenyl | 3-F | 1 | |
| 1.40 | $CH_2CH_2OCH_2$ | Phenyl | 4-F | 1 | 3,66 (dd, 2H), 3,88 (dd, 2H), 4,54 (s, 2H), 5,50 (bs, 2H), 7,00-7,10 (m, 2H), 7,20-7,40 (m, 2H) |
| 1.41 | $CH_2CH_2OCH_2$ | Phenyl | 2-Cl | 1 | |
| 1.42 | $CH_2CH_2OCH_2$ | Phenyl | 3-Cl | 1 | |
| 1.43 | $CH_2CH_2OCH_2$ | Phenyl | 4-Cl | 1 | 3,65 (dd, 2H), 3,84 (dd, 2H), 4,54 (s, 2H), 5,50 (bs, 2H), 7,20-7,40 (m, 4H) |
| 1.44 | $CH_2CH_2OCH_2$ | Phenyl | $2-CH_3$ | 1 | |
| 1.45 | $CH_2CH_2OCH_2$ | Phenyl | $3-CH_3$ | 1 | 2,35 (s, 3H), 3,65 (dd, 2H), 3,87 (dd, 2H), 4,54 (s, 2H), 5,50 (bs, 2H), 7,00-7,30 (m, 4H) |
| 1.46 | $CH_2CH_2OCH_2$ | Phenyl | $4-CH_3$ | 1 | 2,35 (s, 3H), 3,64 (dd, 2H), 3,84 (dd, 2H), 4,53 (s, 2H), 5,50 (bs, 2H), 7,10-7,30 (m, 4H) |
| 1.47 | $CH_2CH_2OCH_2$ | Phenyl | $4-tC_4H_9$ | 1 | 1,33 (s, 9H), 3,65 (dd, 2H), 3,89 (dd, 2H), 4,55 (s, 2H), 5,50 (bs, 2H), 7,20-7,50 (m, 4H) |

Tabelle A (Fort.)

| Nr. | A | Z | X | n | phys. Daten / $^1H$-NMR [δ in ppm] |
|---|---|---|---|---|---|
| 1.48 | $CH_2CH_2SCH_2$ | Phenyl | - | 0 | 2,66 (t, 2H), 3,70-3,85 (m, 4H), 5,40 (bs, 2H), 7,20-7,50 (m, 5H) |
| 1.49 | $CH_2CH_2SCH_2$ | Phenyl | 4-F | 1 | 7.00 (m, 2H), 7.25 (m, 2H) |
| 1.50 | $CH_2CH_2SCH_2$ | Phenyl | 4-Cl | 1 | 2,65 (t, 2H), 3,72 (s, 2H), 3,78 (t, 2H), 5,40 (bs, 2H), 7,28 (s, 4H) |
| 1.51 | $CH_2CH_2CH_2CH_2O$ | Phenyl | - | 0 | |
| 1.52 | $CH_2CH_2CH_2CH_2O$ | Phenyl | 2-F | 1 | |
| 1.53 | $CH_2CH_2CH_2CH_2O$ | Phenyl | 3-F | 1 | |
| 1.54 | $CH_2CH_2CH_2CH_2O$ | Phenyl | 4-F | 1 | 6.80-7.00 (m, 4H) |
| 1.55 | $CH_2CH_2CH_2CH_2O$ | Phenyl | 4-Cl | 1 | 6.80 (m, 2H), 7.20 (m, 2H) |
| 1.56 | $CH_2CH_2CH_2CH_2O$ | Phenyl | 2,6-$Cl_2$ | 2 | 6.95 (t, 1H), 7,30 (d, 2H) |
| 1.57 | $CH_2CH_2OCH_2CH_2$ | Phenyl | - | 0 | |
| 1.58 | $CH_2CH_2OCH_2CH_2$ | Phenyl | 4-F | 1 | 6.93 (m, 2H), 7.13 (m, 2H) |
| 1.59 | $CH_2CH_2OCH_2CH_2$ | Phenyl | 4-Cl | 1 | |
| 1.60 | $CH_2CH_2CH_2CH_2CH_2O$ | Phenyl | - | 0 | |
| 1.61 | $CH_2CH_2CH_2CH_2CH_2O$ | Phenyl | 4-F | 1 | |
| 1.62 | $CH_2CH_2CH_2CH_2CH_2O$ | Phenyl | 4-Cl | 1 | |

Tabelle B

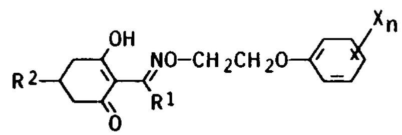

I (A = $-CH_2CH_2O-$; Z = Phenyl)

| Nr. | $R^1$ | $R^2$ | $X_n$ | phys. Daten / $^1$H-NMR [δ in ppm], Fp. [°C] |
|---|---|---|---|---|
| 2.01 | Ethyl | Tetrahydropyran-3-yl | - | 42- 45 |
| 2.02 | Propyl | Tetrahydropyran-3-yl | - | 3,90 (m,2H), 4,20 (t,2H), 4,40 (m, 2H), 6,80-7,00 (m,3H), 7,13-7,37 (m,2H) |
| 2.03 | Ethyl | Tetrahydropyran-4-yl | - | 106-107 |
| 2.04 | Propyl | Tetrahydropyran-4-yl | - | 72- 73 |
| 2.05 | Ethyl | Tetrahydrothiopyran-3-yl | - | 52- 55 |
| 2.06 | Propyl | Tetrahydrothiopyran-3-yl | - | 92 |
| 2.07 | Ethyl | Tetrahydropyran-3-yl | 2-F | 76- 78 |
| 2.08 | Propyl | Tetrahydropyran-3-yl | 2-F | 72- 77 |
| 2.09 | Ethyl | Tetrahydropyran-4-yl | 2-F | 121-125 |
| 2.10 | Propyl | Tetrahydropyran-4-yl | 2-F | 103-107 |
| 2.11 | Ethyl | Tetrahydrothiopyran-3-yl | 2-F | 82- 86 |
| 2.12 | Propyl | Tetrahydrothiopyran-3-yl | 2-F | 81- 85 |
| 2.13 | Ethyl | Tetrahydropyran-3-yl | 3-F | 62- 68 |
| 2.14 | Propyl | Tetrahydropyran-3-yl | 3-F | 3,90 (m,2H), 4,20 (t,2H), 4,40 (m,2H) 6,70 (m,3H), 7,25 (m,1H), |
| 2.15 | Ethyl | Tetrahydropyran-4-yl | 3-F | 103-109 |
| 2.16 | Propyl | Tetrahydropyran-4-yl | 3-F | 73- 79 |
| 2.17 | Ethyl | Tetrahydrothiopyran-3-yl | 3-F | 4,20 (t,2H), 4,40 (m,2H) 6,70 (m,3H), 7,25 (m,1H) |

Tabelle B (Fortsetzung)

| Nr. | $R^1$ | $R^2$ | $X_n$ | phys. Daten / $^1H$-NMR [δ in ppm], Fp. [°C] |
|---|---|---|---|---|
| 2.18 | Propyl | Tetrahydrothiopyran-3-yl | 3-F | |
| 2.19 | Ethyl | Tetrahydropyran-3-yl | 4-F | 64- 67 |
| 2.20 | Propyl | Tetrahydropyran-3-yl | 4-F | 70- 72 |
| 2.21 | Ethyl | Tetrahydropyran-4-yl | 4-F | 101-103 |
| 2.22 | Propyl | Tetrahydropyran-4-yl | 4-F | 107-109 |
| 2.23 | Ethyl | Tetrahydrothiopyran-3-yl | 4-F | 105-108 |
| 2.24 | Propyl | Tetrahydrothiopyran-3-yl | 4-F | 82- 84 |
| 2.25 | Ethyl | Tetrahydropyran-3-yl | 2-Cl | 74- 80 |
| 2.26 | Propyl | Tetrahydropyran-3-yl | 2-Cl | 67- 71 |
| 2.27 | Ethyl | Tetrahydropyran-4-yl | 2-Cl | 4,00 (m,2H), 4,27 (t,2H), 4,47 (m,2H), 7,20 (t,1H), 7,37 (d,1H) |
| 2.28 | Propyl | Tetrahydropyran-4-yl | 2-Cl | 68- 72 |
| 2.29 | Ethyl | Tetrahydrothiopyran-3-yl | 2-Cl | 74- 78 |
| 2.30 | Propyl | Tetrahydrothiopyran-3-yl | 2-Cl | 72-78 |
| 2.31 | Ethyl | Tetrahydropyran-3-yl | 3-Cl | |
| 2.32 | Propyl | Tetrahydropyran-3-yl | 3-Cl | |
| 2.33 | Ethyl | Tetrahydropyran-4-yl | 3-Cl | |
| 2.34 | Propyl | Tetrahydropyran-4-yl | 3-Cl | |
| 2.35 | Ethyl | Tetrahydrothiopyran-3-yl | 3-Cl | |
| 2.36 | Propyl | Tetrahydrothiopyran-3-yl | 3-Cl | |
| 2.37 | Ethyl | Tetrahydropyran-3-yl | 4-Cl | 3,93 (m,2H), 4,20 (t,2H), 4,43 (m,2H), 6,90 (m, 2H), 7,25 (m,2H) |
| 2.38 | Propyl | Tetrahydropyran-3-yl | 4-Cl | 3,93 (m,2H), 4,20 (t,2H), 4,43 (m,2H), 6,90 (m, 2H), 7,25 (m,2H) |

Tabelle B (Fortsetzung)

| Nr. | $R^1$ | $R^2$ | $X_n$ | phys. Daten / $^1H$-NMR [$\delta$ in ppm], Fp. [°C] |
|---|---|---|---|---|
| 2.39 | Ethyl | Tetrahydropyran-4-yl | 4-Cl | 116-118 |
| 2.40 | Propyl | Tetrahydropyran-4-yl | 4-Cl | 104-106 |
| 2.41 | Ethyl | Tetrahydrothiopyran-3-yl | 4-Cl | 74- 77 |
| 2.42 | Propyl | Tetrahydrothiopyran-3-yl | 4-Cl | 86- 88 |
| 2.43 | Ethyl | Tetrahydropyran-3-yl | $2-CF_3$ | |
| 2.44 | Propyl | Tetrahydropyran-3-yl | $2-CF_3$ | |
| 2.45 | Ethyl | Tetrahydropyran-4-yl | $2-CF_3$ | |
| 2.46 | Propyl | Tetrahydropyran-4-yl | $2-CF_3$ | |
| 2.47 | Ethyl | Tetrahydrothiopyran-3-yl | $2-CF_3$ | |
| 2.48 | Propyl | Tetrahydrothiopyran-3-yl | $2-CF_3$ | |
| 2.49 | Ethyl | Tetrahydropyran-3-yl | $3-CF_3$ | |
| 2.50 | Propyl | Tetrahydropyran-3-yl | $3-CF_3$ | |
| 2.51 | Ethyl | Tetrahydropyran-4-yl | $3-CF_3$ | |
| 2.52 | Propyl | Tetrahydropyran-4-yl | $3-CF_3$ | |
| 2.53 | Ethyl | Tetrahydrothiopyran-3-yl | $3-CF_3$ | |
| 2.54 | Propyl | Tetrahydrothiopyran-3-yl | $3-CF_3$ | |
| 2.55 | Ethyl | Tetrahydropyran-3-yl | $4-CF_3$ | 72- 77 |
| 2.56 | Propyl | Tetrahydropyran-3-yl | $4-CF_3$ | 3,90 (m,2H), 4,27 (t,2H), 4,47 (m,2H) 7,00 (d,2H), 7,55 (d,2H) |
| 2.57 | Ethyl | Tetrahydropyran-4-yl | $4-CF_3$ | |
| 2.58 | Propyl | Tetrahydropyran-4-yl | $4-CF_3$ | 90- 94 |
| 2.59 | Ethyl | Tetrahydrothiopyran-3-yl | $4-CF_3$ | 73- 79 |
| 2.60 | Propyl | Tetrahydrothiopyran-3-yl | $4-CF_3$ | 4,27 (t,2H), 4,47 (m,2H), 7,00 (d,2H) 7,55 (d,2H) |
| 2.61 | Ethyl | Tetrahydropyran-3-yl | $2,4-Cl_2$ | 73- 75 |

Tabelle B (Fortsetzung)

| Nr. | $R^1$ | $R^2$ | $X_n$ | phys. Daten / $^1H$-NMR [δ in ppm], Fp. [°C] |
|---|---|---|---|---|
| 2.62 | Propyl | Tetrahydropyran-3-yl | 2,4-$Cl_2$ | 69- 73 |
| 2.63 | Ethyl | Tetrahydropyran-4-yl | 2,4-$Cl_2$ | 4,00 (m,2H), 4,25 (t,2H), 4,45 (t,2H) 6,87 (d,1H), 7,17 (d,1H), 7,37 (d,1H) |
| 2.64 | Propyl | Tetrahydropyran-4-yl | 2,4-$Cl_2$ | 4,00 (m,2H), 4,25 (t,2H), 4,45 (t,2H) 6,87 (d,1H), 7,17 (d,1H), 7,37 (d,1H) |
| 2.65 | Ethyl | Tetrahydrothiopyran-3-yl | 2,4-$Cl_2$ | 4,25 (t,2H), 4,45 (t,2H), 6,87 (d,1H) 7,17 (d,1H), 7,37 (d,1H) |
| 2.66 | Propyl | Tetrahydrothiopyran-3-yl | 2,4-$Cl_2$ | 4,25 (t,2H), 4,45 (t,2H), 6,87 (d,1H) 7,17 (d,1H), 7,37 (d,1H) |
| 2.67 | Ethyl | Tetrahydropyran-3-yl | 2,4,6-$Cl_3$ | 90- 93 |
| 2.68 | Propyl | Tetrahydropyran-3-yl | 2,4,6-$Cl_3$ | 83- 87 |
| 2.69 | Ethyl | Tetrahydropyran-4-yl | 2,4,6-$Cl_3$ | 79- 82 |
| 2.70 | Propyl | Tetrahydropyran-4-yl | 2,4,6-$Cl_3$ | 4,00 (m,2H), 4,27 (t,2H), 4,45 (m,2H), 7,32 (s,2H) |
| 2.71 | Ethyl | Tetrahydrothiopyran-3-yl | 2,4,6-$Cl_3$ | 105-108 |
| 2.72 | Propyl | Tetrahydrothiopyran-3-yl | 2,4,6-$Cl_3$ | 4,27 (t,2H), 4,45 (m,2H), 7,82 (s,2H) |
| 2.73 | Ethyl | Tetrahydropyran-3-yl | 4-$NO_2$ | 3,90 (m,2H), 4,32 (m,2H), 4,50 (m,2H), 7,00 (d,2H), 8,20 (d,2H) |
| 2.74 | Propyl | Tetrahydropyran-3-yl | 4-$NO_2$ | 3,90 (m,2H), 4,32 (m,2H), 4,50 (m,2H) 7,00 (d,2H), 8,20 (d,2H) |
| 2.75 | Ethyl | Tetrahydropyran-4-yl | 4-$NO_2$ | 126-129 |
| 2.76 | Propyl | Tetrahydropyran-4-yl | 4-$NO_2$ | 138-141 |
| 2.77 | Ethyl | Tetrahydrothiopyran-3-yl | 4-$NO_2$ | 4,32 (m,2H), 4,50 (m,2H), 7,00 (d,2H), 8,20 (d,2H) |
| 2.78 | Propyl | Tetrahydrothiopyran-3-yl | 4-$NO_2$ | 4,32 (m,2H), 4,50 (m,2H), 7,00 (d,2H) 8,20 (d,2H) |

Tabelle C

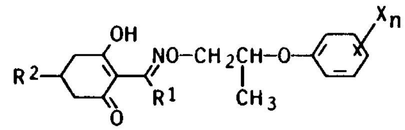 I (A = $CH_2CH(CH_3)$-O-; Z = Phenyl)

| Nr. | $R^1$ | $R^2$ | $X_n$ | phys. Daten / $^1$H-NMR [δ in ppm], Fp. [°C] |
|---|---|---|---|---|
| 3.01 | Ethyl | Tetrahydropyran-3-yl | - | |
| 3.02 | Propyl | Tetrahydropyran-3-yl | - | |
| 3.03 | Ethyl | Tetrahydropyran-4-yl | - | |
| 3.04 | Propyl | Tetrahydropyran-4-yl | - | |
| 3.05 | Ethyl | Tetrahydrothiopyran-3-yl | - | |
| 3.06 | Propyl | Tetrahydrothiopyran-3-yl | - | |
| 3.07 | Ethyl | Tetrahydropyran-3-yl | 4-F | |
| 3.08 | Propyl | Tetrahydropyran-3-yl | 4-F | |
| 3.09 | Ethyl | Tetrahydropyran-4-yl | 4-F | |
| 3.10 | Propyl | Tetrahydropyran-4-yl | 4-F | |
| 3.11 | Ethyl | Tetrahydrothiopyran-3-yl | 4-F | |
| 3.12 | Propyl | Tetrahydrothiopyran-3-yl | 4-F | |
| 3.13 | Ethyl | Tetrahydropyran-3-yl | 4-Cl | |
| 3.14 | Propyl | Tetrahydropyran-3-yl | 4-Cl | |
| 3.15 | Ethyl | Tetrahydropyran-4-yl | 4-Cl | 1,35 (m,3H), 4,05-4,30 (m,2H), 4,60 (m,1H), 6,80-7,40 (m,4H) |
| 3.16 | Propyl | Tetrahydropyran-4-yl | 4-Cl | 1,35 (m,3H), 4,05-4,30 (m,2H), 4,60 (m,1H), 6,80-7,40 (m,4H) |
| 3.17 | Ethyl | Tetrahydrothiopyran-3-yl | 4-Cl | 1,35 (m,3H), 4,05-4,25 (m,2H), 4,60 (m,1H), 6,80-7,40 (m,4H) |
| 3.18 | Propyl | Tetrahydrothiopyran-3-yl | 4-Cl | 1,35 (m,3H), 4,05-4,30 (m,2H), 4,60 (m,1H), 6,80-7,40 (m,4H) |

Tabelle D

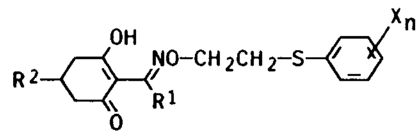

I (A = $-CH_2CH_2-S-$; Z = Phenyl)

| Nr. | $R^1$ | $R^2$ | $X_n$ | phys. Daten / $^1H$-NMR [δ in ppm], Fp. [°C] |
|---|---|---|---|---|
| 4.01 | Ethyl | Tetrahydropyran-3-yl | - | 3,90 (m, 2H), 4,23 (t, 2H), 7,17-7,43 (m, 5H) |
| 4.02 | Propyl | Tetrahydropyran-3-yl | - | 65 |
| 4.03 | Ethyl | Tetrahydropyran-4-yl | - | 3,97 (m, 2H), 4,23 (t, 2H), 7,17-7,43 (m, 5H) |
| 4.04 | Propyl | Tetrahydropyran-4-yl | - | 3,97 (m, 2H), 4,23 (t, 2H), 7,17-7,43 (m, 5H) |
| 4.05 | Ethyl | Tetrahydrothiopyran-3-yl | - | 4,23 (t, 2H), 7,17-7,43 (m, 5H) |
| 4.06 | Propyl | Tetrahydrothiopyran-3-yl | - | 4,23 (t, 2H), 7,17-7,43 (m, 5H) |
| 4.07 | Ethyl | Tetrahydropyran-3-yl | 4-F | 3,90 (m, 2H), 4,17 (t, 2H), 7,00 (m, 2H), 7,40 (m, 2H) |
| 4.08 | Propyl | Tetrahydropyran-3-yl | 4-F | 3,90 (m, 2H), 4,17 (t, 2H), 7,00 (m, 2H), 7,40 (m, 2H) |
| 4.09 | Ethyl | Tetrahydropyran-4-yl | 4-F | 4,00 (m, 2H), 4,17 (t, 2H), 7,00 (m, 2H), 7,40 (m, 2H) |
| 4.10 | Propyl | Tetrahydropyran-4-yl | 4-F | 4,00 (m, 2H), 4,17 (t, 2H), 7,00 (m, 2H), 7,40 (m, 2H) |
| 4.11 | Ethyl | Tetrahydrothiopyran-3-yl | 4-F | 4,17 (t, 2H), 7,00 (m, 2H), 7,40 (m, 2H), |
| 4.12 | Propyl | Tetrahydrothiopyran-3-yl | 4-F | 4,17 (t, 2H), 7,00 (m, 2H), 7,40 (m, 2H), |
| 4.13 | Ethyl | Tetrahydropyran-3-yl | 4-Cl | 71- 75 |

Tabelle D (Fortsetzung)

| Nr. | $R^1$ | $R^2$ | $X_n$ | phys. Daten / $^1H$-NMR [$\delta$ in ppm], Fp. [°C] |
|---|---|---|---|---|
| 4.14 | Propyl | Tetrahydropyran-3-yl | 4-Cl | 63- 65 |
| 4.15 | Ethyl | Tetrahydropyran-4-yl | 4-Cl | 4,00 (m, 2H), 4,20 (t, 2H), 7,30 (m, 4H) |
| 4.16 | Propyl | Tetrahydropyran-4-yl | 4-Cl | 4,00 (m, 2H), 4,20 (t, 2H), 7,30 (m, 4H) |
| 4.17 | Ethyl | Tetrahydrothiopyran-3-yl | 4-Cl | 4,20 (t, 2H), 7,30 (m, 4H) |
| 4.18 | Propyl | Tetrahydrothiopyran-3-yl | 4-Cl | 4,20 (t, 2H), 7,30 (m, 4H) |
| 4.19 | Ethyl | Tetrahydropyran-3-yl | 2-Cl | 3,90 (m, 2H), 4,25 (t, 2H), 7,10-7,50 (m, 4H) |
| 4.20 | Propyl | Tetrahydropyran-3-yl | 2-Cl | 3,90 (m, 2H), 4,25 (t, 2H), 7,10-7,50 (m, 4H) |
| 4.21 | Ethyl | Tetrahydropyran-4-yl | 2-Cl | 4,00 (m, 2H), 4,25 (t, 2H), 7,10-7,50 (m, 4H) |
| 4.22 | Propyl | Tetrahydropyran-4-yl | 2-Cl | 4,00 (m, 2H), 4,25 (t, 2H), 7,10-7,50 (m, 4H) |
| 4.23 | Ethyl | Tetrahydrothiopyran-3-yl | 2-Cl | 4,25 (t, 2H) 7,10-7,50 (m, 4H) |
| 4.24 | Propyl | Tetrahydrothiopyran-3-yl | 2-Cl | 4,25 (t, 2H) 7,10-7,50 (m, 4H) |
| 4.25 | Ethyl | Tetrahydropyran-3-yl | 2,6-$Cl_2$ | 3,90 (m, 2H) 4,20 (t, 2H), 7,20 (t, 1H) 7,40 (d, 2H) |
| 4.26 | Propyl | Tetrahydropyran-3-yl | 2,6-$Cl_2$ | 3,90 (m, 2H) 4,20 (t, 2H), 7,20 (t, 1H) 7,40 (d, 2H) |
| 4.27 | Ethyl | Tetrahydropyran-4-yl | 2,6-$Cl_2$ | 61- 64 |
| 4.28 | Propyl | Tetrahydropyran-4-yl | 2,6-$Cl_2$ | 4,00 (m, 2H) 4,20 (t, 2H), 7,20 (t, 1H) 7,40 (d, 2H) |
| 4.29 | Ethyl | Tetrahydrothiopyran-3-yl | 2,6-$Cl_2$ | 4,20 (t, 2H) 7,20 (t, 2H), 7,40 (d, 2H) |
| 4.30 | Propyl | Tetrahydrothiopyran-3-yl | 2,6-$Cl_2$ | 4,20 (t, 2H) 7,20 (t, 2H), 7,40 (d, 2H) |

Tabelle E

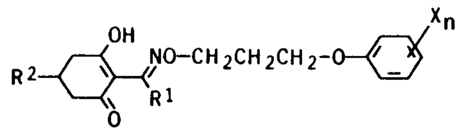 I (A = $-CH_2CH_2-CH_2O-$; Z = Phenyl)

| Nr. | $R^1$ | $R^2$ | $X_n$ | phys. Daten / $^1$H-NMR [δ in ppm], Fp. [°C] |
|---|---|---|---|---|
| 5.01 | Ethyl | Tetrahydropyran-3-yl | - | 3,90 (m,2H), 4,03 (t,2H), 4,23 (t,2H), 6,90 (m,3H), 7,27 (m,2H) |
| 5.02 | Propyl | Tetrahydropyran-3-yl | - | 3,90 (m,2H), 4,03 (t,2H), 4,23 (t,2H), 6,90 (m,3H), 7,27 (m,2H) |
| 5.03 | Ethyl | Tetrahydropyran-4-yl | - | 3,97 (m,2H), 4,03 (t,2H), 4,23 (t,2H), 6,90 (m,3H), 7,27 (m,2H) |
| 5.04 | Propyl | Tetrahydropyran-4-yl | - | 3,97 (m,2H), 4,03 (t,2H), 4,23 (t,2H), 6,90 (m,3H), 7,27 (m,2H) |
| 5.05 | Ethyl | Tetrahydrothiopyran-3-yl | - | 4,03 (t,2H), 4,23 (t,2H), 6,90 (m,3H), 7,27 (m,2H) |
| 5.06 | Propyl | Tetrahydrothiopyran-3-yl | - | 4,03 (t,2H), 4,23 (t,2H), 6,90 (m,3H), 7,27 (m,2H) |
| 5.07 | Ethyl | Tetrahydropyran-3-yl | 2-F | 3,90 (m,2H), 4,10 (t,2H), 4,27 (t,2H), 6,80-7,15 (m,4H) |
| 5.08 | Propyl | Tetrahydropyran-3-yl | 2-F | 3,90 (m,2H), 4,10 (t,2H), 4,27 (t,2H), 6,80-7,15 (m,4H) |
| 5.09 | Ethyl | Tetrahydropyran-4-yl | 2-F | 4,00 (m,2H), 4,10 (t,2H), 4,27 (t,2H), 6,80-7,15 (m,4H) |
| 5.10 | Propyl | Tetrahydropyran-4-yl | 2-F | 76- 80 |

Tabelle E (Fortsetzung)

| Nr. | $R^1$ | $R^2$ | $X_n$ | phys. Daten / $^1H$-NMR [δ in ppm], Fp. [°C] |
|---|---|---|---|---|
| 5.11 | Ethyl | Tetrahydrothiopyran-3-yl | 2-F | 4,10 (t,2H), 4,27 (t,2H), 6,80-7,15(m,4H), |
| 5.12 | Propyl | Tetrahydrothiopyran-3-yl | 2-F | 4,10 (t,2H), 4,27 (t,2H), 6,80-7,15(m,4H), |
| 5.13 | Ethyl | Tetrahydropyran-3-yl | 3-F | 3,90 (m,2H), 4,05 (t,2H), 4,27 (t,2H), 6,67 (m,3H), 7,23 (m,1H) |
| 5.14 | Propyl | Tetrahydropyran-3-yl | 3-F | 3,90 (m,2H), 4,05 (t,2H), 4,27 (t,2H), 6,67 (m,3H), 7,23 (m,1H) |
| 5.15 | Ethyl | Tetrahydropyran-4-yl | 3-F | 3,90-4,10 (m,4H), 4,27 (t,2H), 6,67 (m,3H), 7,23 (m,1H) |
| 5.16 | Propyl | Tetrahydropyran-4-yl | 3-F | 3,90-4,10 (m,4H), 4,27 (t,2H), 6,67 (m,3H), 7,23 (m,1H) |
| 5.17 | Ethyl | Tetrahydrothiopyran-3-yl | 3-F | 4,05 (t,2H), 4,27 (t,2H), 6,67 (m,3H) 7,23 (m,1H) |
| 5.18 | Propyl | Tetrahydrothiopyran-3-yl | 3-F | 4,05 (t,2H), 4,27 (t,2H), 6,67 (m,3H) 7,23 (m,1H) |
| 5.19 | Ethyl | Tetrahydropyran-3-yl | 4-F | 3,90 (m,2H), 4,03 (t,2H), 4,27 (t,2H), 6,90 (m,2H), 7,00 (m,2H) |
| 5.20 | Propyl | Tetrahydropyran-3-yl | 4-F | 3,90 (m,2H), 4,03 (t,2H), 4,27 (t,2H), 6,90 (m,2H), 7,00 (m,2H) |
| 5.21 | Ethyl | Tetrahydropyran-4-yl | 4-F | 3,90-4,06 (m,4H), 4,23 (t,2H), 6,90 (m,2H), 7,00 (m,2H) |
| 5.22 | Propyl | Tetrahydropyran-4-yl | 4-F | 3,90-4,06 (m,4H), 4,28 (t,2H), 6,90 (m,2H), 7,00 (m,2H) |

Tabelle E (Fortsetzung)

| Nr. | $R^1$ | $R^2$ | $X_n$ | phys. Daten / $^1H$-NMR [$\delta$ in ppm], Fp. [°C] |
|---|---|---|---|---|
| 5.23 | Ethyl | Tetrahydrothiopyran-3-yl | 4-F | 4,03 (t,2H), 4,27 (t,2H), 6,90 (m,2H), 7,00 (m,2H) |
| 5.24 | Propyl | Tetrahydrothiopyran-3-yl | 4-F | 4,03 (t,2H), 4,27 (t,2H), 6,90 (m,2H), 7,00 (m,2H) |
| 5.25 | Ethyl | Tetrahydropyran-3-yl | 2-Cl | |
| 5.26 | Propyl | Tetrahydropyran-3-yl | 2-Cl | |
| 5.27 | Ethyl | Tetrahydropyran-4-yl | 2-Cl | |
| 5.28 | Propyl | Tetrahydropyran-4-yl | 2-Cl | |
| 5.29 | Ethyl | Tetrahydrothiopyran-3-yl | 2-Cl | |
| 5.30 | Propyl | Tetrahydrothiopyran-3-yl | 2-Cl | |
| 5.31 | Ethyl | Tetrahydropyran-3-yl | 3-Cl | 3,90 (m,2H), 4,06 (t,2H), 4,27 (t,2H), 6,77 (m,1H), 6,90 (m,2H), 7,17 (m,1H) |
| 5.32 | Propyl | Tetrahydropyran-3-yl | 3-Cl | 3,90 (m,2H), 4,06 (t,2H), 4,27 (t,2H), 6,77 (m,1H), 6,90 (m,2H), 7,17 (m,1H) |
| 5.33 | Ethyl | Tetrahydropyran-4-yl | 3-Cl | 3,90-4,10 (m,4H), 4,27 (t,2H), 6,77 (m,1H), 6,90 (m,2H), 7,17 (m,1H) |
| 5.34 | Propyl | Tetrahydropyran-4-yl | 3-Cl | 3,90-4,10 (m,4H), 4,27 (t,2H), 6,77 (m,1H), 6,90 (m,2H), 7,17 (m,1H) |
| 5.35 | Ethyl | Tetrahydrothiopyran-3-yl | 3-Cl | 4,06 (t,2H), 4,27 (t,2H), 6,77 (m,1H), 6,90 (m,2H), 7,17 (m,1H) |

Tabelle E (Fortsetzung)

| Nr. | $R^1$ | $R^2$ | $X_n$ | phys. Daten / $^1$H-NMR [δ in ppm], Fp. [°C] |
|---|---|---|---|---|
| 5.36 | Propyl | Tetrahydrothiopyran-3-yl | 3-Cl | 4,06 (t,2H), 4,27 (t,2H), 6,77 (m,1H), 6,90 (m,2H), 7,17 (m,1H) |
| 5.37 | Ethyl | Tetrahydropyran-3-yl | 4-Cl | 3,90 (m,2H), 4,03 (t,2H), 4,23 (t,2H), 6,80 (m,2H), 7,20 (m,2H) |
| 5.38 | Propyl | Tetrahydropyran-3-yl | 4-Cl | 3,90 (m,2H), 4,03 (t,2H), 4,23 (t,2H), 6,80 (m,2H), 7,20 (m,2H) |
| 5.39 | Ethyl | Tetrahydropyran-4-yl | 4-Cl | 3,90-4,09 (m,4H), 4,23 (t,2H), 6,80 (m,2H), 7,20 (m,2H) |
| 5.40 | Propyl | Tetrahydropyran-4-yl | 4-Cl | 3,90-4,09 (m,4H), 4,23 (t,2H), 6,80 (m,2H), 7,20 (m,2H) |
| 5.41 | Ethyl | Tetrahydrothiopyran-3-yl | 4-Cl | 4,03 (t,2H), 4,23 (t,2H), 6,80 (m,2H), 7,20 (m,2H) |
| 5.42 | Propyl | Tetrahydrothiopyran-3-yl | 4-Cl | 4,03 (t,2H), 4,23 (t,2H), 6,80 (m,2H), 7,20 (m,2H) |
| 5.43 | Ethyl | Tetrahydropyran-3-yl | 4-$NO_2$ | 3,90 (m,2H), 4,20 (t,2H), 4,28 (t,2H), 6,93 (d,2H), 8,20 (d,H) |
| 5.44 | Propyl | Tetrahydropyran-3-yl | 4-$NO_2$ | 3,90 (m,2H), 4,20 (t,2H), 4,28 (t,2H), 6,93 (d,2H), 8,20 (d,2H) |
| 5.45 | Ethyl | Tetrahydropyran-4-yl | 4-$NO_2$ | 4,00 (m,2H), 4,20 (t,2H), 4,28 (t,2H), 6,93 (d,2H), 8,20 (d,2H) |

Tabelle E (Fortsetzung)

| Nr. | $R^1$ | $R^2$ | $X_n$ | phys. Daten / $^1$H-NMR [δ in ppm], Fp. [°C] |
|---|---|---|---|---|
| 5.46 | Propyl | Tetrahydropyran-4-yl | 4-$NO_2$ | 4,00 (m,2H), 4,20 (t,2H), 4,28 (t,2H), 6,93 (d,2H), 8,20 (d,2H) |
| 5.47 | Ethyl | Tetrahydrothiopyran-3-yl | 4-$NO_2$ | 4,20 (t,2H), 4,28 (t,2H), 6,93 (d,2H), 8,20 (d,2H) |
| 5.48 | Propyl | Tetrahydrothiopyran-3-yl | 4-$NO_2$ | 4,20 (t,2H), 4,28 (t,2H), 6,93 (d,2H), 8,20 (d,2H) |
| 5.49 | Ethyl | Tetrahydropyran-3-yl | 4-Br | 3,90 (m,2H), 4,00 (t,2H), 4,27 (t,2H), 6,80 (d,2H), 7,37 (d,2H) |
| 5.50 | Propyl | Tetrahydropyran-3-yl | 4-Br | 3,90 (m,2H), 4,00 (t,2H), 4,27 (t,2H), 6,80 (d,2H), 7,37 (d,2H) |
| 5.51 | Ethyl | Tetrahydropyran-4-yl | 4-Br | 3,90-4,10 (m,4H), 4,27 (t,2H), 6,80 (d,2H), 7,37 (d,2H) |
| 5.52 | Propyl | Tetrahydropyran-4-yl | 4-Br | 3,90-4,10 (m,4H), 4,27 (t,2H), 6,80 (d,2H), 7,37 (d,2H) |
| 5.53 | Ethyl | Tetrahydrothiopyran-4-yl | 4-Br | 4,00 (t,2H), 4,27 (t,2H), 6,80 (d,2H), 7,37 (d,2H) |
| 5.54 | Propyl | Tetrahydrothiopyran-4-yl | 4-Br | 4,00 (t,2H), 4,27 (t,2H), 6,80 (d,2H), 7,37 (d,2H) |

Tabelle F

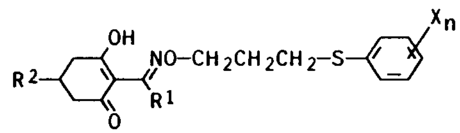 I (A = $-CH_2CH_2CH_2-S-$; Z = Phenyl)

| Nr. | $R^1$ | $R^2$ | $X_n$ | phys. Daten / $^1H$-NMR [δ in ppm], Fp. [°C] |
|---|---|---|---|---|
| 6.01 | Ethyl | Tetrahydropyran-3-yl | - | 3,90 (m,2H), 4,17 (t,2H), 7,10-7,40 (m,5H) |
| 6.02 | Propyl | Tetrahydropyran-3-yl | - | 3,90 (m,2H), 4,17 (t,2H), 7,10-7,40 (m,5H) |
| 6.03 | Ethyl | Tetrahydropyran-4-yl | - | 4,00 (m,2H), 4,17 (t,2H), 7,10-7,40 (m,5H) |
| 6.04 | Propyl | Tetrahydropyran-4-yl | - | 4,00 (m,2H), 4,17 (t,2H), 7,10-7,40 (m,5H) |
| 6.05 | Ethyl | Tetrahydrothiopyran-3-yl | - | 4,17 (t,2H), 7,10-7,40 (m,5H) |
| 6.06 | Propyl | Tetrahydrothiopyran-3-yl | - | 4,17 (t,2H), 7,10-7,40 (m,5H) |
| 6.07 | Ethyl | Tetrahydropyran-3-yl | 4-F | 3,90 (m,2H), 4,17 (t,2H), 7,00 (t,2H), 7,33 (m,2H) |
| 6.08 | Propyl | Tetrahydropyran-3-yl | 4-F | 3,90 (m,2H), 4,17 (t,2H), 7,00 (t,2H), 7,33 (m,2H) |
| 6.09 | Ethyl | Tetrahydropyran-4-yl | 4-F | 4,00 (m,2H), 4,17 (t,2H), 7,00 (t,2H), 7,33 (m,2H) |
| 6.10 | Propyl | Tetrahydropyran-4-yl | 4-F | 4,00 (m,2H), 4,17 (t,2H), 7,00 (t,2H), 7,33 (m,2H) |
| 6.11 | Ethyl | Tetrahydrothiopyran-3-yl | 4-F | 4,17 (t,2H), 7,00 (t,2H), 7,33 (m,2H) |
| 6.12 | Propyl | Tetrahydrothiopyran-3-yl | 4-F | 4,17 (t,2H), 7,00 (t,2H), 7,33 (m,2H) |
| 6.13 | Ethyl | Tetrahydropyran-3-yl | 4-Cl | 3,90 (m,2H), 4,17 (t,2H), 7,27 (s,4H) |
| 6.14 | Propyl | Tetrahydropyran-3-yl | 4-Cl | 3,90 (m,2H), 4,17 (t,2H), 7,27 (s,4H) |

Tabelle F (Fortsetzung)

| Nr. | $R^1$ | $R^2$ | $X_n$ | phys. Daten / $^1H$-NMR [$\delta$ in ppm], Fp. [°C] |
|---|---|---|---|---|
| 6.15 | Ethyl | Tetrahydropyran-4-yl | 4-Cl | 4,00 (m,2H), 4,17 (t,2H), 7,27 (s,4H) |
| 6.16 | Propyl | Tetrahydropyran-4-yl | 4-Cl | 4,00 (m,2H), 4,17 (t,2H), 7,27 (s,4H) |
| 6.17 | Ethyl | Tetrahydrothiopyran-3-yl | 4-Cl | 4,17 (t,2H), 7,27 (s,4H) |
| 6.18 | Propyl | Tetrahydrothiopyran-3-yl | 4-Cl | 4,17 (t,2H), 7,27 (s,4H) |
| 6.19 | Ethyl | Tetrahydropyran-3-yl | 2-Cl | 3,90 (m,2H), 4,20 (t,2H), 7,07-7,40 (m,4H) |
| 6.20 | Propyl | Tetrahydropyran-3-yl | 2-Cl | 3,90 (m,2H), 4,20 (t,2H), 7,07-7,40 (m,4H) |
| 6.21 | Ethyl | Tetrahydropyran-4-yl | 2-Cl | 4,00 (m,2H), 4,20 (t,2H), 7,07-7,40 (m,4H) |
| 6.22 | Propyl | Tetrahydropyran-4-yl | 2-Cl | 4,00 (m,2H), 4,20 (t,2H), 7,07-7,40 (m,4H) |
| 6.23 | Ethyl | Tetrahydrothiopyran-3-yl | 2-Cl | 4,20 (t,2H), 7,07-7,40 (m,4H) |
| 6.24 | Propyl | Tetrahydrothiopyran-3-yl | 2-Cl | 4,20 (t,2H), 7,07-7,40 (m,4H) |
| 6.25 | Ethyl | Tetrahydropyran-3-yl | 3-Cl | 3,90 (m,2H), 4,20 (t,2H), 7,17 (m,3H) 7,30 (m,1H) |
| 6.26 | Propyl | Tetrahydropyran-3-yl | 3-Cl | 3,90 (m,2H), 4,20 (t,2H), 7,17 (m,3H) 7,30 (m,1H) |
| 6.27 | Ethyl | Tetrahydropyran-4-yl | 3-Cl | 4,00 (m,2H), 4,20 (t,2H), 7,17 (m,3H) 7,30 (m,1H) |
| 6.28 | Propyl | Tetrahydropyran-4-yl | 3-Cl | 4,00 (m,2H), 4,20 (t,2H), 7,17 (m,3H) 7,30 (m,1H) |

Tabelle F (Fortsetzung)

| Nr. | $R^1$ | $R^2$ | $X_n$ | phys. Daten / $^1$H-NMR [δ in ppm], Fp. [°C] |
|---|---|---|---|---|
| 6.29 | Ethyl | Tetrahydrothiopyran-3-yl | 3-Cl | 4,20 (t,2H), 7,17 (m,3H), 7,30 (m,1H) |
| 6.30 | Propyl | Tetrahydrothiopyran-3-yl | 3-Cl | 4,20 (t,2H), 7,17 (m,3H), 7,30 (m,1H) |
| 6.31 | Ethyl | Tetrahydropyran-3-yl | 2,5-$Cl_2$ | 3,90 (m,2H), 4,20 (t,2H), 7,07 (dd,1H), 7,20 (d,1H) 7,30 (d,1H) |
| 6.32 | Propyl | Tetrahydropyran-3-yl | 2,5-$Cl_2$ | 3,90 (m,2H), 4,20 (t,2H), 7,07 (dd,1H), 7,20 (d,1H) 7,30 (d,1H) |
| 6.33 | Ethyl | Tetrahydropyran-4-yl | 2,5-$Cl_2$ | 4,00 (m,2H), 4,20 (t,2H), 7,07 (dd,1H), 7,20 (d,1H) 7,30 (d,1H) |
| 6.34 | Propyl | Tetrahydropyran-4-yl | 2,5-$Cl_2$ | 4,00 (m,2H), 4,20 (t,2H), 7,07 (dd,1H), 7,20 (d,1H) 7,30 (d,1H) |
| 6.35 | Ethyl | Tetrahydrothiopyran-3-yl | 2,5-$Cl_2$ | 4,20 (t,2H), 7,07 (dd,1H), 7,20 (d,1H) 7,30 (d,1H) |
| 6.36 | Propyl | Tetrahydrothiopyran-3-yl | 2,5-$Cl_2$ | 4,20 (t,2H), 7,07 (dd,1H), 7,20 (d,1H) 7,30 (d,1H) |
| 6.37 | Ethyl | Tetrahydropyran-3-yl | 2,6-$Cl_2$ | 3,90 (m,2H), 4,20 (t,2H), 7,20 (t,1H) 7,40 (d,2H) |
| 6.38 | Propyl | Tetrahydropyran-3-yl | 2,6-$Cl_2$ | 3,90 (m,2H), 4,20 (t,2H), 7,20 (t,1H) 7,40 (d,2H) |

Tabelle F (Fortsetzung)

| Nr. | $R^1$ | $R^2$ | $X_n$ | phys. Daten / $^1H$-NMR [δ in ppm], Fp. [°C] |
|---|---|---|---|---|
| 6.39 | Ethyl | Tetrahydropyran-4-yl | 2,6-$Cl_2$ | 4,00 (m,2H), 4,20 (t,2H), 7,20 (t,1H) 7,40 (d,2H) |
| 6.40 | Propyl | Tetrahydropyran-4-yl | 2,6-$Cl_2$ | 4,00 (m,2H), 4,20 (t,2H), 7,20 (t,1H) 7,40 (d,2H) |
| 6.41 | Ethyl | Tetrahydrothiopyran-3-yl | 2,6-$Cl_2$ | 4,20 (t,2H), 7,20 (t,1H), 7,40 (d,2H) |
| 6.42 | Propyl | Tetrahydrothiopyran-3-yl | 2,6-$Cl_2$ | 4,20 (t,2H), 7,20 (t,1H), 7,40 (d,2H) |

Tabelle G

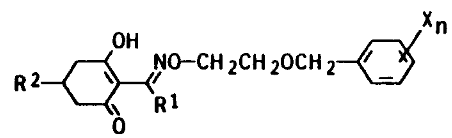

I (A = $-CH_2CH_2-O-CH_2-$; Z = Phenyl)

| Nr. | $R^1$ | $R^2$ | $X_n$ | phys. Daten / $^1H$-NMR [δ in ppm], Fp. [°C] |
|---|---|---|---|---|
| 7.01 | Ethyl | Tetrahydropyran-3-yl | - | 3,90 (m,2H), 4,25 (t,2H), 4,58 (s,2H), 7,38 (s,5H) |
| 7.02 | Propyl | Tetrahydropyran-3-yl | - | 3,90 (m,2H), 4,25 (t,2H), 4,58 (s,2H), 7,38 (s,5H) |
| 7.03 | Ethyl | Tetrahydropyran-4-yl | - | 4,03 (m,2H), 4,33 (m,2H), 4,60 (s,2H), 7,40 (s,5H) |
| 7.04 | Propyl | Tetrahydropyran-4-yl | - | 4,03 (m,2H), 4,33 (m,2H), 4,60 (s,2H), 7,40 (s,5H) |
| 7.05 | Ethyl | Tetrahydrothiopyran-3-yl | - | 4,27 (m,2H), 4,57 (s,2H), 7,35 (s,5H) |
| 7.06 | Propyl | Tetrahydrothiopyran-3-yl | - | 4,27 (m,2H), 4,57 (s,2H), 7,35 (s,5H) |
| 7.07 | Ethyl | Tetrahydropyran-3-yl | 2-F | 3,93 (m,2H), 4,27 (m,2H), 4,67 (s,2H), 6,93-7,50 (m,4H) |
| 7.08 | Propyl | Tetrahydropyran-3-yl | 2-F | 3,93 (m,2H), 4,27 (m,2H), 4,67 (s,2H), 6,93-7,50 (m,4H) |
| 7.09 | Ethyl | Tetrahydropyran-4-yl | 2-F | 4,03 (m,2H), 4,27 (m,2H), 4,63 (s,2H), 6,97-7,50 (m,4H) |
| 7.10 | Propyl | Tetrahydropyran-4-yl | 2-F | 4,03 (m,2H), 4,27 (m,2H), 4,63 (s,2H), 6,97-7,50 (m,4H) |

Tabelle G (Fortsetzung)

| Nr. | $R^1$ | $R^2$ | $X_n$ | phys. Daten / $^1H$-NMR [$\delta$ in ppm], Fp. [°C] |
|---|---|---|---|---|
| 7.11 | Ethyl | Tetrahydrothiopyran-3-yl | 2-F | 4,27 (m,2H), 4,67 (s,2H), 6,97-7,50 (m,4H) |
| 7.12 | Propyl | Tetrahydrothiopyran-3-yl | 2-F | 4,27 (m,2H), 4,67 (s,2H), 6,97-7,50 (m,4H) |
| 7.13 | Ethyl | Tetrahydropyran-3-yl | 3-F | 3,93 (m,2H), 4,27 (m,2H), 4,57 (s,2H), 6,90-7,15 (m,3H), 7,23-7,40 (m,1H) |
| 7.14 | Propyl | Tetrahydropyran-3-yl | 3-F | 3,93 (m,2H), 4,27 (m,2H), 4,57 (s,2H), 6,90-7,15 (m,3H), 7,23-7,40 (m,1H) |
| 7.15 | Ethyl | Tetrahydropyran-4-yl | 3-F | 4,03 (m,2H), 4,25 (m,2H), 4,60 (s,2H), 6,90-7,18 (m,3H), 7,26-7,40 (m,1H) |
| 7.16 | Propyl | Tetrahydropyran-4-yl | 3-F | 4,03 (m,2H), 4,25 (m,2H), 4,60 (s,2H), 6,90-7,18 (m,3H), 7,26-7,40 (m,1H) |
| 7.17 | Ethyl | Tetrahydrothiopyran-3-yl | 3-F | 4,27 (m,2H), 4,60 (s,2H), 6,90-7,15 (m,3H), 7,23-7,40 (m,1H) |
| 7.18 | Propyl | Tetrahydrothiopyran-3-yl | 3-F | 4,27 (m,2H), 4,60 (s,2H), 6,90-7,15 (m,3H), 7,23-7,40 (m,1H) |
| 7.19 | Ethyl | Tetrahydropyran-3-yl | 4-F | 3,93 (m,2H), 4,23 (m,2H), 4,53 (s,2H), 7,00 (m,2H), 7,30 (m,2H) |
| 7.20 | Propyl | Tetrahydropyran-3-yl | 4-F | 3,93 (m,2H), 4,23 (m,2H), 4,53 (s,2H), 7,00 (m,2H), 7,30 (m,2H) |
| 7.21 | Ethyl | Tetrahydropyran-4-yl | 4-F | 92 |
| 7.22 | Propyl | Tetrahydropyran-4-yl | 4-F | 4,00 (m,2H), 4,23 (m,2H), 4,53 (s,2H), 7,03 (m,2H), 7,30 (m,2H) |

Tabelle G (Fortsetzung)

| Nr. | $R^1$ | $R^2$ | $X_n$ | phys. Daten / $^1H$-NMR [δ in ppm], Fp. [°C] |
|---|---|---|---|---|
| 7.23 | Ethyl | Tetrahydrothiopyran-3-yl | 4-F | 4,27 (m, 2H), 4,53 (s, 2H), 7,03 (m, 2H), 7,30 (m, 2H) |
| 7.24 | Propyl | Tetrahydrothiopyran-3-yl | 4-F | 4,27 (m, 2H), 4,53 (s, 2H), 7,03 (m, 2H), 7,30 (m, 2H) |
| 7.25 | Ethyl | Tetrahydropyran-3-yl | 2-Cl | |
| 7.26 | Propyl | Tetrahydropyran-3-yl | 2-Cl | |
| 7.27 | Ethyl | Tetrahydropyran-4-yl | 2-Cl | |
| 7.28 | Propyl | Tetrahydropyran-4-yl | 2-Cl | |
| 7.29 | Ethyl | Tetrahydrothiopyran-3-yl | 2-Cl | |
| 7.30 | Propyl | Tetrahydrothiopyran-3-yl | 2-Cl | |
| 7.31 | Ethyl | Tetrahydropyran-3-yl | 3-Cl | |
| 7.32 | Propyl | Tetrahydropyran-3-yl | 3-Cl | |
| 7.33 | Ethyl | Tetrahydropyran-4-yl | 3-Cl | |
| 7.34 | Propyl | Tetrahydropyran-4-yl | 3-Cl | |
| 7.35 | Ethyl | Tetrahydrothiopyran-3-yl | 3-Cl | |
| 7.36 | Propyl | Tetrahydrothiopyran-3-yl | 3-Cl | |
| 7.37 | Ethyl | Tetrahydropyran-3-yl | 4-Cl | 3,93 (m, 2H), 4,27 (m, 2H), 4,53 (s, 2H), 7,28 (m, 4H) |
| 7.38 | Propyl | Tetrahydropyran-3-yl | 4-Cl | 3,93 (m, 2H), 4,27 (m, 2H), 4,53 (s, 2H), 7,28 (m, 4H) |
| 7.39 | Ethyl | Tetrahydropyran-4-yl | 4-Cl | 67- 72 |
| 7.40 | Propyl | Tetrahydropyran-4-yl | 4-Cl | 4,00 (m, 2H), 4,23 (m, 2H), 4,53 (s, 2H), 7,28 (m, 4H) |

Tabelle G (Fortsetzung)

| Nr. | $R^1$ | $R^2$ | $X_n$ | phys. Daten / $^1$H-NMR [δ in ppm], Fp. [°C] |
|---|---|---|---|---|
| 7.41 | Ethyl | Tetrahydrothiopyran-3-yl | 4-Cl | 4,27 (m,2H), 4,53 (s,2H), 7,28 (m,4H) |
| 7.42 | Propyl | Tetrahydrothiopyran-3-yl | 4-Cl | 4,27 (m,2H), 4,53 (s,2H), 7,28 (m,4H) |
| 7.43 | Ethyl | Tetrahydropyran-3-yl | 2-$CH_3$ | 3,93 (m,2H), 4,23 (m,2H), 4,57 (s,2H), 7,09-7,33 (m,4H) |
| 7.44 | Propyl | Tetrahydropyran-3-yl | 2-$CH_3$ | 3,93 (m,2H), 4,23 (m,2H), 4,57 (s,2H), 7,09-7,33 (m,4H) |
| 7.45 | Ethyl | Tetrahydropyran-4-yl | 2-$CH_3$ | 4,00 (m,2H), 4,23 (m,2H), 4,57 (s,2H), 7,09-7,33 (m,4H) |
| 7.46 | Propyl | Tetrahydropyran-4-yl | 2-$CH_3$ | 4,00 (m,2H), 4,23 (m,2H), 4,57 (s,2H), 7,09-7,33 (m,4H) |
| 7.47 | Ethyl | Tetrahydrothiopyran-3-yl | 2-$CH_3$ | 4,23 (m,2H), 4,57 (s,2H), 7,09-7,33 (m,4H) |
| 7.48 | Propyl | Tetrahydrothiopyran-3-yl | 2-$CH_3$ | 4,23 (m,2H), 4,57 (s,2H), 7,09-7,33 (m,4H) |
| 7.49 | Ethyl | Tetrahydropyran-3-yl | 3-$CH_3$ | 3,93 (m,2H), 4,25 (m,2H), 4,57 (s,2H), 7,00-7,32 (m,4H) |
| 7.50 | Propyl | Tetrahydropyran-3-yl | 3-$CH_3$ | 3,93 (m,2H), 4,25 (m,2H), 4,57 (s,2H), 7,00-7,32 (m,4H) |
| 7.51 | Ethyl | Tetrahydropyran-4-yl | 3-$CH_3$ | 4,00 (m,2H), 4,27 (m,2H), 4,57 (s,2H), 7,00-7,32 (m,4H) |
| 7.52 | Propyl | Tetrahydropyran-4-yl | 3-$CH_3$ | 4,00 (m,2H), 4,27 (m,2H), 4,57 (s,2H), 7,00-7,32 (m,4H) |

Tabelle G (Fortsetzung)

| Nr. | $R^1$ | $R^2$ | $X_n$ | phys. Daten / $^1H$-NMR [δ in ppm], Fp. [°C] |
|---|---|---|---|---|
| 7.53 | Ethyl | Tetrahydrothiopyran-3-yl | $3-CH_3$ | 4,27 (m,2H), 4,60 (s,2H), 7,00-7,32 (m,4H) |
| 7.54 | Propyl | Tetrahydrothiopyran-3-yl | $3-CH_3$ | 4,27 (m,2H), 4,60 (s,2H), 7,00-7,32 (m,4H) |
| 7.55 | Ethyl | Tetrahydropyran-3-yl | $4-CH_3$ | 3,93 (m,2H), 4,20 (m,2H), 4,53 (s,2H), 7,07-7,30 (m,4H) |
| 7.56 | Propyl | Tetrahydropyran-3-yl | $4-CH_3$ | 3,93 (m,2H), 4,20 (m,2H), 4,53 (s,2H), 7,07-7,30 (m,4H) |
| 7.57 | Ethyl | Tetrahydropyran-4-yl | $4-CH_3$ | 4,00 (m,2H), 4,23 (m,2H), 4,57 (s,2H), 7,03-7,27 (m,4H) |
| 7.58 | Propyl | Tetrahydropyran-4-yl | $4-CH_3$ | 4,00 (m,2H), 4,23 (m,2H), 4,57 (s,2H), 7,03-7,27 (m,4H) |
| 7.59 | Ethyl | Tetrahydrothiopyran-3-yl | $4-CH_3$ | 4,23 (m,2H), 4,57 (s,2H), 7,07-7,30 (m,4H) |
| 7.60 | Propyl | Tetrahydrothiopyran-3-yl | $4-CH_3$ | 4,28 (m,2H), 4,57 (s,2H), 7,07-7,30 (m,4H) |
| 7.61 | Ethyl | Tetrahydropyran-3-yl | 4-tert.-$C_4H_9$ | 3,93 (m,2H), 4,23 (m,2H), 4,53 (s,2H), 7,20-7,40 (m,4H) |
| 7.62 | Propyl | Tetrahydropyran-3-yl | 4-tert.-$C_4H_9$ | 3,93 (m,2H), 4,23 (m,2H), 4,53 (s,2H), 7,20-7,40 (m,4H) |
| 7.63 | Ethyl | Tetrahydropyran-4-yl | 4-tert.-$C_4H_9$ | 4,00 (m,2H), 4,23 (m,2H), 4,53 (s,2H), 7,20-7,40 (m,4H) |

Tabelle G (Fortsetzung)

| Nr. | $R^1$ | $R^2$ | $X_n$ | phys. Daten / $^1H$-NMR [δ in ppm], Fp. [°C] |
|---|---|---|---|---|
| 7.64 | Propyl | Tetrahydropyran-4-yl | 4-tert.-$C_4H_9$ | 4,00 (m,2H), 4,23 (m,2H), 4,53 (s,2H), 7,20-7,40 (m,4H) |
| 7.65 | Ethyl | Tetrahydrothiopyran-3-yl | 4-tert.-$C_4H_9$ | 4,23 (m,2H), 4,53 (s,2H), 7,20-7,40 (m,4H) |
| 7.66 | Propyl | Tetrahydrothiopyran-3-yl | 4-tert.-$C_4H_9$ | 4,23 (m,2H), 4,53 (s,2H), 7,20-7,40 (m,4H) |

Tabelle H

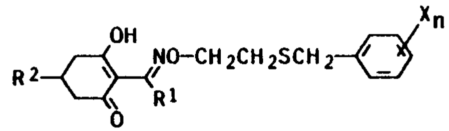

I (A = $-CH_2CH_2-S-CH_2-$; Z = Phenyl)

| Nr. | $R^1$ | $R^2$ | $X_n$ | phys. Daten / $^1$H-NMR [δ in ppm], Fp. [°C] |
|---|---|---|---|---|
| 8.01 | Ethyl | Tetrahydropyran-3-yl | - | 3,73 (s,2H), 3,90 (m,2H), 4,17 (t,2H), 7,28 (s,5H) |
| 8.02 | Propyl | Tetrahydropyran-3-yl | - | 3,73 (s,2H), 3,90 (m,2H), 4,17 (t,2H), 7,28 (s,5H) |
| 8.03 | Ethyl | Tetrahydropyran-4-yl | - | 3,77 (s,2H), 4,00 (m,2H), 4,13 (t,2H), 7,28 (s,5H) |
| 8.04 | Propyl | Tetrahydropyran-4-yl | - | 3,77 (s,2H), 4,00 (m,2H), 4,13 (t,2H), 7,28 (s,5H) |
| 8.05 | Ethyl | Tetrahydrothiopyran-3-yl | - | 3,80 (s,2H), 4,13 (t,2H), 7,28 (s,5H) |
| 8.06 | Propyl | Tetrahydrothiopyran-3-yl | - | 3,80 (s,2H), 4,13 (t,2H), 7,28 (s,5H) |
| 8.07 | Ethyl | Tetrahydropyran-3-yl | 4-F | 3,72 (s,2H), 3,90 (m,2H), 4,13 (t,2H), 7,00 (m,2H), 7,30 (m,2H) |
| 8.08 | Propyl | Tetrahydropyran-3-yl | 4-F | 3,72 (s,2H), 3,90 (m,2H), 4,13 (t,2H), 7,00 (m,2H), 7,30 (m,2H) |
| 8.09 | Ethyl | Tetrahydropyran-4-yl | 4-F | 63- 65 |
| 8.10 | Propyl | Tetrahydropyran-4-yl | 4-F | 3,73 (s,2H), 4,00 (m,2H), 4,13 (t,2H), 7,00 (m,2H), 7,30 (m,2H) |

Tabelle H (Fortsetzung)

| Nr. | $R^1$ | $R^2$ | $X_n$ | phys. Daten / $^1H$-NMR [δ in ppm], Fp. [°C] |
|---|---|---|---|---|
| 8.11 | Ethyl | Tetrahydrothiopyran-3-yl | 4-F | 3,75 (s,2H), 4,13 (t,2H), 7,00 (m,2H), 7,30 (m,2H) |
| 8.12 | Propyl | Tetrahydrothiopyran-3-yl | 4-F | 3,75 (s,2H), 4,13 (t,2H), 7,00 (m,2H), 7,30 (m,2H) |
| 8.13 | Ethyl | Tetrahydropyran-3-yl | 4-Cl | 3,77 (s,2H), 3,93 (m,2H), 4,13 (t,2H), 7,30 (s,4H) |
| 8.14 | Propyl | Tetrahydropyran-3-yl | 4-Cl | 3,77 (s,2H), 3,93 (m,2H), 4,13 (t,2H), 7,30 (s,4H) |
| 8.15 | Ethyl | Tetrahydropyran-4-yl | 4-Cl | 3,73 (s,2H), 4,00 (m,2H), 4,17 (t,2H), 7,30 (s,4H) |
| 8.16 | Propyl | Tetrahydropyran-4-yl | 4-Cl | 3,73 (s,2H), 4,00 (m,2H), 4,17 (t,2H), 7,30 (s,4H) |
| 8.17 | Ethyl | Tetrahydrothiopyran-3-yl | 4-Cl | 3,73 (s,2H), 4,13 (m,2H), 7,30 (s,4H) |
| 8.18 | Propyl | Tetrahydrothiopyran-3-yl | 4-Cl | 3,73 (s,2H), 4,13 (m,2H), 7,30 (s,4H) |

Tabelle I

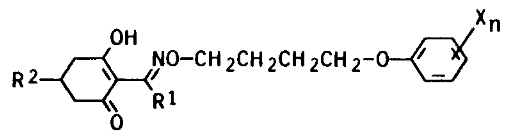

I (A = $-CH_2CH_2CH_2CH_2-O-$; Z = Phenyl)

| Nr. | R1 | R2 | Xn | phys. Daten / $^1$H-NMR [δ in ppm], Fp. [°C] |
|---|---|---|---|---|
| 9.01 | Ethyl | Tetrahydropyran-3-yl | - | 3,70-4,20 (m,6H), 6,90 (m,3H), 7,30 (m,2H) |
| 9.02 | Propyl | Tetrahydropyran-3-yl | - | 3,70-4,20 (m,6H), 6,90 (m,3H), 7,30 (m,2H) |
| 9.03 | Ethyl | Tetrahydropyran-4-yl | - | 3,83-4,23 (m,6H), 6,90 (m,3H), 7,30 (m,2H) |
| 9.04 | Propyl | Tetrahydropyran-4-yl | - | 3,83-4,23 (m,6H), 6,90 (m,3H), 7,30 (m,2H) |
| 9.05 | Ethyl | Tetrahydrothiopyran-3-yl | - | 4,00 (bs,2H), 4,13 (bs,2H), 6,90 (m,3H) 7,30 (m,2H) |
| 9.06 | Propyl | Tetrahydrothiopyran-3-yl | - | 4,00 (bs,2H), 4,13 (bs,2H), 6,90 (m,3H) 7,30 (m,2H) |
| 9.07 | Ethyl | Tetrahydropyran-3-yl | 2-F | 3,93 (m,2H), 4,00-4,20 (m,4H), 6,80-7,15 (m,4H) |
| 9.08 | Propyl | Tetrahydropyran-3-yl | 2-F | 3,93 (m,2H), 4,00-4,20 (m,4H), 6,80-7,15 (m,4H) |
| 9.09 | Ethyl | Tetrahydropyran-4-yl | 2-F | 68- 72 |
| 9.10 | Propyl | Tetrahydropyran-4-yl | 2-F | 3,90-4,20 (m,6H), 6,80-7,15 (m,4H) |

Tabelle I (Fortsetzung)

| Nr. | $R^1$ | $R^2$ | $X_n$ | phys. Daten / $^1H$-NMR [$\delta$ in ppm], Fp. [°C] |
|---|---|---|---|---|
| 9.11 | Ethyl | Tetrahydrothiopyran-3-yl | 2-F | 4,00-4,20 (m,4H), 6,80-7,15 (m,4H) |
| 9.12 | Propyl | Tetrahydrothiopyran-3-yl | 2-F | 4,00-4,20 (m,4H), 6,80-7,15 (m,4H) |
| 9.13 | Ethyl | Tetrahydropyran-3-yl | 3-F | |
| 9.14 | Propyl | Tetrahydropyran-3-yl | 3-F | |
| 9.15 | Ethyl | Tetrahydropyran-4-yl | 3-F | |
| 9.16 | Propyl | Tetrahydropyran-4-yl | 3-F | |
| 9.17 | Ethyl | Tetrahydrothiopyran-3-yl | 3-F | |
| 9.18 | Propyl | Tetrahydrothiopyran-3-yl | 3-F | |
| 9.19 | Ethyl | Tetrahydropyran-3-yl | 4-F | 3,80-4,20 (m,6H), 6,75-7,05 (m,4H) |
| 9.20 | Propyl | Tetrahydropyran-3-yl | 4-F | 3,80-4,20 (m,6H), 6,75-7,05 (m,4H) |
| 9.21 | Ethyl | Tetrahydropyran-4-yl | 4-F | 3,90-4,20 (m,6H), 6,75-7,05 (m,4H) |
| 9.22 | Propyl | Tetrahydropyran-4-yl | 4-F | 3,90-4,20 (m,6H), 6,75-7,05 (m,4H) |
| 9.23 | Ethyl | Tetrahydrothiopyran-3-yl | 4-F | 3,90-4,20 (m,4H), 6,75-7,05 (m,4H) |
| 9.24 | Propyl | Tetrahydrothiopyran-3-yl | 4-F | 3,90-4,20 (m,4H), 6,75-7,05 (m,4H) |
| 9.25 | Ethyl | Tetrahydropyran-3-yl | 4-Cl | 3,80-4,20 (m,6H), 6,80 (m,2H), 7,20 (m,2H) |
| 9.26 | Propyl | Tetrahydropyran-3-yl | 4-Cl | 3,80-4,20 (m,6H), 6,80 (m,2H), 7,20 (m,2H) |
| 9.27 | Ethyl | Tetrahydropyran-4-yl | 4-Cl | 3,90-4,20 (m,6H), 6,80 (m,2H), 7,20 (m,2H) |
| 9.28 | Propyl | Tetrahydropyran-4-yl | 4-Cl | 3,90-4,20 (m,6H), 6,80 (m,2H), 7,20 (m,2H) |

Tabelle I (Fortsetzung)

| Nr. | $R^1$ | $R^2$ | $X_n$ | phys. Daten / $^1$H-NMR [δ in ppm], Fp. [°C] |
|---|---|---|---|---|
| 9.29 | Ethyl | Tetrahydrothiopyran-3-yl | 4-Cl | 3,90-4,20 (m,4H), 6,80 (m,2H), 7,20 (m,2H) |
| 9.30 | Propyl | Tetrahydrothiopyran-3-yl | 4-Cl | 3,90-4,20 (m,4H), 6,80 (m,2H), 7,20 (m,2H) |
| 9.31 | Ethyl | Tetrahydropyran-3-yl | 2,6-$Cl_2$ | 3,93 (m,2H), 4,00-4,25 (m,4H), 7,00 (t,1H), 7,30 (d,2H) |
| 9.32 | Propl | Tetrahydropyran-3-yl | 2,6-$Cl_2$ | 3,93 (m,2H), 4,00-4,25 (m,4H), 7,00 (t,1H), 7,30 (d,2H) |
| 9.33 | Ethyl | Tetrahydropyran-4-yl | 2,6-$Cl_2$ | 3,90-4,25 (m,6H), 7,00 (t,1H), 7,30 (d,2H) |
| 9.34 | Propyl | Tetrahydropyran-4-yl | 2,6-$Cl_2$ | 3,90-4,25 (m,6H), 7,00 (t,1H), 7,30 (d,2H) |
| 9.35 | Ethyl | Tetrahydrothiopyran-3-yl | 2,6-$Cl_2$ | 4,00-4,20 (m,4H), 7,00 (t,1H), 7,30 (d,2H) |
| 9.36 | Propyl | Tetrahydrothiopyran-3-yl | 2,6-$Cl_2$ | 4,00-4,20 (m,4H), 7,00 (t,1H), 7,30 (d,2H) |

Tabelle J

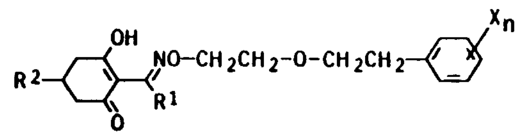

I (A = $-CH_2CH_2-O-CH_2CH_2-$; Z = Phenyl)

| Nr. | $R^1$ | $R^2$ | $X_n$ | phys. Daten / $^1$H-NMR [δ in ppm], Fp. [°C] |
|---|---|---|---|---|
| 10.01 | Ethyl | Tetrahydropyran-3-yl | - | 3,90 (m,2H), 4,20 (m,2H), 7,25 (m,5H) |
| 10.02 | Propyl | Tetrahydropyran-3-yl | - | 3,90 (m,2H), 4,20 (m,2H), 7,25 (m,5H) |
| 10.03 | Ethyl | Tetrahydropyran-4-yl | - | |
| 10.04 | Propyl | Tetrahydropyran-4-yl | - | |
| 10.05 | Ethyl | Tetrahydrothiopyran-3-yl | - | 4,20 (m,2H), 7,25 (m,5H) |
| 10.06 | Propyl | Tetrahydrothiopyran-3-yl | - | 4,20 (m,2H), 7,25 (m,5H) |
| 10.07 | Ethyl | Tetrahydropyran-3-yl | 4-F | 3,90 (m,2H), 4,17 (m,2H), 6,93 (m,2H), 7,13 (m,2H) |
| 10.08 | Propyl | Tetrahydropyran-3-yl | 4-F | 3,90 (m,2H), 4,17 (m,2H), 6,93 (m,2H), 7,13 (m,2H) |
| 10.09 | Ethyl | Tetrahydropyran-4-yl | 4-F | |
| 10.10 | Propyl | Tetrahydropyran-4-yl | 4-F | |
| 10.11 | Ethyl | Tetrahydrothiopyran-3-yl | 4-F | 4,17 (m,2H), 6,93 (m,2H), 7,13 (m,2H) |
| 10.12 | Propyl | Tetrahydrothiopyran-3-yl | 4-F | 4,17 (m,2H), 6,93 (m,2H), 7,13 (m,2H) |
| 10.13 | Ethyl | Tetrahydropyran-3-yl | 4-Cl | 3,90 (m,2H), 4,17 (m,2H), 7,13 (m,4H) |
| 10.14 | Propyl | Tetrahydropyran-3-yl | 4-Cl | 3,90 (m,2H), 4,17 (m,2H), 7,13 (m,4H) |
| 10.15 | Ethyl | Tetrahydropyran-4-yl | 4-Cl | |
| 10.16 | Propyl | Tetrahydropyran-4-yl | 4-Cl | |
| 10.17 | Ethyl | Tetrahydrothiopyran-3-yl | 4-Cl | 4,17 (m,2H), 7,13 (m,4H) |
| 10.18 | Propyl | Tetrahydrothiopyran-3-yl | 4-Cl | 4,17 (m,2H), 7,13 (m,4H) |

Tabelle K

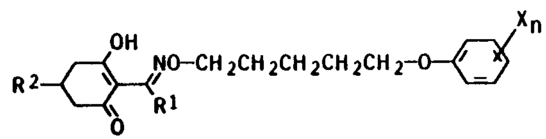 I (A = $-CH_2(CH_2)_3CH_2-O-$; Z = Phenyl)

| Nr. | R1 | R2 | Xn | phys. Daten / 1H-NMR [δ in ppm], Fp. [°C] |
|---|---|---|---|---|
| 11.01 | Ethyl | Tetrahydropyran-3-yl | - | 3,80-4,17 (m,6H), 6,90 (m,3H), 7,27 (m,2H) |
| 11.02 | Propyl | Tetrahydropyran-3-yl | - | 3,80-4,17 (m,6H), 6,90 (m,3H), 7,27 (m,2H) |
| 11.03 | Ethyl | Tetrahydropyran-4-yl | - | 3,90-4,17 (m,6H), 6,90 (m,3H), 7,27 (m,2H) |
| 11.04 | Propyl | Tetrahydropyran-4-yl | - | 3,90-4,17 (m,6H), 6,90 (m,3H), 7,27 (m,2H) |
| 11.05 | Ethyl | Tetrahydrothiopyran-3-yl | - | 3,97 (t,2H), 4,07 (t,2H), 6,90 (m,3H), 7,27 (m,2H) |
| 11.06 | Propyl | Tetrahydrothiopyran-3-yl | - | 3,97 (t,2H), 4,07 (t,2H), 6,90 (m,3H), 7,27 (m,2H) |
| 11.07 | Ethyl | Tetrahydropyran-3-yl | 4-F | 3,90 (m,4H), 4,03 (t,2H), 6,70-7,03 (m,4H) |
| 11.08 | Propyl | Tetrahydropyran-3-yl | 4-F | 3,90 (m,4H), 4,03 (t,2H), 6,70-7,03 (m,4H) |
| 11.09 | Ethyl | Tetrahydropyran-4-yl | 4-F | 3,83-4,13 (m,6H), 6,70-7,03 (m,4H) |
| 11.10 | Propyl | Tetrahydropyran-4-yl | 4-F | 3,83-4,13 (m,6H), 6,70-7,03 (m,4H) |
| 11.11 | Ethyl | Tetrahydrothiopyran-3-yl | 4-F | 3,90 (t,2H), 4,03 (t,2H) 6,70-7,03 (m,4H) |
| 11.12 | Propyl | Tetrahydrothiopyran-3-yl | 4-F | 3,90 (t,2H), 4,03 (t,2H) 6,70-7,03 (m,4H) |
| 11.13 | Ethyl | Tetrahydropyran-3-yl | 4-Cl | 3,80-4,10 (m,6H), 6,80 (d,2H), 7,20 (d,2H) |
| 11.14 | Propyl | Tetrahydropyran-3-yl | 4-Cl | 3,80-4,10 (m,6H), 6,80 (d,2H), 7,20 (d,2H) |
| 11.15 | Ethyl | Tetrahydropyran-4-yl | 4-Cl | 3,87-4,10 (m,6H), 6,80 (d,2H), 7,20 (d,2H) |
| 11.16 | Propyl | Tetrahydropyran-4-yl | 4-Cl | 3,87-4,10 (m,6H), 6,80 (d,2H), 7,20 (d,2H) |
| 11.17 | Ethyl | Tetrahydrothiopyran-3-yl | 4-Cl | 54- 61 |
| 11.18 | Propyl | Tetrahydrothiopyran-3-yl | 4-Cl | 3,90 (t,2H), 4,07 (t,2H), 6,80 (d,2H) 7,20 (d,2H) |

## Anwendungsbeispiele

Die herbizide Wirkung der Cyclohexenonoximether der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 0,25 kg/ha a.S.

Die Pflanzen wurden artenspezifisch bei Temperaturen von 10-25 °C bzw. 20-35 °C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Oryza sativa | Reis | rice |
| Setaria italica | Kolbenhirse | foxtail millet |
| Setaria viridis | Grüne Borstenhirse | green foxtail |

Mit 0,25 kg/ha a.S. im Nachauflaufverfahren eingesetzt, lassen sich mit den Beispielen 7.21 und 7.23 unerwünschte grasartige Pflanzen sehr gut bekämpfen, bei gleichzeitiger Verträglichkeit an der Beispielkultur Reis.

**Patentansprüche**
**Patentansprüche für folgende Vertragsstaaten : AT, BE, CH, DE, FR, GB, IT, LI, NL**

**1.** Cyclohexenonoximether der allgemeinen Formel I

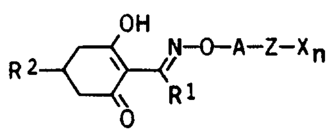

(I)

in der die Variablen folgende Bedeutung haben:

- $R^1$ eine $C_1$-$C_6$-Alkylgruppe;
- A eine ggf. $C_1$-$C_3$-Alkyl-substituierte $C_3$-$C_6$-Alkylen- oder $C_4$-$C_6$-Alkenylenkette, in der eine Methylengruppe durch ein Sauerstoff-, ein Schwefelatom, eine Sulfoxid-, eine Sulfongruppe oder -N($R^a$)-, worin
  - $R^a$ Wasserstoff, eine $C_1$-$C_4$-Alkylgruppe, eine $C_3$-$C_6$-Alkenylgruppe oder eine $C_3$-$C_6$-Alkinylgruppe bedeutet,

  ersetzt ist.
- Z Phenyl,
  ein 5-gliedriger Heteroaromat mit ein bis drei Heteroatomen, ausgewählt aus einer Gruppe bestehend aus drei Stickstoffatomen und einem Sauerstoff- oder Schwefelatom,
  ein 6-gliedriger Heteroaromat mit ein bis vier Stickstoffatomen;
- X eine Aminogruppe -$NR^aR^b$, worin
- $R^a$ Wasserstoff, eine $C_1$-$C_4$-Alkylgruppe, eine $C_3$-$C_6$-Alkenylgruppe oder eine $C_3$-$C_6$-Alkinylgruppe und
- $R^b$ Wasserstoff, eine $C_1$-$C_4$-Alkylgruppe, eine $C_3$-$C_6$-Alkenylgruppe, eine $C_3$-$C_6$-Alkinylgruppe, eine $C_1$-$C_6$-Acylgruppe oder ein Benzoylrest, wobei der aromatische Ring einen bis drei Substituenten tragen kann, ausgewählt aus einer Gruppe bestehend aus Nitro, Cyano, Halogen, $C_1$-$C_4$-Alkyl, $C_1$-$C_4$-Alkoxy, $C_1$-$C_4$-Alkylthio und $C_1$-$C_4$-Halogenalkyl bedeuten oder

Nitro, Cyano, Halogen, $C_1$-$C_4$-Alkyl, partiell oder vollständig halogeniertes $C_1$-$C_4$-Alkyl, $C_1$-$C_4$-Alkoxy, $C_1$-$C_4$-Alkylthio, partiell oder vollständig halogeniertes $C_1$-$C_4$-Alkoxy, Carboxyl, $C_1$-$C_4$-Alkoxycarbonyl, Benzyloxycarbonyl, Phenyl, wobei die aromatischen Reste zusätzlich einen bis drei der folgenden Substituenten tragen können:
Nitro, Cyano, Halogen, $C_1$-$C_4$-Alkyl, partiell oder vollständig halogeniertes $C_1$-$C_4$-Alkyl, $C_1$-$C_4$-Alkoxy, $C_1$-$C_4$-Alkylthio, partiell oder vollständig halogeniertes $C_1$-$C_4$-Alkoxy, Carboxyl, $C_1$-$C_4$-Alkoxycarbonyl, Benzyloxycarbonyl;

n 0 bis 3 oder 1 bis 5 für den Fall, daß X Halogen bedeutet;

$R^2$ eine $C_1$-$C_4$-Alkoxy-$C_1$-$C_6$-alkyl- oder $C_1$-$C_4$-Alkylthio-$C_1$-$C_6$-alkylgruppe;
eine $C_3$-$C_7$-Cycloalkylgruppe oder eine $C_5$-$C_7$-Cycloalkenylgruppe, wobei diese Gruppen ein bis drei Substituenten tragen können, ausgewählt aus einer Gruppe bestehend aus $C_1$-$C_4$-Alkyl, $C_1$-$C_4$-Alkoxy, $C_1$-$C_4$-Alkylthio, partiell oder vollständig halogeniertes $C_1$-$C_4$-Alkyl, Hydroxy und Halogen;
ein 5-gliedriger gesättigter Heterocyclus der ein oder zwei Heteroatome enthält, ausgewählt aus einer Gruppe bestehend aus Sauerstoff und Schwefel, wobei der Heterocyclus noch einen bis drei Reste tragen kann, ausgewählt aus einer Gruppe bestehend aus $C_1$-$C_4$-Alkyl, $C_1$-$C_4$-Alkoxy, $C_1$-$C_4$-Alkylthio und partiell oder vollständig halogeniertem $C_1$-$C_4$-Alkyl;
ein 6- oder 7-gliedriger gesättigter, ein- oder zweifach ungesättigter Heterocyclus, enthaltend ein oder zwei Heteroatome, ausgewählt aus einer Gruppe bestehend aus Sauerstoff und Schwefel, wobei der Heterocyclus noch einen bis drei Reste tragen kann, ausgewählt aus einer Gruppe bestehend aus Hydroxy, Halogen, $C_1$-$C_4$-Alkyl, $C_1$-$C_4$-Alkoxy, $C_1$-$C_4$-Alkylthio und partiell oder vollständig halogeniertem $C_1$-$C_4$-Alkyl;
ein 5-gliedriger Heteroaromat, enthaltend ein bis drei Heteroatome, ausgewählt aus einer Gruppe bestehend aus zwei Stickstoffatomen und einem Sauerstoff- oder Schwefelatom, wobei dieser Ring noch einen bis drei Reste tragen kann, ausgewählt aus einer Gruppe bestehend aus Halogen, Cyano, $C_1$-$C_4$-Alkyl, $C_1$-$C_4$-Alkoxy, $C_1$-$C_4$-Alkylthio, partiell oder vollständig halogeniertem $C_1$-$C_4$-Alkyl, $C_2$-$C_6$-Alkenyl, $C_2$-$C_6$-Alkenyloxy, partiell oder vollständig halogeniertem $C_2$-$C_6$-Alkenyl und $C_1$-$C_4$-Alkoxy-$C_1$-$C_4$-alkyl;
die Phenylgruppe oder die Pyridylgruppe, wobei diese Gruppen noch einen bis drei Reste tragen können, ausgewählt aus einer Gruppe bestehend aus Halogen, Nitro, Cyano, $C_1$-$C_4$-Alkyl, $C_1$-$C_4$-Alkoxy, $C_1$-$C_4$-Alkylthio, partiell oder vollständig halogeniertem $C_1$-$C_4$-Alkyl, $C_3$-$C_6$-Alkenyloxy, $C_3$-$C_6$-Alkinyloxy und $-NR^aR^b$, worin $R^a$ und $R^b$ die oben genannte Bedeutung haben,

bedeuten, sowie ihre landwirtschaftlich nutzbaren Salze und Ester von $C_1$-$C_{10}$-Carbonsäuren und anorganischen Säuren.

2. Cyclohexenoximether der Formel I nach Anspruch 1, wobei Z die Phenylgruppe bedeutet.

3. Verfahren zur Herstellung von Verbindungen der Formel I gemäß Anspruch 1, dadurch gekennzeichnet, daß man ein entsprechendes Cyclohexenon der Formel II

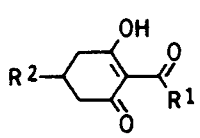

II

in an sich bekannter Weise in einem inerten organischen Lösungsmittel mit einem Hydroxylamin der Formel III

$H_2N$-O-A-Z-$X_n$ III,

oder einem Salz des entsprechenden Hydroxylamins umsetzt, und die Verfahrensprodukte gewünschtenfalls in ihre landwirtschaftlich nutzbaren Salze oder Ester von $C_1$-$C_{10}$-Carbonsäuren oder anorganischen Säuren überführt.

4. Herbizides Mittel, enthaltend inerte Zusatzstoffe und eine herbizid wirksame Menge mindestens einer Verbindung der Formel I gemäß Anspruch 1.

5. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Cyclohexenonderivates der Formel I gemäß Anspruch 1 behandelt.

**Patentansprüche für folgenden Vertragsstaat : ES**

1. Verfahren zur Herstellung von Cyclohexenonoximethern der allgemeinen Formel I

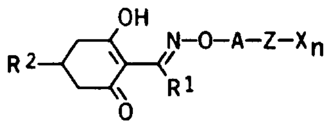

(I)

in der die Variablen folgende Bedeutung haben:

$R^1$ eine $C_1$-$C_6$-Alkylgruppe;

A eine ggf. $C_1$-$C_3$-Alkyl-substituierte $C_3$-$C_6$-Alkylen- oder $C_4$-$C_6$-Alkenylenkette, in der eine Methylengruppe durch ein Sauerstoff-, ein Schwefelatom, eine Sulfoxid-, eine Sulfongruppe oder -N($R^a$)-, worin

$R^a$ Wasserstoff, eine $C_1$-$C_4$-Alkylgruppe, eine $C_3$-$C_6$-Alkenylgruppe oder eine $C_3$-$C_6$-Alkinylgruppe bedeutet,

ersetzt ist.

Z Phenyl,

ein 5-gliedriger Heteroaromat mit ein bis drei Heteroatomen, ausgewählt aus einer Gruppe bestehend aus drei Stickstoffatomen und einem Sauerstoff- oder Schwefelatom,

ein 6-gliedriger Heteroaromat mit ein bis vier Stickstoffatomen;

X eine Aminogruppe -$NR^aR^b$, worin

$R^a$ Wasserstoff, eine $C_1$-$C_4$-Alkylgruppe, eine $C_3$-$C_6$-Alkenylgruppe oder eine $C_3$-$C_6$-Alkinylgruppe und

$R^b$ Wasserstoff, eine $C_1$-$C_4$-Alkylgruppe, eine $C_3$-$C_6$-Alkenylgruppe, eine $C_3$-$C_6$-Alkinylgruppe, eine $C_1$-$C_6$-Acylgruppe oder ein Benzoylrest, wobei der aromatische Ring einen bis drei Substituenten tragen kann, ausgewählt aus einer Gruppe bestehend aus Nitro, Cyano, Halogen, $C_1$-$C_4$-Alkyl, $C_1$-$C_4$-Alkoxy, $C_1$-$C_4$-Alkylthio und $C_1$-$C_4$-Halogenalkyl bedeuten oder

Nitro, Cyano, Halogen, $C_1$-$C_4$-Alkyl, partiell oder vollständig halogeniertes $C_1$-$C_4$-Alkyl, $C_1$-$C_4$-Alkoxy, $C_1$-$C_4$-Alkylthio, partiell oder vollständig halogeniertes $C_1$-$C_4$-Alkoxy, Carboxyl, $C_1$-$C_4$-Alkoxycarbonyl, Benzyloxycarbonyl, Phenyl, wobei die aromatischen Reste zusätzlich einen bis drei der folgenden Substituenten tragen können:

Nitro, Cyano, Halogen, $C_1$-$C_4$-Alkyl, partiell oder vollständig halogeniertes $C_1$-$C_4$-Alkyl, $C_1$-$C_4$-Alkoxy, $C_1$-$C_4$-Alkylthio, partiell oder vollständig halogeniertes $C_1$-$C_4$-Alkoxy, Carboxyl, $C_1$-$C_4$-Alkoxycarbonyl, Benzyloxycarbonyl;

n 0 bis 3 oder 1 bis 5 für den Fall, daß X Halogen bedeutet;

$R^2$ eine $C_1$-$C_4$-Alkoxy-$C_1$-$C_6$-alkyl- oder $C_1$-$C_4$-Alkylthio-$C_1$-$C_6$-alkylgruppe;

eine $C_3$-$C_7$-Cycloalkylgruppe oder eine $C_5$-$C_7$-Cycloalkenylgruppe, wobei diese Gruppen ein bis drei Substituenten tragen können, ausgewählt aus einer Gruppe bestehend aus $C_1$-$C_4$-Alkyl, $C_1$-$C_4$-Alkoxy, $C_1$-$C_4$-Alkylthio, partiell oder vollständig halogeniertes $C_1$-$C_4$-Alkyl, Hydroxy und Halogen;

ein 5-gliedriger gesättigter Heterocyclus der ein oder zwei Heteroatome enthält, ausgewählt aus einer Gruppe bestehend aus Sauerstoff und Schwefel, wobei der Heterocyclus noch einen bis drei Reste tragen kann, ausgewählt aus einer Gruppe bestehend aus $C_1$-$C_4$-Alkyl, $C_1$-$C_4$-Alkoxy, $C_1$-$C_4$-Alkylthio und partiell oder vollständig halogeniertem $C_1$-$C_4$-Alkyl;

ein 6- oder 7-gliedriger gesättigter, ein- oder zweifach ungesättigter Heterocyclus, enthaltend ein oder zwei Heteroatome, ausgewählt aus einer Gruppe bestehend aus Sauerstoff und Schwefel, wobei der Heterocyclus noch einen bis drei Reste tragen kann, ausgewählt aus einer Gruppe bestehend aus Hydroxy, Halogen, $C_1$-$C_4$-Alkyl, $C_1$-$C_4$-Alkoxy, $C_1$-$C_4$-Alkylthio und partiell oder vollständig halogeniertem $C_1$-$C_4$-Alkyl;

ein 5-gliedriger Heteroaromat, enthaltend ein bis drei Heteroatome, ausgewählt aus einer Gruppe bestehend aus zwei Stickstoffatomen und einem Sauerstoff- oder Schwefelatom, wobei dieser Ring noch einen bis drei Reste tragen kann, ausgewählt aus einer Gruppe

bestehend aus Halogen, Cyano, $C_1$-$C_4$-Alkyl, $C_1$-$C_4$-Alkoxy, $C_1$-$C_4$-Alkylthio, partiell oder vollständig halogeniertem $C_1$-$C_4$-Alkyl, $C_2$-$C_6$-Alkenyl, $C_2$-$C_6$-Alkenyloxy, partiell oder vollständig halogeniertem $C_2$-$C_6$-Alkenyl und $C_1$-$C_4$-Alkoxy-$C_1$-$C_4$-alkyl;
die Phenylgruppe oder die Pyridylgruppe, wobei diese Gruppen noch einen bis drei Reste tragen können, ausgewählt aus einer Gruppe bestehend aus Halogen, Nitro, Cyano, $C_1$-$C_4$-Alkyl, $C_1$-$C_4$-Alkoxy, $C_1$-$C_4$-Alkylthio, partiell oder vollständig halogeniertem $C_1$-$C_4$-Alkyl, $C_3$-$C_6$-Alkenyloxy, $C_3$-$C_6$-Alkinyloxy und $-NR^aR^b$, worin $R^a$ und $R^b$ die oben genannte Bedeutung haben,
bedeuten, sowie ihren landwirtschaftlich nutzbaren Salzen und Estern von $C_1$-$C_{10}$-Carbonsäuren und anorganischen Säuren,

dadurch gekennzeichnet, daß man ein entsprechendes Cyclohexenon der Formel II

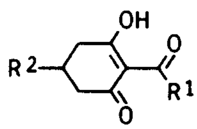

II

in einem inerten organischen Lösungsmittel mit einem Hydroxylamin der Formel III

$H_2N$-O-A-Z-$X_n$ III,

oder einem Salz des entsprechenden Hydroxylamins umsetzt, und die Verfahrensprodukte gewünschtenfalls in ihre landwirtschaftlich nutzbaren Salze oder Ester von $C_1$-$C_{10}$-Carbonsäuren oder anorganischen Säuren überführt.

**2.** Verfahren zur Herstellung von Cyclohexenonoximethern der Formel I nach Anspruch 1, wobei Z die Phenylgruppe bedeutet.

**3.** Herbizides Mittel, enthaltend inerte Zusatzstoffe und eine herbizid wirksame Menge mindestens eines Cyclohexenonoximethers der Formel I gemäß Anspruch 1.

**4.** Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Cyclohexenonoximethers der Formel I gemäß Anspruch 1 behandelt.

## Claims

**Claims for the following Contracting States : AT, BE, CH, DE, FR, GB, IT, LI, NL**

**1.** A cyclohexenone oxime ether of the formula I

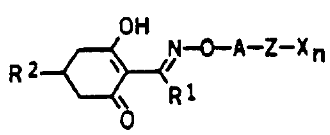

(I)

where

$R^1$ is $C_1$-$C_6$-alkyl;

A is an unsubstituted or $C_1$-$C_3$-alkyl substituted $C_3$-$C_6$-alkylene or $C_4$-$C_6$-alkenylene chain in which a methylene group is replaced by an oxygen atom, a sulfur atom, a sulfoxyl group, a sulfonyl group or $-N(R^a)-$, where $R^a$ is hydrogen, $C_1$-$C_4$-alkyl, $C_3$-$C_6$-alkenyl or $C_3$-$C_6$-alkynyl;

Z is phenyl,
a 5-membered heteroaromatic structure having from one to three hetero atoms selected from the group consisting of three nitrogen atoms and an oxygen or sulfur atom, or
a 6-membered heteroaromatic structure having from one to four nitrogen atoms;

X is an amino group $-NR^aR^b$, where

$R^a$ is hydrogen, $C_1$-$C_4$-alkyl, $C_3$-$C_6$-alkenyl or $C_3$-$C_6$-alkynyl and

$R^b$ is hydrogen, $C_1$-$C_4$-alkyl, $C_3$-$C_6$-alkenyl, $C_3$-$C_6$-alkynyl, $C_1$-$C_6$-acyl or benzoyl, where the aromatic ring may carry from one to three substituents selected from the group consisting of nitro, cyano, halogen, $C_1$-$C_4$-alkyl, $C_1$-$C_4$-alkoxy, $C_1$-$C_4$-alkylthio and $C_1$-$C_4$-haloalkyl or nitro, cyano, halogen, $C_1$-$C_4$-alkyl, partially or completely halogenated $C_1$-$C_4$-alkyl, $C_1$-$C_4$-alkoxy, $C_1$-$C_4$-alkylthio, partially or completely halogenated $C_1$-$C_4$-alkoxy, carboxyl, $C_1$-$C_4$-alkoxycarbonyl, benzyloxycarbonyl or phenyl, where the aromatic radicals may additionally carry from one to three of the following substituents:
nitro, cyano, halogen, $C_1$-$C_4$-alkyl, partially or completely halogenated $C_1$-$C_4$-alkyl, $C_1$-$C_4$-alkoxy, $C_1$-$C_4$-alkylthio, partially or completely halogenated $C_1$-$C_4$-alkoxy, carboxyl, $C_1$-$C_4$-alkoxycarbonyl or benzyloxycarbonyl;

n is from 0 to 3 or from 1 to 5 where X is halogen;

$R^2$ is $C_1$-$C_4$-alkoxy-$C_1$-$C_6$-alkyl or $C_1$-$C_4$-alkylthio-$C_1$-$C_6$-alkyl;
$C_3$-$C_7$-cycloalkyl or $C_5$-$C_7$-cycloalkenyl, where these groups may carry from one to three substituents selected from the group consisting of $C_1$-$C_4$-alkyl, $C_1$-$C_4$-alkoxy, $C_1$-$C_4$-alkylthio, partially or completely halogenated $C_1$-$C_4$-alkyl, hydroxyl and halogen;
a 5-membered saturated heterocyclic structure containing one or two hetero atoms selected from the group consisting of oxygen and sulfur, where the heterocyclic structure may additionally carry from one to three radicals selected from the group consisting of $C_1$-$C_4$-alkyl, $C_1$-$C_4$-alkoxy, $C_1$-$C_4$-alkylthio and partially or completely halogenated $C_1$-$C_4$-alkyl;
a 6- or 7-membered saturated or mono- or diunsaturated heterocyclic structure containing one or two hetero atoms selected from the group consisting of oxygen and sulfur, where the heterocyclic structure may additionally carry from one to three radicals selected from the group consisting of hydroxyl, halogen, $C_1$-$C_4$-alkyl, $C_1$-$C_4$-alkoxy, $C_1$-$C_4$-alkylthio and partially or completely halogenated $C_1$-$C_4$-alkyl;
a 5-membered heteroaromatic structure containing from one to three hetero atoms selected from the group consisting of two nitrogen atoms and an oxygen or sulfur atom, where this ring may additionally carry from one to three radicals selected from the group consisting of halogen, cyano, $C_1$-$C_4$-alkyl, $C_1$-$C_4$-alkoxy, $C_1$-$C_4$-alkylthio, partially or completely halogenated $C_1$-$C_4$-alkyl, $C_2$-$C_6$-alkenyl, $C_2$-$C_6$-alkenyloxy, partially or completely halogenated $C_2$-$C_6$-alkenyl and $C_1$-$C_4$-alkoxy-$C_1$-$C_4$-alkyl;
phenyl or pyridyl, where these groups may additionally carry from one to three radicals selected from the group consisting of halogen, nitro, cyano, $C_1$-$C_4$-alkyl, $C_1$-$C_4$-alkoxy, $C_1$-$C_4$-alkylthio, partially or completely halogenated $C_1$-$C_4$-alkyl, $C_3$-$C_6$-alkenyloxy, $C_3$-$C_6$-alkynyloxy and -$NR^aR^b$, where $R^a$ and $R^b$ have the abovementioned meanings,
or its agriculturally useful salts and esters of $C_1$-$C_{10}$-carboxylic acids and inorganic acids.

2. A cyclohexenone oxime ether of the formula I as claimed in claim 1, where Z is phenyl.

3. A process for the preparation of a compound of the formula I as claimed in claim 1, which comprises reacting a corresponding cyclohexenone of the formula II

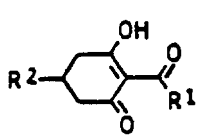

II

in a conventional manner in an inert organic solvent with a hydroxylamine of the formula III

$H_2N$-O-A-Z-$X_n$ III

or with a salt of the corresponding hydroxylamine, and if desired converting the products into their agriculturally useful salts or esters of $C_1$-$C_{10}$-carboxylic acids or inorganic acids.

4. A herbicidal composition containing inert additives and a herbicidally effective amount of at least one compound of the formula I as claimed in claim 1.

**5.** A method of controlling undesirable plant growth, wherein the undesirable plants and/or their habitat are (is) treated with a herbicidally effective amount of a cyclohexenone derivative of the formula I as claimed in claim 1.

**Claims for the following Contracting State : ES**

**1.** A process for the preparation of a cyclohexenone oxime ether of the formula I

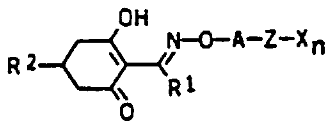

(I)

where

$R^1$ is $C_1$-$C_6$-alkyl;

A is an unsubstituted or $C_1$-$C_3$-alkyl substituted $C_3$-$C_6$-alkylene or $C_4$-$C_6$-alkenylene chain in which a methylene group is replaced by an oxygen atom, a sulfur atom, a sulfoxyl group, a sulfonyl group or -N($R^a$)-, where

$R^a$ is hydrogen, $C_1$-$C_4$-alkyl, $C_3$-$C_6$-alkenyl or $C_3$-$C_6$-alkynyl;

Z is phenyl,
a 5-membered heteroaromatic structure having from one to three hetero atoms selected from the group consisting of three nitrogen atoms and an oxygen or sulfur atom, or a 6-membered heteroaromatic structure having from one to four nitrogen atoms;

X is an amino group -$NR^aR^b$, where

$R^a$ is hydrogen, $C_1$-$C_4$-alkyl, $C_3$-$C_6$-alkenyl or $C_3$-$C_6$-alkynyl and

$R^b$ is hydrogen, $C_1$-$C_4$-alkyl, $C_3$-$C_6$-alkenyl, $C_3$-$C_6$-alkynyl, $C_1$-$C_6$-acyl or benzoyl, where the aromatic ring may carry from one to three substituents selected from the group consisting of nitro, cyano, halogen, $C_1$-$C_4$-alkyl, $C_1$-$C_4$-alkoxy, $C_1$-$C_4$-alkylthio and $C_1$-$C_4$-haloalkyl or
nitro, cyano, halogen, $C_1$-$C_4$-alkyl, partially or completely halogenated $C_1$-$C_4$-alkyl, $C_1$-$C_4$-alkoxy, $C_1$-$C_4$-alkylthio, partially or completely halogenated $C_1$-$C_4$-alkoxy, carboxyl, $C_1$-$C_4$-alkoxycarbonyl, benzyloxycarbonyl or phenyl, where the aromatic radicals may additionally carry from one to three of the following substituents:
nitro, cyano, halogen, $C_1$-$C_4$-alkyl, partially or completely halogenated $C_1$-$C_4$-alkyl, $C_1$-$C_4$-alkoxy, $C_1$-$C_4$-alkylthio, partially or completely halogenated $C_1$-$C_4$-alkoxy, carboxyl, $C_1$-$C_4$-alkoxycarbonyl or benzyloxycarbonyl;

n is from 0 to 3 or from 1 to 5 where X is halogen;

$R^2$ is $C_1$-$C_4$-alkoxy-$C_1$-$C_6$-alkyl or $C_1$-$C_4$-alkylthio-$C_1$-$C_6$-alkyl;
$C_3$-$C_7$-cycloalkyl or $C_5$-$C_7$-cycloalkenyl, where these groups may carry from one to three substituents selected from the group consisting of $C_1$-$C_4$-alkyl, $C_1$-$C_4$-alkoxy, $C_1$-$C_4$-alkylthio, partially or completely halogenated $C_1$-$C_4$-alkyl, hydroxyl and halogen;
a 5-membered saturated heterocyclic structure containing one or two hetero atoms selected from the group consisting of oxygen and sulfur, where the heterocyclic structure may additionally carry from one to three radicals selected from the group consisting of $C_1$-$C_4$-alkyl, $C_1$-$C_4$-alkoxy, $C_1$-$C_4$-alkylthio and partially or completely halogenated $C_1$-$C_4$-alkyl;
a 6- or 7-membered saturated or mono- or diunsaturated heterocyclic structure containing one or two hetero atoms selected from the group consisting of oxygen and sulfur, where the heterocyclic structure may additionally carry from one to three radicals selected from the group consisting of hydroxyl, halogen, $C_1$-$C_4$-alkyl, $C_1$-$C_4$-alkoxy, $C_1$-$C_4$-alkylthio and partially or completely halogenated $C_1$-$C_4$-alkyl;
a 5-membered heteroaromatic structure containing from one to three hetero atoms selected from the group consisting of two nitrogen atoms and an oxygen or sulfur atom, where this ring may additionally carry from one to three radicals selected from the group consisting of halogen, cyano, $C_1$-$C_4$-alkyl, $C_1$-$C_4$-alkoxy, $C_1$-$C_4$-alkylthio, partially or completely halogenated $C_1$-$C_4$-alkyl, $C_2$-$C_6$-alkenyl, $C_2$-$C_6$-alkenyloxy, partially or completely halogenated $C_2$-$C_6$-alkenyl and $C_1$-$C_4$-alkoxy-$C_1$-$C_4$-alkyl;
phenyl or pyridyl, where these groups may additionally carry from one to three radicals selected from the group consisting of halogen, nitro, cyano, $C_1$-$C_4$-alkyl, $C_1$-$C_4$-alkoxy, $C_1$-$C_4$-

alkylthio, partially or completely halogenated $C_1$-$C_4$-alkyl, $C_3$-$C_6$-alkenyloxy, $C_3$-$C_6$-alkynyloxy and $-NR^aR^b$, where $R^a$ and $R^b$ have the abovementioned meanings,

or its agriculturally useful salts and esters of $C_1$-$C_{10}$-carboxylic acids and inorganic acids,

which comprises reacting a corresponding cyclohexenone of the formula II

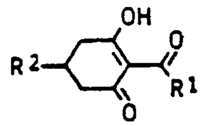

II

in an inert organic solvent with a hydroxylamine of the formula III

$H_2N$-O-A-Z-$X_n$ III

or with a salt of the corresponding hydroxylamine, and if desired converting the products into their agriculturally useful salts or esters of $C_1$-$C_{10}$-carboxylic acids or inorganic acids.

**2.** A process for the preparation of a cyclohexenone oxime ether of the formula I as claimed in claim 1, where Z is phenyl.

**3.** A herbicidal composition containing inert additives and a herbicidally effective amount of at least one cyclohexanone oxime ether of the formula I as claimed in claim 1.

**4.** A method of controlling undesirable plant growth, wherein the undesirable plants and/or their habitat are (is) treated with a herbicidally effective amount of a cyclohexanone oxime ether of the formula I as claimed in claim 1.

**Revendications**

**Revendications pour les Etats contractants suivants : AT, BE, CH, DE, FR, GB, IT, LI, NL**

**1.** Cyclohexenonoximéthers de la formule générale I

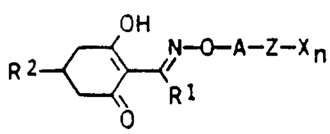

(I)

dans lesquels les variables ont les significations suivantes :

- $R^1$ un groupe alkyle en $C_1$-$C_6$;
- A une chaîne alkylène en $C_3$-$C_6$ ou alcénylène en $C_4$-$C_6$ éventuellement substituée par alkyle en $C_1$-$C_3$ et dans laquelle un groupe méthylène est remplacée par un atome d'oxygène, un atome de soufre, un groupe sulfoxyle, un groupe sulfone ou $-N(R^a)-$
  $R^a$ représentant hydrogène, un groupe alkyle en $C_1$-$C_4$, un groupe alcényle en $C_3$-$C_6$ ou un groupe alcinyle en $C_3$-$C_6$
- Z phényle, un hétéroaromate à 5 chaînons avec un à trois hétéroatomes choisis dans le groupe formé par trois atomes d'azote et un atome d'oxygène ou de soufre;
  un hétéroaromate à 6 chaînons avec un à quatre atomes d'azote.
- X un groupe amino $-NR^aR^b$ où
  $R^a$ représente hydrogène, un groupe alkyle en $C_1$-$C_4$, un groupe alcényle en $C_3$-$C_6$ ou un groupe alcinyle en $C_3$-$C_6$ et
  $R^b$ représente hydrogène, un groupe alkyle en $C_1$-$C_4$, alcényle en $C_3$-$C_6$, alcinyle en $C_3$-$C_6$, acyle en $C_1$-$C_6$ ou un reste benzoyle, le cycle aromatique pouvant porter un à trois substituants choisis dans le groupe formé par nitro, cyano, halogène, alkyle en $C_1$-$C_4$, alcoxy en $C_1$-$C_4$, alkyle en $C_1$-$C_4$-thio et halogénalkyle en $C_1$-$C_4$ ou bien
  nitro, cyano, halogène, alkyle en $C_1$-$C_4$, alkyle en $C_1$-$C_4$, partiellement ou totalement halogéné, alcoxy en $C_1$-$C_4$, alkyle en $C_1$-$C_1$-thio, alcoxy en $C_1$-$C_4$ partiellement ou totalement

halogéné, carboxyle, alcoxy en $C_1$-$C_4$-carbonyle, benzyloxycarbonyle, phényle les restes aromatiques pouvant porter un à trois des substituants suivants :
nitro, cyano, halogène, alkyle en $C_1$-$C_4$, alkyle en $C_1$-$C_4$ partiellement ou totalement halogéné, alcoxy en $C_1$-$C_4$, benzyloxycarbonyle, alkyle en $C_1$-$C_4$-thio, alcoxy en $C_1$-$C_4$ partiellement ou totalement halogéné, carboxyle, alcoxy en $C_1$-$C_4$-carbonyle.

n 0 à 3 ou 1 à 5 dans le cas où X représente halogène

$R^2$ un groupe alcoxy en $C_1$-$C_4$-alkyle en $C_1$-$C_6$ ou alkyle en $C_1$-$C_4$-thio-alkyle en $C_1$-$C_6$.
un groupe cycloalkyle en $C_3$-$C_7$ ou cycloalcényle en $C_5$-$C_7$, ces groupes pouvant porter un à trois substituants choisis dans le groupe formé de alkyle en $C_1$-$C_4$, alkyloxy en $C_1$-$C_4$, alkyle en $C_1$-$C_4$-thio, alkyle en $C_1$-$C_4$ partiellement ou totalement halogéné, hydroxy et halogène, un hétérocycle saturé à 5 chaînons qui contient un ou deux hétéroatomes choisis dans le groupe formé d'oxygène et soufre, l'hétérocycle pouvant porter encore un à trois restes choisis dans le groupe formé de alkyle en $C_1$-$C_4$, alcoxy en $C_1$-$C_4$, alkyle en $C_1$-$C_4$-thio et alkyle en $C_1$-$C_4$ partiellement ou totalement halogéné
un hétérocycle à 6 ou 7 chaînons saturé, une ou deux fois insaturé, contenant un ou deux hétéroatomes, choisis dans le groupe formé d'oxygène et soufre, l'hétérocycle pouvant porter encore un à trois restes choisis dans un groupe formé de hydroxy, halogène, alkyle en $C_1$-$C_4$, alcoxy en $C_1$-$C_4$, alkyle en $C_1$-$C_4$-thio et alkyle en $C_1$-$C_4$ partiellement ou totalement halogéné.
un hétéroaromate à 5 chaînons, contenant un à trois hétéroatomes choisis dans un groupe formé de deux atomes d'azote et d'un atome d'oxygène ou de soufre, ce cycle pouvant porter encore un à trois restes choisis dans un groupe formé de halogène, cyano, alkyle en $C_1$-$C_4$, alcoxy en $C_1$-$C_4$, alkyle en $C_1$-$C_4$-thio, alkyle en $C_1$-$C_4$ partiellement ou totalement halogéné, alcényle en $C_2$-$C_6$, alcényloxy en $C_2$-$C_6$, alcényle en $C_2$-$C_6$ partiellement ou totalement halogéné et alcoxy en $C_1$-$C_4$-alkyle en $C_1$-$C_4$
le groupe phényle ou le groupe pyridyle, ces groupes pouvant porter encore un à trois restes choisis dans le groupe formé d'halogène, nitro, cyano, alkyle en $C_1$-$C_4$,alcoxy en $C_1$-$C_4$, alkyle en $C_1$-$C_4$-thio, alkyle en $C_1$-$C_4$ partiellement ou totalement halogéné, alcényloxy en $C_3$-$C_6$, alcinyloxy en $C_3$-$C_6$ et $NR^aR^b$, $R^a$ et $R^b$ ayant la signification indiquée plus haut,
ainsi que leurs sels et esters d'acides carboxyliques en $C_1$-$C_{10}$ et d'acides inorganiques utilisables en agriculture.

**2.** Cyclohexenoxioximéther de la formule I selon la revendication 1, où Z représente le groupe phényle.

**3.** Procédé de préparation de composés de formule I selon la revendication 1, caractérisé par le fait que l'on fait réagir une cyclohexenone correspondante de formule II

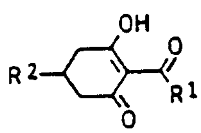

II

de manière connue en soi, dans un solvant organique inerte avec une hydroxylamine de formule III

$H_2N$-O-A-Z-$X_n$ III

ou un sel de l'hydroxylamine correspondante et on transforme le produit obtenu par le procédé, de façon souhaitable en son sel ou ester utilisable en agriculture d'acides carboxyliques en $C_1$-$C_{10}$ ou d'acides inorganiques.

**4.** Agent herbicide contenant des additifs inertes et une quantité à action herbicide d'au moins un composé de formule I selon la revendication 1.

**5.** Procédé de lutte contre la croissance de plantes indésirables, caractérisé par le fait qu'on traite les plantes indésirables et/ou leur biotope avec une quantité à effet herbicide d'un dérivé de dicyclohexenone de formule I selon la revendication 1.

**Revendications pour l'Etat contractant suivant : ES**

**1.** Procédé de préparation de cyclohexenonoximéthers de la formule générale I

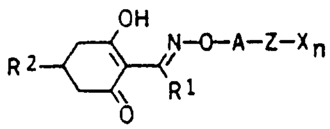

(I)

dans lesquels les variables ont les significations suivantes :

$R^1$ un groupe alkyle en $C_1$-$C_6$;

A une chaîne alkylène en $C_3$-$C_6$ ou alcénylène en $C_4$-$C_6$ éventuellement substituée par alkyle en $C_1$-$C_3$ et dans laquelle un groupe méthylène est remplacée par un atome d'oxygène, un atome de soufre, un groupe sulfoxyle, un groupe sulfone ou -N($R^a$)-
$R^a$ représentant hydrogène, un groupe alkyle en $C_1$-$C_4$, un groupe alcényle en $C_3$-$C_6$ ou un groupe alcinyle en $C_3$-$C_6$

Z phényle, un hétéroaromate à 5 chaînons avec un à trois hétéroatomes choisis dans le groupe formé par trois atomes d'azote et un atome d'oxygène ou de soufre ;
un hétéroaromate à 6 chaînons avec un à quatre atomes d'azote.

X un groupe amino -$NR^aR^b$ où
$R^a$ représente hydrogène, un groupe alkyle en $C_1$-$C_4$, un groupe alcényle en $C_3$-$C_6$ ou un groupe alcinyle en $C_3$-$C_6$ et
$R^b$ représente hydrogène, un groupe alkyle en $C_1$-$C_4$, alcényle en $C_3$-$C_6$, alcinyle en $C_3$-$C_6$, acyle en $C_1$-$C_6$ ou un reste benzoyle, le cycle aromatique pouvant porter un à trois substituants choisis dans le groupe formé par nitro, cyano, halogène, alkyle en $C_1$-$C_4$, alcoxy en $C_1$-$C_4$, alkyl en $C_1$-$C_4$-thio et halogénalkyle en $C_1$-$C_4$ ou bien
nitro, cyano, halogène, alkyle en $C_1$-$C_4$, alkyle en $C_1$-$C_4$, partiellement ou totalement halogéné, alcoxy en $C_1$-$C_4$, alkyle en $C_1$-$C_1$-thio, alcoxy en $C_1$-$C_4$ partiellement ou totalement halogéné, carboxyle, alcoxy en $C_1$-$C_4$-carbonyle, benzyloxycarbonyle, phényle les restes aromatiques pouvant porter un à trois des substituants suivants :
nitro, cyano, halogène, alkyle en $C_1$-$C_4$, alkyle en $C_1$-$C_4$ partiellement ou totalement halogéné, alcoxy en $C_1$-$C_4$, benzyloxycarbonyle, alkyle en $C_1$-$C_4$-thio, alcoxy en $C_1$-$C_4$ partiellement ou totalement halogéné, carboxyle, alcoxy en $C_1$-$C_4$-carbonyle.

n 0 à 3 ou 1 à 5 dans le cas où X représente halogène

$R^2$ un groupe alcoxy en $C_1$-$C_4$-alkyle en $C_1$-$C_6$ ou alkyle en $C_1$-$C_4$-thio-alkyle en $C_1$-$C_6$.
un groupe cycloalkyle en $C_3$-$C_7$ ou cycloalcényle en $C_5$-$C_7$, ces groupes pouvant porter un à trois substituants choisis dans le groupe formé de alkyle en $C_1$-$C_4$, alkyloxy en $C_1$-$C_4$, alkyle en $C_1$-$C_4$-thio, alkyle en $C_1$-$C_4$ partiellement ou totalement halogéné, hydroxy et halogène, un hétérocycle saturé à 5 chaînons qui contient un ou deux hétéroatomes choisis dans le groupe formé d'oxygène et soufre, l'hétérocycle pouvant porter encore un à trois restes choisis dans le groupe formé de alkyle en $C_1$-$C_4$, alcoxy en $C_1$-$C_4$, alkyle en $C_1$-$C_4$-thio et alkyle en $C_1$-$C_4$ partiellement ou totalement halogéné
un hétérocycle à 6 ou 7 chaînons saturé, une ou deux fois insaturé, contenant un ou deux hétéroatomes, choisis dans le groupe formé d'oxygène et soufre, l'hétérocycle pouvant porter encore un à trois restes choisis dans un groupe formé de hydroxy, halogène, alkyle en $C_1$-$C_4$, alcoxy en $C_1$-$C_4$, alkyle en $C_1$-$C_4$-thio et alkyle en $C_1$-$C_4$ partiellement ou totalement halogéné
un hétéroaromate à 5 chaînons, contenant un à trois hétéroatomes choisis dans un groupe formé de deux atomes d'azote et d'un atome d'oxygène ou de soufre, ce cycle pouvant porter encore un à trois restes choisis dans un groupe formé de halogène, cyano, alkyle en $C_1$-$C_4$, alcoxy en $C_1$-$C_4$, alkyle en $C_1$-$C_4$-thio, alkyle en $C_1$-$C_4$ partiellement ou totalement halogéné, alcényle en $C_2$-$C_6$, alcényloxy en $C_2$-$C_6$, alcényle en $C_2$-$C_6$ partiellement ou totalement halogéné et alcoxy en $C_1$-$C_4$-alkyle en $C_1$-$C_4$
le groupe phényle ou le groupe pyridyle, ces groupes pouvant porter encore un à trois restes choisis dans le groupe formé d'halogène, nitro, cyano, alkyle en $C_1$-$C_4$,alcoxy en $C_1$-$C_4$, alkyle en $C_1$-$C_4$-thio, alkyle en $C_1$-$C_4$ partiellement ou totalement halogéné, alcényloxy en $C_3$-$C_6$, alcinyloxy en $C_3$-$C_6$ et -$NR^aR^b$, $R^a$ et $R^b$ ayant la signification indiquée plus haut,

ainsi que leurs sels et esters d'acides carboxyliques en $C_1$-$C_{10}$ et d'acides inorganiques utilisables en agriculture,
caractérisé par le fait que l'on fait réagir une cyclohexenone correspondante de formule II

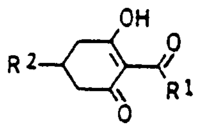

II

de manière connue en soi, dans un solvant organique inerte avec une hydroxylamine de formule III

$H_2N$-O-A-Z-$X_n$    III

ou un sel de l'hydroxylamine correspondante et on transforme le produit obtenu par le procédé, de façon souhaitable en son sel ou ester utilisable en agriculture d'acides carboxyliques en $C_1$-$C_{10}$ ou d'acides inorganiques.

2. Procédé de préparation de cyclohexenoxioximéther de la formule I selon la revendication 1, Z représentnt le groupe phényle.

3. Agent herbicide contenant des additifs inertes et une quantité à action herbicide d'au moins un composé de formule I selon la revendication 1.

4. Procédé de lutte contre la croissance de plantes indésirables, caractérisé par le fait qu'on traite les plantes indésirables et/ou leur biotope avec une quantité à effet herbicide d'un dérivé de dicyclohexenone de formule I selon la revendication 1.